# FASCICULE DE BREVET EUROPEEN

(11) **EP 1 773 835 B1**
(45) Date de publication et mention de la délivrance du brevet: **24.09.2014**
(21) Numéro de dépôt: 05788772.1
(22) Date de dépôt: 13.07.2005
(51) Int. Cl.: C07D 471/04, A61K 31/519

(54) **DERIVES DE PYRIDO-PYRIDO-PYRIMIDINE, LEUR PREPARATION, LEUR APPLICATION DANS LE TRAITEMENT DU CANCER**
PYRIDO-PYRIMIDINDERIVATE, DEREN HERSTELLUNG UND THERAPEUTISCHE VERWENDUNG ZUR BEHANDLUNG VON KREBS
PYRIDO-PYRIDO-PYRIMIDINE DERIVATIVES, PREPARATION THEREOF, THERAPEUTIC USE THEREOF FOR TREATING CANCER

(30) Priorité: 15.07.2004 FR 0407898
(43) Date de publication de la demande: 18.04.2007
(73) Titulaire: SANOFI, 75008 Paris (FR)
(72) Inventeur: BOURRIE, Bernard, F-34980 Saint-Gély-du-Fesc (FR); CASELLAS, Pierre, F-34090 Montpellier (FR); JEGHAM, Samir, F-34980 Montferrier-sur-Lez (FR); PERREAUT, Pierre, F-34980 Saint-Clément-de-Rivière (FR)
(74) Mandataire: Lecocq, Fabrice
(86) Numéro de dépôt international: PCT/FR2005/001809
(87) Numéro de publication internationale: WO 2006/016067

(56) Documents cités:
- WO-A-96/15128
- WO-A-2004/085436
- SCHROEDER M C ET AL: "SOLUBLE 2-SUBSTITUTED AMINOPYRIDOÄ2,3-DÜPYRIMIDIN-7-YL UREAS. STRUCTURE-ACTIVITY RELATIONSHIPS AGAINST SELECTED TYROSINE KINASES AND EXPLORATION OF IN VITRO AND IN VIVO ANTICANCER ACTIVITY" JOURNAL OF MEDICINAL CHEMISTRY, AMERICAN CHEMICAL SOCIETY. WASHINGTON, US, vol. 44, no. 12, 7 juin 2001 (2001-06-07), pages 1915-1926, XP001152609 ISSN: 0022-2623
- THOMPSON A M ET AL: "Synthesis and structure-activity relationships of soluble 7-substituted 3-(3,5-dimethoxyphenyl)-1,6-naphthyridin-2 -amines and related ureas as dual inhibitors of the fibroblast growth factor receptor-1 and vascular endothelial growth factor receptor-2 tyrosine kinases" JOURNAL OF MEDICINAL CHEMISTRY, vol. 48, no. 14, 17 juin 2005 (2005-06-17), pages 4628-4653, XP002372558 ISSN: 0022-2623

## Description

La présente invention a pour objet des dérivés de pyrido[2,3-d]pyrimidine, leur préparation et leur application en thérapeutique.

Des composés dérivés de pyrido[2,3-d]pyrimidine sont décrits dans les demandes de brevets WO 01/55 147 et WO 03/000 011 et dans les brevets EP-B-790 997 et US 5 733 913. Ces composés sont potentiellement utiles pour traiter les troubles de la prolifération cellulaire.

On décrit ici des composés répondant à la formule (I) : 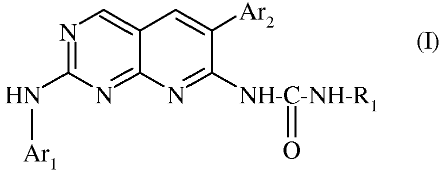 dans laquelle :
- R₁ est sélectionné dans un groupe constitué par (C₁-C₆)alkyle, (C₃-C₇)cycloalkyle, CH₂COR₄, phényle, ou phényle substitué par hydroxy et/ou halogène et/ou (C₁-C₆)alkyle.
- R₄ représente un groupe hydroxyle, (C₁-C₄)alcoxy, amino, (C₁-C₄)alkylamino, di(C₁-C₄)alkylamino ;
- Ar₁ représente un radical choisi parmi : 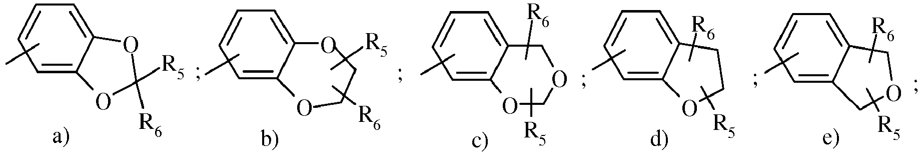
- R₅ représente un groupe cyano, hydroxy(C₁-C₄)alkyle, (C₁-C₆)alcoxy(C₁-C₆)alkyle ou un groupement (CH₂)ₙNR₇R₈, CO₂R₇, CONHNR₇R₈, CONR₇R₈, CONR₈OR₉, (CH₂)ₙNR₇COR₈, (CH₂)ₙNR₇COOR₈ ;
- R₆ représente un atome d'hydrogène, un groupe (C₁-C₄)alkyle ou l'une des valeurs de R₅ ;
- Ou bien R₅ et R₆, tels que définis précédemment, sont liés ensemble pour former un cycle de 4 à 7 chaînons comportant de 0 à 2 hétéroatomes choisis parmi N et O, ledit cycle de 4 à 7 chaînons étant éventuellement substitué par un ou plusieurs substituants indépendamment choisis parmi halogène, (C₁-C₄)alkyle, (C₁-C₄)alkyle halogéné, hydroxy(C₁-C₄)alkyle, (C₁-C₄)alcoxy(C₁-C₄)alkyle, (CH₂)ₘNR₇R₈, ou un groupement *tert*-butoxycarbonyle,
- R₇ et R₈ représentent chacun indépendamment l'un de l'autre un substituant choisi parmi H, (C₁-C₄)alkyle, (C₁-C₄)alkyl-OH, (C₃-C₇)cycloalkyle, -(C₃-C₇)cycloalkyl-NH₂, -(C₁-C₄)alkyl-(C₃-C₇)cycloalkyle, C(=NH)NH₂, SO₂(C₁-C₆)alkyle, SO₂-phényle, R₈ peut également représenter un groupe *tert-*butoxycarbonyle ou benzyloxycarbonyle;
- ou R₇ et R₈ ensemble avec l'atome d'azote auquel ils sont liés constituent un radical azétidinyle, pyrrolidinyle, pipéridinyle, pipérazinyle ou morpholinyle, ledit radical étant non substitué ou substitué une ou plusieurs fois par un groupe (C₁-C₆)alkyle, (C₁-C₄)alkyl-OH, COO(C₁-C₆)alkyle, F;
- R₉ représente un atome d'hydrogène ou un groupe (C₁-C₄)alkyle ;
- Ar₂ représente un groupe phényle non substitué ou substitué de 1 à 5 fois par des substituants semblables ou différents choisis parmi un atome d'halogène, un groupe (C₁-C₄)alkyle, trifluorométhyle ou (C₁-C₄)alcoxy ;
- n représente 1, 2 ou 3 ;
- m représente 0, 1, 2 ou 3.

Les composés de formule (I) peuvent comporter un ou plusieurs atomes de carbone asymétrique. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. Lorsque des composés de formule (I) comportent des fonctions acides libres, par exemple carboxylique, sulfonique, phosphonique, ces fonctions acides peuvent être salifiées à l'aide de bases pour former des sels d'addition. De tels sels d'addition font partie de l'invention.

Les sels d'addition à des acides ou à des bases sont avantageusement préparés avec, respectivement, des acides ou des bases pharmaceutiquement acceptables, mais les sels d'autres acides ou de bases utiles, par exemple, pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de la présente invention, on entend par :
- un atome d'halogène : un fluor, un chlore, un brome ou un iode ;
- un groupe alkyle : un groupe aliphatique saturé, linéaire ou ramifié. A titre d'exemple on peut citer les groupes méthyle, éthyle, *n*-propyle, *n*-butyle, *n-*pentyle, *n*-hexyle, *n*-heptyle, 1-méthyléthyle, 1-méthylpropyle, 2-méthylpropyle, 1,1-diméthyléthyle, 1-méthylbutyle, 2-méthylbutyle, 3-méthylbutyle, 1,1-diméthylpropyle, 1,2-diméthylpropyle, 2,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1,1,2-triméthylpropyle, 1,2,2-triméthylpropyle, 1-éthyl,1-méthylpropyle, 1-éthyl,2-méthylpropyle, 1-éthylbutyle, 2-éthylbutyle, 1-méthylhexyle, 2-méthylhexyle, 3-méthylhexyle, 4-méthylhexyle, 5-méthylhexyle, 1,1-diméthylpentyle, 1,2-diméthylpentyle, 1,3-diméthylpentyle, 1,4-diméthylpentyle, 2,2-diméthylpentyle, 2,3-diméthylpentyle, 2,4-diméthylpentyle, 3,3-diméthylpentyle, 3,4-diméthylpentyle, 4,4-diméthylpentyle, 1,1,2-triméthylbutyle, 1,1,3-triméthylbutyle, 1,2,2-triméthylbutyle, 1,2,3-triméthylbutyle, 1,3,3-triméthylbutyle, 2,2,3-triméthylbutyle, 2,3,3-triméthylbutyle, 1,1,2,2-tétraméthylpropyle, 1-éthylpentyle, 2-éthylpentyle, 3-éthylpentyle, 1-éthyl,1-méthylbutyle, 1-éthyl,2-méthylbutyle, 1-éthyl,3-méthylbutyle, 2-éthyl,1-méthylbutyle, 2-éthyl,2-méthylbutyle, 2-éthyl,3-méthylbutyle, 1-propylbutyle, 1-(1-méthyléthyl)butyle, 1-(1-méthyléthyl),2-méthylpropyle.
- un groupe cycloalkyle : cyclopropyle, cyclobutyle, cyclopentyle, cyclohexyle, cycloheptyle, bicyclo[2.2.1]heptyle, cyclooctyle, bicyclo[2.2.2]octyle, bicyclo[3.2.1]octyle, adamantyle.

Parmi les composés de formule (I), on peut citer les composés qui se définissent comme suit :
- R₁ représente un groupe *tert*-butyle, éthyle ou phényle ;
- et/ou Ar₁ représente un radical choisi parmi : 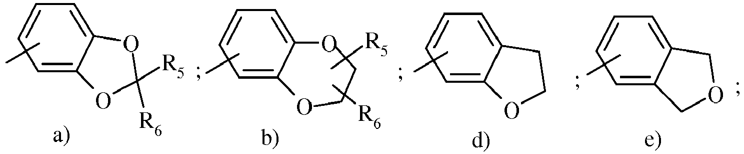
- et/ou R₅ représente un groupement (CH₂)ₙNR₇R₈, CONHNR₇R₈, CONR₇R₈, hydroxy(C₁-C₄)alkyle, ou (CH₂)ₙNR₇COR₈;
- et/ou R₆ représente un atome d'hydrogène, un groupe méthyle ou un groupement (CH₂)ₙNR₇R₈ ou hydroxyméthyle ;
- et/ou Ar₂ représente un groupe aryle substitué par 1 à 2 substituants indépendamment choisis parmi halogène, (C₁-C₄)alkyle, (C₁-C₄)alcoxy ;
- n, m, R₇ et R₈ étant tels que définis précédemment pour un composé de formule (I);
à l'état de base ou de sel d'addition à un acide, ainsi qu'à l'état d'hydrate ou de solvate.

Des produits auront avantageusement un substituant R₅ choisi parmi (CH₂)ₙNR₇R₈, CONR₇R₈, et (CH₂)ₙNR₇COR₈.

Un produit peut être présent sous forme non chirale, ou racémique, ou enrichie en un stéréo-isomère, ou enrichie en un énantiomère ; être éventuellement sous forme solvatée ou hydratée, et être éventuellement salifié.

La présente invention concerne des composés de formule (I) suivante: 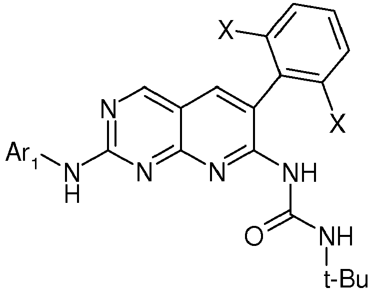 dans laquelle :
- soit Ar₁ représente 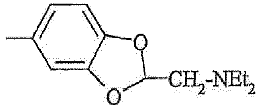 et X représente Cl ;
- soit Ar₁ représente 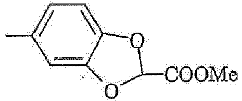 et X représente Cl ;
- soit Ar₁ représente 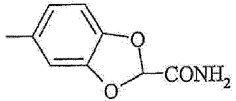 et X représente Cl ;
- soit Ar₁ représente 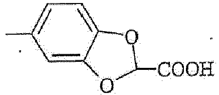 et X représente Cl ;
- soit Ar₁ représente 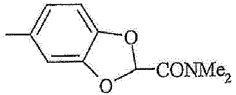 et X représente Cl ;
- soit Ar₁ représente 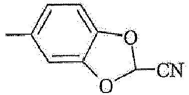 et X représente Cl ;
- soit Ar₁ représente 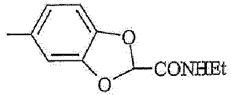 et X représente Cl ;
- soit Ar₁ représente 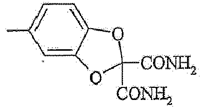 et X représente Cl ;
- soit Ar₁ représente 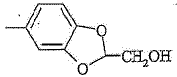 et X représente Cl ;
- soit Ar₁ représente 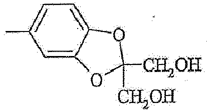 et X représente Cl ;
- soit Ar₁ représente 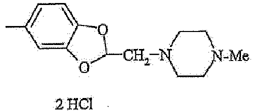 et X représente Cl ;
- soit Ar₁ représente 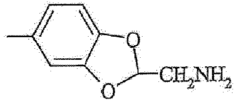 et X représente Cl ;
- soit Ar₁ représente 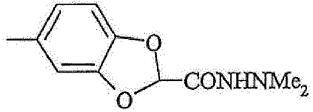 et X représente Cl ;
- soit Ar₁ représente 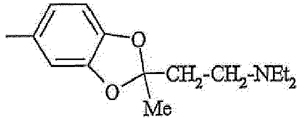 et X représente Cl ;
- soit Ar₁ représente 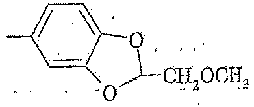 et X représente Cl ;
- soit Ar₁ représente 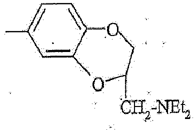 et X représente Cl ;
- soit Ar₁ représente 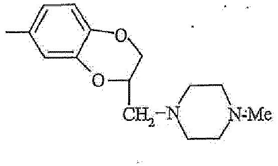 et X représente Cl ;
- soit Ar₁ représente 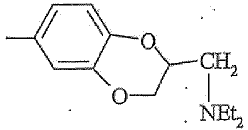 et X représente Cl ;
- soit Ar₁ représente 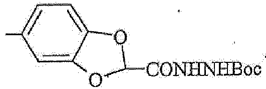 et X représente Cl ;
- soit Ar₁ représente 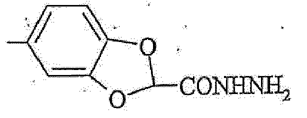 et X représente Cl ;
- soit Ar₁ représente 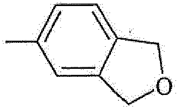 et X représente Cl ;
- soit Ar₁ représente 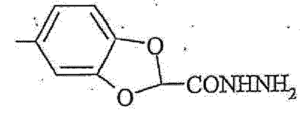 et X représente Cl ;
- soit Ar₁ représente 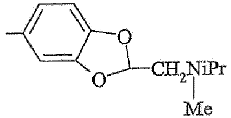 et X représente Cl ;
- soit Ar₁ représente 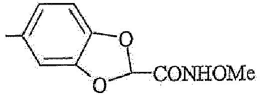 et X représente Cl ;
- soit Ar₁ représente 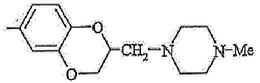 et X représente Cl ;
- soit Ar₁ représente 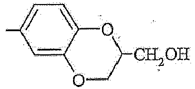 et X représente Cl ;
- soit Ar₁ représente 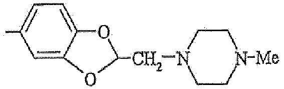 et X représente Me;
- soit Ar₁ représente 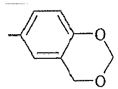 et X représente Cl ;
- soit Ar₁ représente 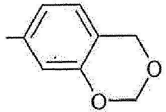 et X représente Cl ;
- soit Ar₁ représente 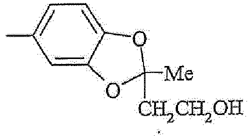 et X représente Cl ;
- soit Ar₁ représente 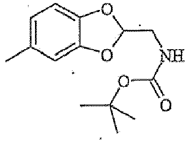 et X représente Cl ;
- soit Ar₁ représente 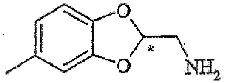 (dextrogyre) et X représente Cl ;
- soit Ar₁ représente 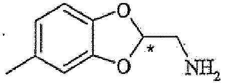 (levogyre) et X représente Cl ;
- soit Ar₁ représente 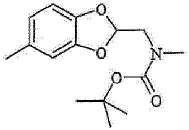 et X représente Cl ;
- soit Ar₁ représente 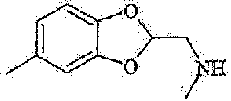 et X représente Cl ;
- soit Ar₁ représente 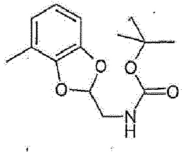 et X représente Cl ;
- soit Ar₁ représente 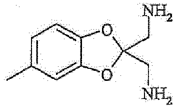 et X représente Cl ;
- soit Ar₁ représente 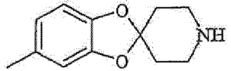 et X représente Cl ;
- soit Ar₁ représente 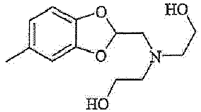 et X représente Cl ;
- soit Ar₁ représente 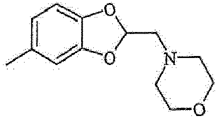 et X représente Cl ;
- soit Ar₁ représente 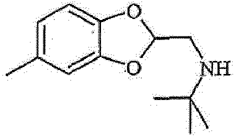 et X représente Cl ;
- soit Ar₁ représente 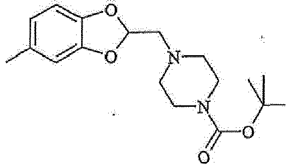 et X représente Cl ;
- soit Ar₁ représente 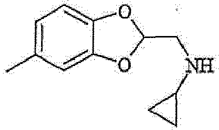 et X représente Cl ;
- soit Ar₁ représente 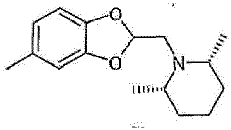 et X représente Cl ;
- soit Ar₁ représente 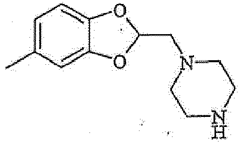 et X représente Cl ;
- soit Ar₁ représente 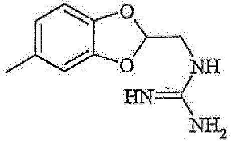 et X représente Cl ;
- soit Ar₁ représente 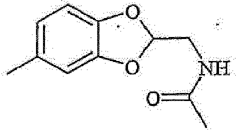 et X représente Cl ;
- soit Ar₁ représente 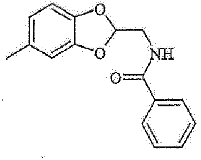 et X représente Cl ;
- soit Ar₁ représente 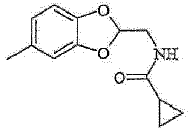 et X représente Cl ;
- soit Ar₁ représente 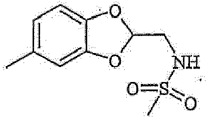 et X représente Cl ;
- soit Ar₁ représente 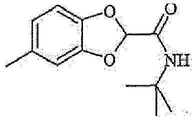 et X représente Cl ;
- soit Ar₁ représente 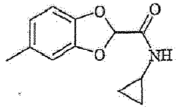 et X représente Cl ;
- soit Ar₁ représente 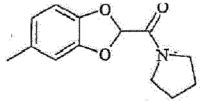 et X représente Cl ;
- soit Ar₁ représente 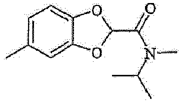 et X représente Cl ;
- soit Ar₁ représente 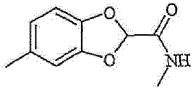 et X représente Cl ;
- soit Ar₁ représente 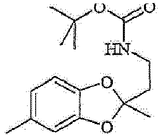 et X représente Cl ;
- soit Ar₁ représente 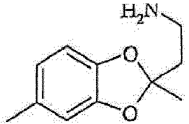 et X représente Cl ;
- soit Ar₁ représente 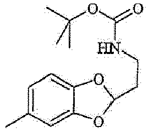 et X représente Cl ;
- soit Ar₁ représente 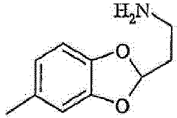 et X représente Cl ;
- soit Ar₁ représente 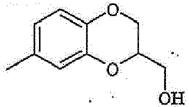 et X représente Cl ;
- soit Ar₁ représente 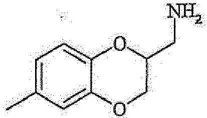 et X représente Cl ;
- soit Ar₁ représente 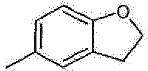 et X représente Cl ;
- soit Ar₁ représente 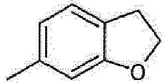 et X représente Cl ;
- soit Ar₁ représente 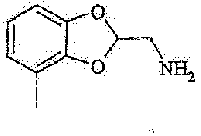 et X représente Cl ;
- soit Ar₁ représente 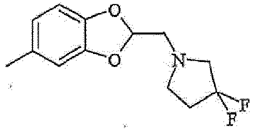 et X représente Cl ;
- soit Ar₁ représente 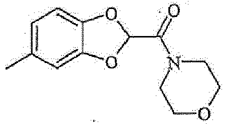 et X représente Cl ;
- soit Ar₁ représente 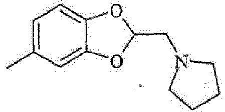 et X représente Cl ;
- soit Ar₁ représente 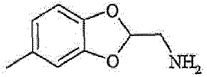 et X représente Br ;
- soit Ar₁ représente 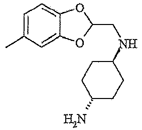 et X représente Cl ;
- soit Ar₁ représente 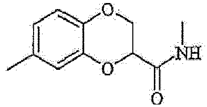 et X représente Cl ;
- soit Ar₁ représente 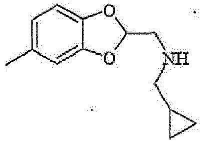 et X représente Cl ;
- L'invention concerne également des composés choisis parmi l'un des composés suivants :

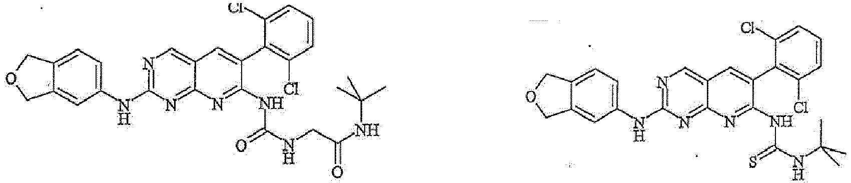

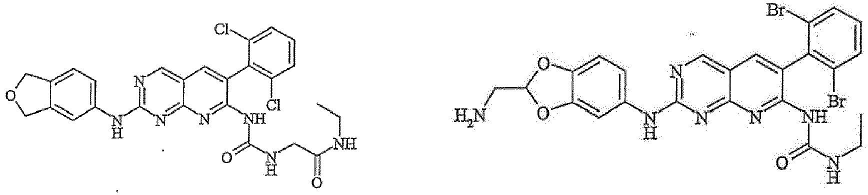

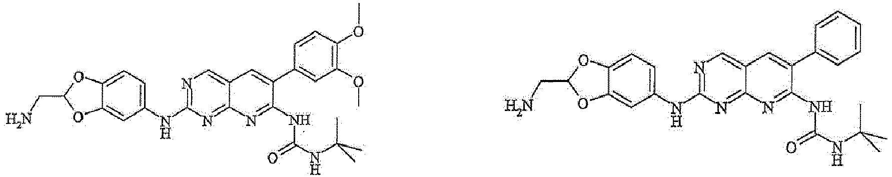

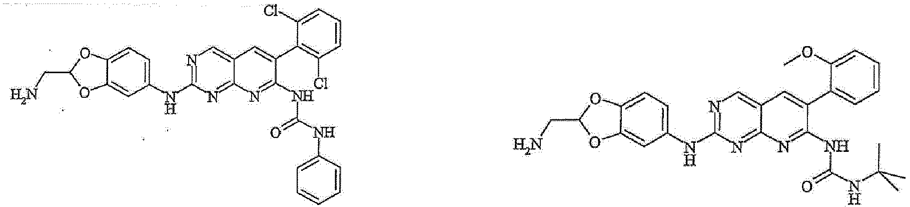

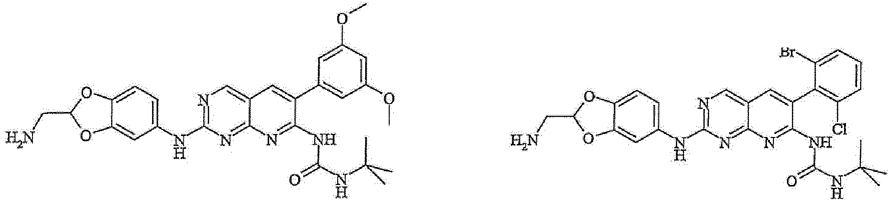

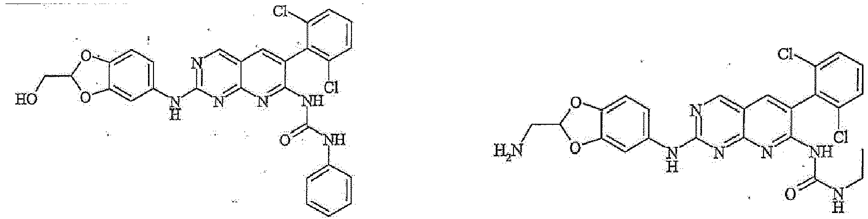

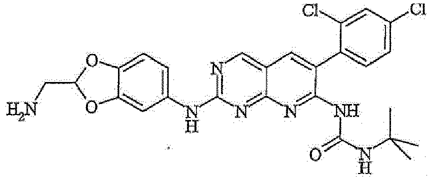

Elle concerne également des composés répondant à la formule suivante : 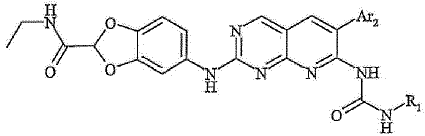 dans laquelle :
- soit R₁ représente Phényle et Ar₂ représente 2,6-dichlorophényle ;
- soit R₁ représente t-Butyle et Ar₂ représente 3,5-diméthoxyphényle ;
- soit R₁ représente éthyle et Ar₂ représente 2,6-dichlorophényle ;
- soit R₁ représente t-Butyle et Ar₂ représente 3,4-diméthoxyphényle ;
- soit R₁ représente t-Butyle et Ar₂ représente phényle ;
- soit R₁ représente t-Butyle et Ar₂ représente 2-méthoxyhényle ;
- soit R₁ représente t-Butyle et Ar₂ représente 2,6-dibromophényle ;
- v soit R₁ représente t-Butyle et Ar₂ représente 2-bromo-6-chlorophényle ;
- soit R₁ représente éthyle et Ar₂ représente 2,6-dibromophényle ;
- soit R₁ représente Phényle et Ar₂ représente 2-bromo-6-chlorophényle ;
- soit R₁ représente Phényle et Ar₂ représente 2,6-dibromophényle ;
ainsi que des composés répondant à la formule suivante : 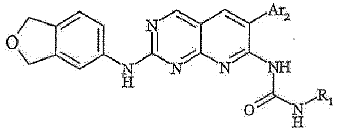 dans laquelle :
- soit R₁ représente Phényle et Ar₂ représente 2,6-dichlorophényle ;
- soit R₁ représente t-Butyle et Ar₂ représente 3,5-diméthoxyphényle ;
- soit R₁ représente éthyle et Ar₂ représente 2,6-dichlorophényle ;
- soit R₁ représente t-Butyle et Ar₂ représente 3,4-diméthoxyphényle ;
- soit R₁ représente t-Butyle et Ar₂ représente phényle ;
- soit R₁ représente t-Butyle et Ar₂ représente 2-méthoxyhényle ;
- soit R₁ représente t-Butyle et Ar₂ représente 2,6-dibromophényle ;
- v soit R₁ représente t-Butyle et Ar₂ représente 2-bromo-6-chlorophényle ;
- soit R₁ représente éthyle et Ar₂ représente 2,6-dibromophényle ;
- soit R₁ représente Phényle et Ar₂ représente 2-bromo-6-chlorophényle ;
   soit R₁ représente Phényle et Ar₂ représente 2,6-dibromophényle.

On décrit également la préparation d'intermédiaires de synthèse utiles pour la préparation de produits selon son premier aspect, lesdits intermédiaires répondant à la formule générale suivante 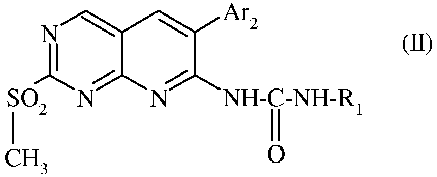 dans laquelle R1 et Ar2 sont tels que définis précédemment.

On décrit également la préparation d'intermédiaires selon son premier et son second aspect, répondant à la formule générale suivante 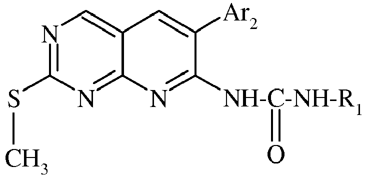 dans laquelle R₁ et Ar₂ sont tels que définis précédemment.

Selon un quatrième aspect, on décrit également la préparation d'intermédiaires selon son premier, son second, et son troisième aspect, répondant à la formule générale suivante 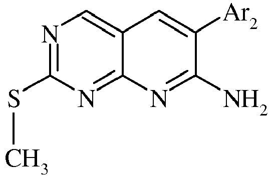 dans laquelle Ar2 est tel que défini précédemment.

Des intermédiaires de synthèse conformes aux second à quatrième aspects comprennent un substituant Ar2 choisi parmi phényl, 2-méthoxyphényl, 2,6-dichlorophényl, 3,5-diméthoxyphényl, 3,4-diméthoxyphényl, 2,6-dibromophényl, 2-bromo-6-chlorophényl, 2,4-dichlorophényl, et 3,5-dichlorophényl.

Des intermédiaires de synthèse conformes aux second et troisième aspects comprennent un substituant R1 est choisi parmi éthyl, tertiobutyl et phényl.

On peut préparer les composés de formule (I) selon un procédé caractérisé en ce que l'on fait réagir :
(i) un composé de formule : 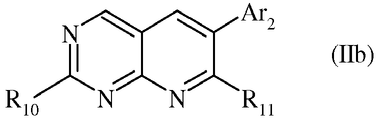 dans laquelle R₁₀ est un groupe partant tel que : (a) halogène, en particulier Cl ou Br, ou (b) alkyl-S(O)m- avec m = 0, 1, ou 2 ; R₁₁ est NHC(=R₁₂)-NH-R₁, avec R₁₂ = O ou S ; et
(ii) une amine de formule Ar'₁NH₂ (III) dans laquelle Ar'₁ représente Ar₁ tel que défini pour (I) ou un précurseur de Ar₁ ; le cas échéant on transforme le groupe Ar'₁ du composé ainsi obtenu en un groupe Ar₁.

Lorsque R₁₀ est halogène ou alkyl-S(O)m- avec m = 2, La réaction est effectuée dans un solvant, de préférence polaire :
(i) par exemple le térahydrofurane, le diméthylsulfoxide ou l'éthanol, éventuellement en présence d'une trace d'acide tel que l'acide chlorhydrique ; ou
(ii) dans le diméthylsulfoxide en présence d'une base forte telle que tBuOK ;
à une température comprise entre la température ambiante et la température de reflux du solvant.

Lorsque R10 est alkyl-S(O)m- avec m = 0 ou 1, on peut effectuer la réaction avec Ar'₁NH₂ (III) à l'état fondu, de préférence à une température voisine de 200° C, sans catalyseur.

Le cas échéant, les fonctions amines présentes dans le groupe Ar'₁ du composé (III) sont préalablement salifiées ou protégées.

Par précurseur de Ar₁, on entend un groupement a), b), c), d) ou e) tel que défini ci-dessus pour (I) dans lequel les substituants R₅ et/ou R₆ sont tels que définis ci-dessus pour (I) ou sont des précurseurs de R₅ et/ou R₆.

Les composés de formule (II) sont préparés en suivant le mode opératoire décrit dans le brevet européen 790 997 et le brevet US 5 733 913, comme décrit dans le Schéma 1 ci-après : 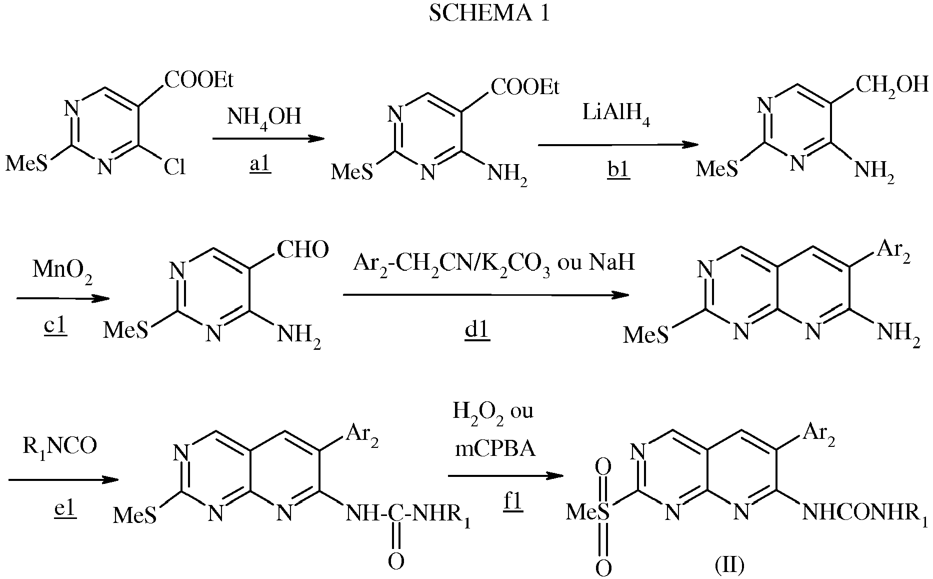 mCPBA : acide *méta*-chloroperbenzoïque.

Les amines de formule (III) sont connues ou préparées par des méthodes connues à partir des dérivés nitrés Ar'₁NO₂ (IV) correspondants, par réduction soit (i) en milieu acide en présence d'un métal tel que le fer ou le zinc en poudre, soit (ii) par l'hydrogène en présence d'un catalyseur tel que Pd/C.

Les composés de formule (IV) sont connus ou préparés par des méthodes connues.

Ainsi, les 5-nitro-1,3-benzodioxoles monosubstitués en 2 par un groupe R₅ = méthoxycarbonyle, peuvent être préparés par action du dichloroacétate de méthyle sur le 4-nitro-catéchol (4-nitrobenzène-1,2-diol).

Les 5-nitro-1,3-benzodioxoles, gem-disubstitués en 2 peuvent être préparés selon Pharmazie, 2003, 58 (1), 13-17 par action du dibromomalonate d'éthyle sur le 4-nitrocatéchol (4-nitrobenzène-1,2-diol).

Les 7-nitro-2,3-dihydro-1,4-benzodioxines substitués en 2 ou 3 par R₅ et R₆ peuvent être préparés selon la méthode décrite dans la demande de brevet WO 01/021 577 à partir du 4-nitrocatéchol ou par des transformations chimiques connues.

La 1,3-dihydro-2-benzofuran-5-amine est décrite dans J. Med. Chem., 1978, 21, 965-978 ; la 4*H*-1,3-benzodioxin-6-amine est décrite dans J. Org. Chem., 1994, 59 (4), 754-757 ; la 4*H*-1,3-benzodioxin-7-amine est décrite dans Chimie Therapeutique, 1972, 7, 443-449.

On opère par des méthodes connues telles que décrites dans March's Advanced Organic Chemistry, 5th Edition, 2005, ISBN 0471585890, pour transformer le groupement R₅ et/ou R₆ des composés de formule (IV), selon les substituants R₅ et/ou R₆ désirés pour les composés de formule (I). On peut également transformer le groupement R₅ et/ou R₆ des composés de formule (I) pour obtenir de nouveaux composés de formule (I) portant les substituants R₅ et/ou R₆ désirés.

Ainsi, le groupe R₅ = CO₂Me permet de préparer des composés de formule (IV) ou (I) dans laquelle R₅ représente un groupement CO₂H, CN, CH₂OH, CONR₇R_{8.} CONHNR₇R₈, CONR₈OR₉, CH₂NR₇R₈, par des méthodes connues de l'homme de l'art.

A partir d'un composé de formule (IV) ou (I) comportant un groupe R₅ = (CH₂)ₙ-OH, avec n = 1, 2, ou 3, on peut préparer un composé de formule (IV) ou (I) dans laquelle R₅ = mésyloxyméthyle par action du chlorure de mésyle, puis un composé de formule (IV) ou (I) dans laquelle R₅ = -CH₂NR₇R₈ par action de HNR₇R₈, R₇ et R₈ étant tels que définis pour les composés de formule (I).

Les composés susmentionnés sont obtenus sous forme racémique ; on peut ensuite préparer les isomères optiquement purs en utilisant des méthodes de dédoublement connues de l'homme de l'art, telle que la cristallisation par formation de sels avec des agents chiraux. On peut également préparer des composés selon l'invention sous forme optiquement pure en utilisant des méthodes de synthèse asymétrique ou stéréospécifique, ou des techniques chromatographiques utilisant une phase chirale. Par ailleurs, les produits de l'invention peuvent être séparés *via* la formation de diastéréoisomères, leur séparation, puis la décomposition du diastéréoisomère pharmacologiquement utile en son produit actif énantiomériquement pur. Des techniques enzymatiques peuvent aussi être employées. Des techniques séparatives supplémentaires connues peuvent être utilisées. Elles incluent celles divulguées dans: Enantiomers, Racemates, and Resolutions, John Wiley and Sons, New York (1981).

Les composés susmentionnés peuvent également être préparés sous forme enrichie en un stéréoisomère dès la préparation des intermédiaires de synthèse. Ainsi, le dédoublement des énantiomères des amines de formule (III) ou des précurseurs nitrés (IV) peut être réalisé par une des méthodes précitées.

Les exemples suivants décrivent la préparation de certains intermédiaires et de composés tels que mentionnés ci-desus. Ces exemples ne sont pas limitatifs et ne font qu'illustrer la présente demande.

Dans les exemples, on utilise les abréviations suivantes :
F : point de fusion
Boc : *tert*-butoxycarbonyle
BOP : benzotriazol-1-yloxytris(diméthylamino)phosphonium hexafluorophosphate.
THF : tétrahydrofurane
TA : température ambiante
DCM : dichlorométhane
MeOH : méthanol.
DCCI : dicyclohexylcarbodiimide
DIPEA :diisopropyléthylamine
KHSO₄ / K₂SO₄ : solution à 5 % de KHSO₄ / K2SO₄
Z : benzyloxycarbonyle

Les spectres de résonance magnétique nucléaires (RMN) du proton sont enregistrés à 200 MHz ou à 250 MHz dans le DMSO-d₆, sauf indication contraire. Le signal DMSO-d₆ est à 2,5 ppm et sert de référence. Pour l'interprétation des spectres, on utilise les abréviations suivantes : s : singulet, d : doublet, t : triplet, m : massif, mt : multiplet, se : singulet élargi, dd : doublet de doublet, qd : quadruplet, qt : quintuplet.

### Préparation d'un composé de formule (II).

### Préparation 1

N-(t-Butyl)-N'-[6-(2,6-dichlorophényl)-2-(méthylsulfonyl)pyrido[2,3-d]pyrimidin-7-yl]urée.

### 1.1 4-Amino-2-(méthylthio)pyrimidine-5-carboxylate d'éthyle.

A une suspension de 50,7 g de 4-chloro-2-(méthylthio)pyrimidin-5-carboxylate d'éthyle dans 400 mL d'EtOH on ajoute en 20 minutes, et en maintenant la température vers 20°C, 140 mL d'une solution NH₄OH à 20%. Après 20 heures d'agitation à température ambiante, le milieu réactionnel est concentré sous vide presque à sec, puis le résidu est repris dans 350 mL d'eau, agité 20 minutes, filtré, lavé avec 3x60 mL d'eau puis est séché sous vide en présence de P₂O₅. On obtient un solide blanc, F = 134-135°C, m = 39,9 g.

### 1.2 [4-Amino-2-(méthylthio)pyrimidin-5-yl]méthanol.

A 39,68 g d'ester obtenu à l'étape précédente dissous dans 1 litre de THF on ajoute en 45 minutes 210 mL d'une solution 1M en LiAlH₄ dans le THF, en maintenant la température inférieure à 30°C. On agite encore 1 heure puis abaisse la température à 5°C et ajoute successivement goutte à goutte 9 mL d'eau, 6,5 mL de soude 5N puis 32 mL d'eau. Après 10 minutes d'agitation, le solide est filtré puis rincé au THF. Le filtrat est concentré sous vide à sec puis le résidu est redissous dans 600 mL de toluène à ébullition, on filtre rapidement à chaud pour éliminer un peu d'insoluble et laisse refroidir une nuit le filtrat. Les cristaux blancs obtenus sont filtrés, lavés avec un peu de toluène puis d'éther et séchés, F = 124-127°C, m = 23,9 g.

### 1.3 4-Amino-2-(méthylthio)pyrimidine-5-carbaldéhyde.

A une suspension de 23,8 g de l'alcool obtenu à l'étape précédente dans 1600 mL de chloroforme, on ajoute en 2 minutes 79,5 g de MnO₂ actif et agite 1 nuit à température ambiante ; le solide est filtré, lavé par 3x75 mL de CHCl₃ et le filtrat concentré sous vide à sec ; le résidu solide blanc est repris dans l'éther, filtré, séché, F = 184-186°C, m = 21,05 g.

### 1.4 6-(2,6-Dichlorophényl)-2-(méthylthio)pyrido[2,3-d]pyrimidin-7-amine.

A 21 g de l'aldéhyde obtenu à l'étape précédente, dissous dans 240 mL de DMF et refroidis à 5°C on ajoute en 5 minutes 5,47 g de NaH 60% puis en 20 minutes, par petites fractions, 29,05 g de 2,6-dichlorophénylacétonitrile. L'agitation est poursuivie 30 minutes à 5°C puis une nuit à température ambiante. Le milieu réactionnel est refroidi à 5°C et on ajoute 65 mL d'une solution saturée en NH₄Cl puis 500 mL d'un mélange eau/glace ; il se forme un précipité rouge qui est filtré, lavé 2 fois à l'eau, essoré au maximum, lavé à l'éther, par 100 mL de chloroforme, puis à l'éther à nouveau ; après séchage on obtient un solide beige, F = 250-253°C, m = 29,92 g.

Les phases éther et chloroforme de lavage sont concentrées à sec, on reprend dans un peu de chloroforme auquel on ajoute de l'éther : on obtient un deuxième jet de 3,15 g, m totale= 33,07 g.

### 1.5 N-(t-Butyl)-N'-[6-(2,6-dichlorophényl)-2-(méthylthio)pyrido[2,3-d]pyrimidin-7-yl]urée.

A 29,9 g de l'amine obtenue ci-dessus en solution dans 300 mL de DMF, on ajoute en 10 minutes et en maintenant la température inférieure à 25°C, 4,6 g de NaH à 60% ; on agite encore 20 minutes puis ajoute en 20 minutes 12,2 mL d'isocyanate de tertiobutyle puis agite une nuit. Le milieu réactionnel est versé lentement sur 800 mL d'un mélange eau/glace +100 mL d'HCl 6N ; le précipité formé est filtré, lavé à l'eau, essoré puis agité 1 heure dans 300 mL d'éther, puis filtré, lavé à l'éther et séché. On obtient un solide beige, F = 195-196°C (dec.), m = 26,5 g.

### 1.6 N-(t-Butyl)-N'-[6-(2,6-dichlorophényl)-2-(méthylsulfonyl)pyrido[2,3-d]pyrimidin-7-yl]urée.

A 21,95 g d'urée obtenue ci dessus en solution dans 300 mL de chloroforme, on ajoute en 25 minutes, et en maintenant la température inférieure à 25°C, 27 g d'acide métachloroperbenzoïque. Il se forme un précipité. Après 2 heures le milieu réactionnel est dilué par 1 litre de dichlorométhane et on ajoute du Na₂SO₄, puis 14 g de Ca(OH)₂ ; après 30 minutes d'agitation le solide est filtré, lavé par du dichlorométhane puis le filtrat concentré à sec ; le résidu est trituré dans 80 mL d'éther à chaud ; on laisse refroidir puis le solide blanc est filtré, lavé avec de l'éther et séché, F = 138-140°C, m= 20,5 g.

De la même manière que pour le composé décrit à la préparation 1, on peut préparer les composés de formule générale (II) suivants:

| **Ar2** | **R1** | **RMN** |
|---|---|---|
| **2,6-dichlorophényl** | ***tert-*butyl** | **1,40: s: 9H; 3,50: s: 3H; 7,50-7,70: m: 3H; 8,55: s: 1H; 9,10: s: 1H; 9,60: s: 1H; 9,95: s: 1H.** |
| 2,6-dichlorophényl | phényl | 3,50 ppm : s : 3H ; 7,10 ppm : t : 1H ; 7,40 ppm : t : 2H ; 7,55-7,75 ppm : m : 5H ; 8,60 ppm : s : 1H ; 9,60 ppm : s : 1H ; 9,80 ppm : s : 1H ; 11,90 ppm : s : 1H. |
| 3,5-diméthoxyphényl | *tert-*butyl | 1,40 ppm : s : 9H ; 3,50 ppm : s : 3H ; 3,80 ppm : s : 6H ; 6,65-6,80 ppm : mt : 3H ; 7,75 ppm : s : 1H ; 8,45 ppm : s : 1H ; 9,60 ppm : s : 1H ; 9,80 ppm : s : 1H. |
| 2,6-dichlorophényl | éthyl | 1,20 ppm : t : 3H ; 3,40 ppm : qd : 2H ; 3,50 ppm : s : 3H ; 7,50 ppm - 7,75 ppm : m : 3H ; 8,55 ppm : s : 1H ; 9,40 ppm : s : 1H ; 9,60 ppm : s : 1H ; 9,70 ppm : s : 1H. |
| 3,4-diméthoxyphényl | *tert-*butyl | 1.40 ppm : s : 9H ; 3.45 ppm : s : 3H ; 3.80 ppm : s : 3H ; 3.90 ppm : s : 3H ; 7.10 - 7,20 ppm : m : 3H ; 7.75 ppm : s : 1H ; 8.45 ppm : s : 1H ; 9.55 ppm : s : 1H ; 9.80 ppm : s : 1H. |
| Phényl | *tert-*butyl | 1,40 ppm : s : 9H ; 3,50 ppm : s : 3H ; 7,60 ppm : se : 6H ; 8,45 ppm : s : 1H ; 9,40 ppm : s : 1H ; 9,80 ppm : s : 1H. |
| 2-méthoxyphényl | *tert-*butyl | 1,40 ppm : s : 9H ; 3,.50 ppm : s : 3H ; 3,80 ppm : s : 3H ; 7,10 - 7,40 ppm : mt : 4H ; 7,60 ppm : t : 1H ; 8,40 ppm : s : 1H ; 9,60 ppm : s : 1H ; 9,80 ppm : s : 1H. |
| 2,6-dibromophényl | *tert-*butyl | 1,40 ppm : s : 9H ; 3,50 ppm : s : 3H ; 7,40 ppm : t : 1H ; 7,85 ppm : d : 2H ; 8,50 ppm : s : 1H ; 9,00 ppm : s : 1H ; 9,60 ppm : s : 1H ; 10,00 ppm : s : 1H. |
| 2-bromo-6-chlorophényl | *tert-*butyl | 1,40 ppm : s : 9H ; 3,45 ppm : s : 3H ; 7,50 ppm : t : 1H ; 7,65 ppm : d : 1H ; 7,80 ppm : d : 1H ; 8,50 ppm : s : 1H ; 9,00 ppm : s : 1H ; 9,50 ppm : s : 1H ; 9,90 ppm : s : 1H. |
| 2,6-dibromophényl | éthyl | 1,15 ppm : t : 3H ; 3,30 ppm : qd : 2H (masqué par DOH) ; 3,50 ppm : s :3H ; 7,40 ppm : t : 1H ; 7,85 ppm : d : 2H ; 8,50 ppm : s : 1H ; 9,25 ppm : s : 1H ; 9,60 ppm : s : 1H ; 9,70 ppm : s : 1H. |
| 2-bromo-6-chlorophényl | Phényl | (DMSO + TFA) 3,55 ppm : s : 3H ; 7,10 ppm : t : 1H ; 7,30 - 7,90 ppm : m : 7H ; 8,60 ppm : s : 1H ; 9,65 ppm : s : 1H. |
| 2,6-dibromophényl | Phényl | 3,55 ppm : s : 3H ; 7,10 ppm : t : 1H ; 7,35 ppm : qd : 3H ; 7,60 ppm : d : 2H ; 7,85 ppm : d : 2H ; 8,60 ppm : s : 1H ; 9,70 ppm : s : 1H ; 9,80 ppm : s : 1H ; 12,00 ppm : s : 1H. |
| 2,4-dichlorophényl | *tert-*butyl | 1,35 ppm : s : 9H ; 3,50 ppm : s : 3H ; 7,45 - 7,60 ppm : mt : 2H ; 7,80 ppm : s : 1H ; 8,40 ppm : s : 1H ; 8,80 ppm : s : 1H ; 9,55 ppm : s : 1H ; 9,80 ppm : s : 1H. |

### Préparation des composés de formule (III).

Les numéros de préparations utilisés renvoient aux numéros des composés des tableaux 1 et 2 ci-après. Lorsqu'ils contiennent un carbone asymétrique, ces composés sont obtenus sous forme racémique, sauf indication contraire.

### Préparation 2

### 2.1 5-Nitro-1,3-benzodioxole-2-carboxylate de méthyle.

A 17,6 g de NaH 60 % en suspension dans 300 mL de DMF, on ajoute en 1 heure, 31,0 g de 4-nitrocatéchol en refroidissant pour maintenir la température inférieure à 30°C. On agite encore 15 minutes puis additionne en 1 heure, 104 mL de dichloroacétate de méthyle puis on agite 4 heures à 90°C. Le milieu réactionnel est versé sur un mélange de 2 litres de glace/eau, puis on extrait 4 fois par 400 mL d'AcOEt. Les phases organiques jointes sont lavées 1 fois par une solution NaCl saturée puis séchées et concentrées sous vide (évaporation de DMF). Le résidu est repris dans un mélange AcOEt/H₂O et on amène à pH = 8,6 par Na₂CO₃ ; la phase organique est décantée, lavée par NaHCO₃ saturée, H₂O, KHSO₄/K₂SO₄ 5%, H₂O, NaCl saturée puis séchée et évaporée sous vide ; on obtient un résidu semi-solide qui est repris puis trituré dans l'heptane pour donner un solide, m = 27,7 g, F = 90-92°C .

### 2.2 5-Amino-1,3-benzodioxole-2-carboxylate de méthyle.

A 900 mg d'ester de l'étape ci-dessus, dissous dans 30 mL de THF, on ajoute 3,92 g de zinc en poudre et, après refroidissement à -5°C, on ajoute en 30 minutes 4 mL d'acide acétique dilué par 4 mL de THF puis on laisse remonter la température. Après 1 heure et demie, on filtre, lave le solide par un peu de THF et méthanol. Le filtrat est dilué par AcOEt et on lave par H₂O, NaHCO₃ saturée, H₂O, NaCl saturée ; après séchage et concentration sous vide on obtient une cire jaune identifiée par RMN, m = 800 mg.

### Préparation 3

### 3.1 5-Nitro-1,3-benzodioxole-2-carboxamide.

Sur 1,12 g d'ester méthylique de la Préparation 2.1, on verse 20 mL d'une solution d'ammoniac 2M dans le méthanol. Après 25 minutes, on concentre sous vide, on reprend le résidu solide dans Et₂O, filtre et sèche, m = 0,99 g, F = 202-207°C.

### 3.2 5-Amino-1,3-benzodioxole-2-carboxamide.

A 0,98 g d'amide de la Préparation 3.1 dans 35 mL de THF, on ajoute 4,57 g de zinc en poudre ; après refroidissement à -5°C, on ajoute en 30 minutes, 5 mL d'acide acétique dilué dans 5 mL de THF. A la fin de l'addition, on laisse remonter la température. Après 1 heure le solide est filtré, lavé avec un peu de THF, méthanol, AcOEt. Le filtrat est dilué par AcOEt, on y ajoute de l'eau et on amène à pH = 6 par NaHCO₃ saturée. Le précipité formé est éliminé par filtration, le filtrat est décanté puis la phase organique lavée par NaHCO₃ saturée, H₂O, NaCl saturée, séchée et évaporée, on obtient une cire qui durcit au froid, m = 0,63 g.

### Préparation 4

### 4.1 5-Nitro-(1,3-benzodioxol-2-yl)méthanol.

A 5,02 g d'ester méthylique, obtenu à la préparation 2.1, dissous dans 25 mL de THF, on ajoute à -5°C en 1 heure 15 minutes 22,3 mL d'une solution LiAlH₄ 1M dans le THF ; 20 minutes après la fin de l'addition, on ajoute, goutte à goutte, 20 mL d'AcOEt puis 9 mL NaOH 1N ; le précipité formé est éliminé par filtration, lavé par AcOEt ; le filtrat est dilué par AcOEt et on lave par H₂O, KHSO₄K₂SO₄ 5%, H₂O, NaCl saturée ; après séchage et concentration sous vide on obtient une cire qui cristallise, m = 2,74 g, F = 80-82°C.

A l'étape suivante, le dérivé nitro de la Préparation 4.1 est réduit selon les méthodes décrites ci-dessus pour obtenir l'amine de formule (III) de la Préparation 4.2.

### Préparation 5

### 5.1 (5-Nitro-1,3-benzodioxol-2-yl)méthylsulfonate.

A 4,12 g d'alcool, obtenu à la préparation 4.1, dissous dans 30 mL CH₂Cl₂, on ajoute à 5°C, 3 mL de triéthylamine puis en 15 minutes, 1,85 g de chlorure de mésyle. Après 15 minutes, on enlève le bain de glace. Après 55 minutes, le milieu réactionnel est dilué par du CH₂Cl₂ et de l'eau ; la phase organique est décantée, lavée par H₂O, NaCl saturée, séchée, évaporée. Après trituration dans l'heptane, on obtient un solide marron, m = 5,20 g, F = 112-115°C.

### 5.2 [(5-Nitro-1,3-benzodioxol-2-yl)methyl]diéthylamine.

A 2,91 g de mésylate obtenu à l'étape ci-dessus dans 18 mL de DMF, on ajoute 2,19 g de diéthylamine et chauffe à 80°C. On rajoute 0,73 g de diéthylamine après 15 heures puis encore 0,73 g après 8 heures. Après 48 heures au total, le milieu réactionnel est dilué par AcOEt, lavé par H₂O puis NaCl saturée ; après séchage AcOEt est évaporé puis le résidu est repris dans 40 mL Et₂O + 10 mL AcOEt et extrait 2 fois par 60 mL HCl 0,25 N ; les phases acides sont mélangées, mises au contact d'AcOEt et on amène à pH = 9 par NaOH 10N ; la phase organique est décantée, lavée par H₂O puis NaCl saturée, puis séchée et évaporée. On obtient une huile m=1,55 g.

### 5.3 [(5-Amino-1,3-benzodioxol-2-yl)méthyl]diéthylamine.

A 1,93 g de composé nitro obtenu à l'étape ci-dessus, dissous dans 70 mL THF, on ajoute 7,45 g de zinc en poudre puis à -5°C, on additionne en 25 minutes 7,6 mL d'AcOH puis l'agitation est poursuivie entre 0 et 5°C. Après 1 heure et demie, le solide est filtré, lavé par du THF et un peu de méthanol ; le filtrat est dilué par AcOEt + H₂O et on amène à pH = 9 par NaOH 10N ; le précipité formé est éliminé par filtration ; le filtrat est décanté ; la phase organique lavée par H₂O, NaCl saturée, séchée, évaporée, on obtient une huile noire, m = 1,75 g

### Préparation 6

### 6.1 1-Méthyl-4-((5-nitro-1,3-benzodioxol-2-yl)méthyl)pipérazine.

Réaction selon le mode opératoire 5.2, produit isolé sous forme de dichlorhydrate, 6.2 2-((Méthylpipérazin-1-yl)méthyl)-1,3-benzodioxol-5-amine.

### Réaction selon le mode opératoire 5.3

### Préparation 7

### 7.1 Acide 5-nitro-1,3-benzodioxol-2-carboxylique.

A 1,12 g d'ester obtenu à la préparation 2.1 dans 12 mL de méthanol, on ajoute en 30 minutes 1,5 mL de soude 5N. 35 minutes après la fin de l'addition le milieu réactionnel est dilué par AcOEt et H₂O, et on amène à pH = 2 par HCl 2N ; la phase organique est décantée, lavée par H₂O et NaCl saturée, puis on sèche puis on évapore, on obtient 1,25 g d'huile.

### 7.2 N,N-Diméthyl-5-nitro-1,3-benzodioxol-2-carboxamide.

A 0,84 g d'acide obtenu à l'étape ci-dessus, dissous dans 15 mL de dichlorométhane, on ajoute 0,36 g de chlorhydrate de diméthylamine, 0,77 mL de DIPEA puis 0,91 g de DCCI. Après 3 heures d'agitation à température ambiante, le milieu réactionnel est filtré puis le filtrat est dilué par CH₂Cl₂ que l'on lave successivement par une solution NaHCO₃ saturée, H₂O, KHSO₄/K2SO₄ 5%, H₂O, NaCl saturée, après séchage, le solvant est évaporé puis le résidu chromatographié sur silice par un mélange dichlorométhane/méthanol 99/1. On obtient 0,5 g de produit solide F=109°C.

### Préparation 9

### 9.1 5-Nitro-1,3-benzodioxol-2-carbonitrile.

A 45 mL de DMF refroidi à 5°C, on ajoute lentement 3,7 mL de POCl₃. Après 30 minutes d'agitation à 5°C, on ajoute en 1 fois 1,67 g d'amide obtenu à la préparation 3.1. Après 3 heures d'agitation à température ambiante, le mélange réactionnel est versé sur un mélange de 250 mL eau/glace. Le précipité formé est filtré, lavé à l'eau puis est séché, m = 1,32 g, F = 105-110°C.

### 9.2 5-Amino-1,3-benzodioxol-2-carbonitrile.

La réduction du NO₂ du produit obtenu à l'étape 9.1 en NH₂ est réalisée selon la méthode décrite précédemment, en mettant en oeuvre un mélange Zn/AcOH.

### Préparation 11

### 11.1 5-Nitro-1,3-benzodioxol-2,2-dicarboxylate d'éthyle.

Ce composé est préparé selon le mode opératoire décrit dans Pharmazie 2003 58 (1) 13-17

### 11.2 5-Nitro-1,3-benzodioxol-2,2-dicarboxamide.

1,24 g de diester obtenu à l'étape précédente sont ajoutés en 1 fois à 14 mL d'une solution d'ammoniac 2M dans le méthanol. Après 30 minutes d'agitation, on concentre à sec le milieu réactionnel puis on reprend le résidu solide dans l'éther, filtre et sèche, m = 1,01 g, F = 231-233°C

### Préparation 12

### 12.1 (5-Nitro-1,3-benzodioxol-2,2-diyl)diméthanol.

A 1,87 g de diester obtenu à la Préparation 11.1 dans 60 mL de THF, on ajoute à température ambiante 1,50 g de NaBH₄ en 1 heure ; 25 minutes après la fin de l'addition on dilue par 250 mL d'AcOEt puis 5 mL de méthanol puis 40 mL d'eau goutte à goutte. La phase organique est décantée, lavée par H₂O, une solution KHSO₄/K₂SO₄ 5%, puis H₂O, une solution NaCl saturée. Après séchage et évaporation le résidu est chromatographié sur silice avec un mélange chloroforme /méthanol (98/2) on obtient 0,64 g d'huile qui prend en masse, F = 111-113°C.

### Préparation 13

### 13.1 3-(2-Méthyl-5-nitro-1,3-benzodioxol-2-yl)propanoate d'éthyle.

A 15,51 g de 4-nitrocatéchol en suspension dans 22,10 g d'acétoacétate d'éthyle, on ajoute à 70°C, en 15 minutes, 22,4 g d'anhydride phosphorique. Après 1 heure 45 minutes le milieu réactionnel est refroidi puis extrait 4 x 150 mL de toluène tiède. Les phases toluèniques sont jointes, lavées par H₂O, NaOH 1N, H₂O, KHSO₄/K₂SO₄ 5%, H₂O, une solution de NaCl saturée. Après séchage et évaporation, le produit est purifié par chromatographie sur silice en éluant avec du chloroforme on obtient 2,44 g de solide, F = 76-78°C.

### 13.2 3-(2-Méthyl-5-nitro-1,3-benzodioxol-2-yl)propan-1-ol.

A 2,33 g de l'ester obtenu ci-dessus dissous dans 40 mL de THF on ajoute à -5°C, en 45 minutes, 8 mL de LiAlH₄ 1M dans le THF. Après 35 minutes, 8 mL d'acétate d'éthyle sont ajoutés goutte à goutte puis 1 mL d'eau puis 1 mL de soude 1N. Le solide est éliminé par filtration ; le filtrat est dilué par AcOEt, lavé par H₂O, KHSO₄/K₂SO₄ 5%, H₂O, NaCl saturée ; après séchage la phase organique est concentrée sous vide ; on obtient une huile m =1,90 g.

### 13.3 3-(2-Méthyl-5-nitro-1,3-benzodioxol-2-yl)propylméthanesulfonate.

A 1,89 g d'alcool obtenu précédemment à l'étape 13.2, dans 40 mL de dichlorométhane à 5°C, on ajoute 1,01 g de triéthylamine puis 1,14 g de chlorure de mésyle en 20 minutes. A la fin de l'addition le bain de glace est enlevé et l'agitation poursuivie 1 heure ; le milieu réactionnel est dilué par CH₂Cl₂ et on lave par H₂O, puis une solution de NaCl saturée ; après séchage le solvant est évaporé ; on obtient 2,46 g de cire qui solidifie à froid.

### 13.4 N,N-Diéthyl-3-(2-méthyl-5-nitro-1,3-benzodioxol-2-yl)propan-1-amine.

A 1,21 g de mésylate dans 20 mL de DMF on ajoute 0,87 g de diéthylamine puis on chauffe à 80°C. Après 8 heures et demie, on rajoute 0,44 g de diéthylamine et poursuit le chauffage 14 heures. Le milieu réactionnel est concentré sous vide, le résidu redissous dans AcOEt et on amène à pH = 9,5 par NaOH 1N, la phase organique est décantée, lavée par H₂O, une solution de NaCl saturée, puis séchée, après évaporation on obtient le produit brut qui est redissous dans 20 mL AcOEt plus 20 mL Et₂O puis on extrait 2 fois par 50 mL HCl 0,5 N ; les 2 phases aqueuses sont mélangées, mises au contact d'AcOEt et on amène à pH = 9 par NaOH 10 N ; la phase organique est décantée, relavée par H₂O puis par une solution de NaCl saturée, séchée, évaporée, on obtient 0,66 g d'huile.

### 13.5

Le substituant NO₂ du produit obtenu à l'étape 13.4 est réduit en NH₂ par la méthode décrite précédemment utilisant un mélange Zn/AcOH pour l'obtention d'un produit réduit 13.5.

### 13.6

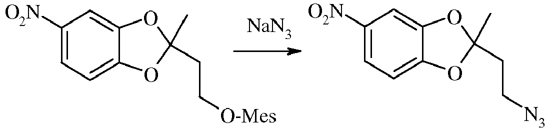

A 2,00 g du produit obtenu à la préparation 13.3 dans 20 mL de DMF, on ajoute 0,85 g d'azoture de sodium et agite 5 jours à température ambiante, extraction à l'éther et lavage de la phase organique à l'eau puis avec une solution NaCl saturée. On obtient une huile m = 1,50 g. RMN conforme.

### 13.7

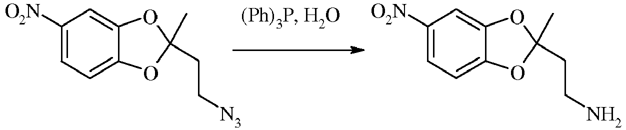

Un mélange de 1,49 g du produit obtenu à la préparation 13.6, 1,71 g de triphénylphosphine et 0,12 g d'eau dans 25 mL THF est agité pendant 24 heures. Le milieu réactionnel est ensuite extrait par AcOEt et lavé à l'eau. Le produit brut obtenu est dissous dans un mélange AcOEt/Et₂O et extrait par une solution aqueuse de HCl 1N. La phase aqueuse acide est mise au contact d'AcOEt et on amène à pH 9 par NaOH 10N. La phase organique est isolée, lavée par de l'eau, puis une solution saturée en NaCl. La phase organique est concentrée sous pression réduite pour obtenir 1,10 g d'huile.

### 13.8

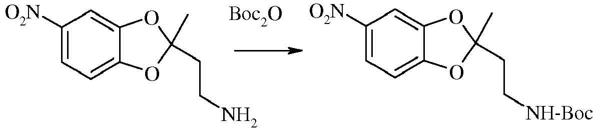

1,09 g de l'amine obtenue à l'étape 13.7 est dissous dans 10 mL de dichlorométhane (DCM) puis on ajoute 0,20 g de triéthylamine puis 1,18 g de Boc₂O. Après 5H, le milieu réactionnel est dilué par DCM puis est lavé par, successivement, une solution à 5% KHSO₄/K₂SO₄, de l'eau, une solution saturée en NaCl. Après séchage et évaporation sous pression réduite de la phase organique isolée, on obtient 1,36 g d'huile.

### 13.9 (précurseur de l'exemple 55)

1,35 g du produit obtenu à l'étape 13.8 est traité par Zn/AcOH comme décrit à la préparation 2.2 pour réduire le groupe NO₂ en NH₂. On obtient 1,13 g de cire.

### Préparation 14

### 14.1 2-(2-Méthoxyméthyl)-5-nitro-1,3-benzodioxole.

1,45 g d'hydroxyméthyle obtenu à la préparation 4.1 sont dissous dans 25 mL de THF; après refroidissement à 5°C addition, par petites fractions, de 353 mg NaH à 60 % ; après 30 minutes addition de 0,92 mL d'iodure de méthyle puis agitation 1 nuit à température ambiante ; 1 mL d'iodure de méthyle est ajouté et l'agitation est poursuivie pendant 5 heures ; 30 mL de solution NH₄Cl saturée sont ajoutés au milieu réactionnel puis de l'eau et de l'acétate d'éthyle ; la phase organique est décantée, relavée par H₂O puis NaCl saturée, séchée et évaporée. Le brut est chromatographié sur silice en éluant par CHCl₃/heptane 9/1. On obtient 595 mg d'huile identifiée par RMN.

### Préparation 16

### 16.1 2-((5-Nitro-1,3-benzodioxol-2-yl)carbonyl)hydrazinecarboxylate de tert-butyle.

Un mélange de 900 mg d'ester (Préparation 2.1) et 2,114 g de tertiobutylcarbazate dans 40 mL de méthanol est chauffé à 60°C pendant 60 heures ; on ajoute 600 mg de tertiobutylcarbazate et chauffe encore 3 heures. Le méthanol est évaporé, le résidu est repris dans de l'acétate d'éthyle et lavé par de l'eau, de l'acide chlorhydrique 0,2N, une solution saturée de bicarbonate de sodium, de l'eau et une solution saturée de NaCl. On obtient une cire qui se prend en masse, m = 1,27 g.

### 16.2 2-((5-Amino-1,3-benzodioxol-2-yl)carbonyl)hydrazinecarboxylate de tert-butyle.

A 1,30 g de produit tel qu'obtenu à l'étape précédente, dissous dans 25 mL de THF, on ajoute 3,92 g de zinc en poudre puis à -5°C 4,8 g d'acide acétique en 30 minutes ; on enlève le bain de glace et poursuit l'agitation 2 heures ; le solide est filtré, lavé avec un peu de THF puis par AcOEt ; on ajoute de l'eau au filtrat et amène à pH 6,5 par une solution à 15 % de Na₂CO₃ ; l'AcOEt est décanté, lavé par NaHCO₃ saturée, H₂O, NaCl saturée, séché et évaporé. On obtient le composé attendu sous forme d'une l'huile noire, m = 1,12 g.

### Préparation 21

### 21.1

A 2,14 g de mésylate obtenu à l'étape 5.1 dissous dans 17 mL de DMF, on ajoute 1,51 g d'azoture de sodium et on chauffe à 70°C pendant 3 heures. Le milieu réactionnel est extrait par AcOEt que l'on lave à l'eau puis par une solution saturée de NaCl. On obtient une huile.
m = 1,71 g.

### 21.2

A 1,7 g du produit obtenu à l'étape 21.1, dissous dans 20 mL AcOEt, on ajoute par petites portions 3,41 g de triphénylphosphine puis après 10 minutes, 2,34 mL d'eau et on chauffe à 60° C. Après 1heure, le milieu réactionnel est évaporé à sec, puis repris dans Et₂O. Les insolubles sont éliminés puis une solution d'HCl saturé dans l'éther est ajoutée en excès. Le solide formé est filtré, lavé à l'éther puis séché, pour l'obtention du produit attendu sous forme de chlorhydrate. L'amine correspondante est obtenue par libération du chlorhydrate.

### 21.3

Le produit obtenu à l'étape 21.2 peut être séparé en ses deux énantiomères :
A 2 g de l'amine obtenue à l'étape 21.2 dissoute dans 70 mL d'eau et 7 mL de dioxane à 70°C, sont ajoutés 1,97 g d'acide (S)(+)mandélique. Le milieu réactionnel est ensuite laissé revenir doucement à 30°C sous agitation magnétique. Le précipité obtenu est filtré, dissous dans 40 mL d'eau et 4 mL de dioxane à 70°C, puis est laissé revenir doucement à 30°C sous agitation. Le solide qui se forme lors du refroidissement est filtré et séché. m = 0,49 g. Le produit ainsi obtenu est repris dans 20 mL d'eau et 100 mL d'AcOEt puis est amené à pH = 9,5 par ajout de NaOH 1N. Le mélange est décanté, la phase organique est isolée, lavée plusieurs fois par de l'eau, puis par une solution saturée de NaHCO₃, par de l'eau, et enfin par une solution saturée en NaCl. La phase organique est collectée, séchée et le solvant est évaporé sous pression réduite. On obtient 0,27g d'une cire qui durcit progressivement. [α]_{D} = +94,7° , à 25°C; C = 0,5 (MeOH); pureté optique: HPLC chirale: 96/4 (pouvoir rotatoire de l'énantiomère purifié à 100% = 102°).

### 21.4

Au produit obtenu à l'étape 21.2 dans 30 mL de DCM, on ajoute 1,49 mL de triéthylamine puis par petites portions 2,53 g de Boc₂O. Après 1 heure, le milieu réactionnel est lavé par KHSO₄/K₂SO₄ 5%, H₂O, NaCl saturé. Après séchage, la phase organique est concentrée à sec puis le résidu est trituré dans l'heptane ; on obtient 2 g de solide.

### 21.5

A 480 mg de NaH 60 % dans 20 mL de THF, on ajoute à 5°C en 30 minutes, 1,79 g du produit obtenu à l'étape 21.4 dissous 15 mL de THF. Le mélange est agité 45 minutes à température ambiante puis 1,2 mL d'iodométhane est additionné en 10 minutes. Après 3 heures, le milieu réactionnel est versé sur 60 mL d'une solution aqueuse saturée en acide citrique puis extrait à l'acétate d'éthyle. La phase organique est isolée, lavée à l'eau, puis par une solution saturée en NaCl, séchée et évaporée à sec. Le produit brut obtenu est purifié par chromotographie flash avec un gradient de dichlorométhane dans le cyclohexane. On obtient un solide blanc : m = 1,4 g.

### 21.6

Le produit issu de la préparation 21.5 est réduit selon la méthode habituelle par Zn/AcOH.

### Préparation 22

### 22.1

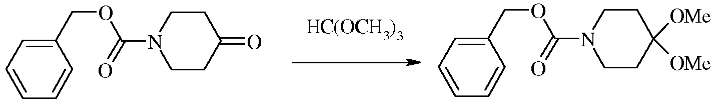

Un mélange de 11,65 g de 4-N-Z-pipéridone, 6,35 g d'orthoformiate de méthyle et 40 mg d'acide paratoluènesulfonique est chauffé dans un Claisen à 60°C pendant 1heure puis à 70°C pendant 1heure, en laissant distiller le formiate de méthyle. Le résidu est dilué par AcOEt + H₂O et on ajoute quelques gouttes de soude 1N pour amener à pH 7. La phase organique est décantée, isolée, lavée à l'eau puis par une solution saturée en NaCl, séchée, évaporée. On obtient 14 g d'huile incolore.

### 22.2

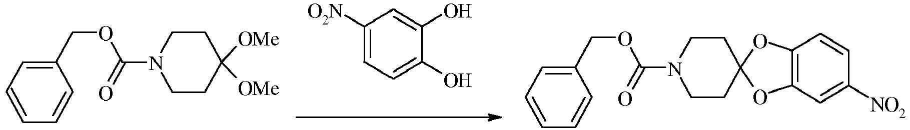

16,09 g de cétal obtenu à l'étape 22.1, 10,80 g de 4-nitrocatéchol et 60 mg d'acide paratoluènesulfonique sont chauffés dans 120 mL de toluène avec lente distillation du toluène. Après 4h30 le milieu réactionnel est dilué par du toluène, refroidi ; on élimine par filtration l'insoluble, lave le filtrat par NaOH 1N, H₂O, une solution KHSO₄/K₂SO₄ 5 %, H2O, NaCl saturée. Après séchage et évaporation, le produit brut est chromatographié sur silice, éluant CHCl₃/AcOEt 98/2. On obtient 0,65 g de produit attendu.

### 22.3

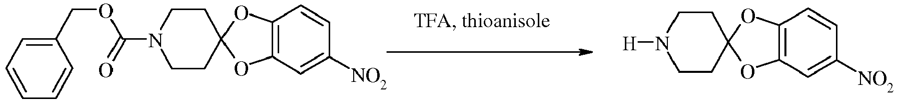

A 0,60 g de produit obtenu ci-dessus dans 5 mL d'acide trifluoroacétique, on ajoute 0,5 mL de thioanisole. Après 3H le milieu réactionnel est concentré sous vide ; le résidu est repris dans CH₂Cl₂ avec H₂O et on amène à pH 9 par NaOH 1N. Après décantation, la phase organique est relavée par de l'eau puis par une solution saturée de NaCl. La phase organique est séchée et évaporée. Le produit brut recueilli est chromatographié sur silice avec CHCl₃/ MeOH/NH₄OH 95/5/0,1 ; on obtient 100 mg du produit attendu, solide.

### 22.4

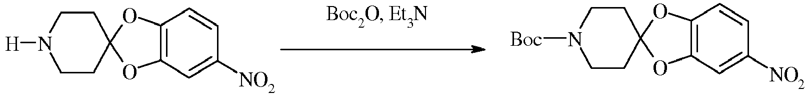

95 mg de l'amine obtenue à l'étape 22.3 sont traités par 98 mg de Boc₂O et 20 mg de triéthylamine dans 3,5 mL de dichlorométhane pendant 1H. Après réaction et traitement habituels, on obtient un solide blanc m = 130 mg.

### 22.5

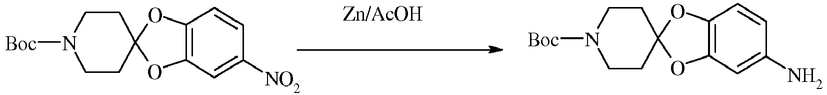

Le groupe NO₂ est réduit en amine par Zn/AcOH selon le mode opératoire déjà décrit 23.1

A 985 mg de produit de la préparation 4.1 dissous dans 25 mL de DCM, on ajoute 0,46 mL de pyridine. 0,84 mL d'anhydride triflique dissous dans 3 mL de DCM sont ensuite additionnés à 5°C en 20 minutes. Après 1 H à 5°C, le milieu réactionnel est lavé par de l'eau glacée puis par une solution saturée en NaCl. La phase organique est séchée et concentrée sous vide. On obtient 1,42 g de solide.

### 23.2

A 1,15 g de produit obtenu à l'étape 23.1 dissous dans 10 mL de DCM + 0,5 mL DMF, sont ajoutés 75 mg de diéthanolamine. Après une nuit d'agitation, le milieu réactionnel est dilué par 100 mL de DCM, lavé à l'eau, lavé par une solution saturée en NaCl, séchée et concentrée sous pression réduite. Le résidu est purifié par flash chromatographie avec un gradient de méthanol de 0% à 15% dans le chloroforme. On obtient 730 mg de solide.

### 23.3

Le groupe NO₂ est réduit en amine par Zn/AcOH comme précédemment décrit. A partir de 720 mg de produit obtenu à l'étape 23.2, on obtient 400 mg du produit attendu sous forme de gomme.

### Préparation 24

### 24.1

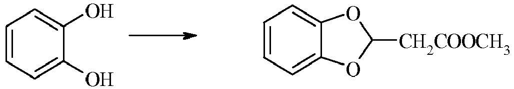

Le produit a été préparé selon le mode opératoire décrit dans Org. Lett. 2001, 3(9), 1399-1402.

### Préparation 24.2

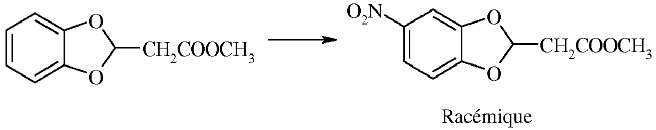

A 2,73 g de produit obtenu à l'étape 24.1 dans 30 mL de DCM on ajoute à 5°C en 2 minutes 5 mL d'acide nitrique à 69%. Après 2H30 d'agitation le milieu réactionnel est dilué par Et₂O ; la phase organique est lavée 2 fois par H₂O, 2 fois par une solution Na₂CO₃ 7% glacée, 1 fois à l'eau, 1 fois par une solution KHSO₄/K2SO₄ 5%, 1 fois à l'eau puis 1 fois par une solution de NaCl saturée. Après séchage et évaporation on obtient un solide blanc m = 3,20 g.

### Préparation 24.3

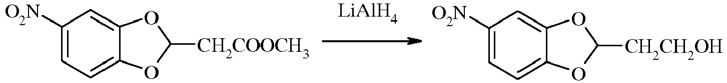

3,63 g de produit obtenu selon le mode opératoire ci-dessus dans 80 mL THF sont traités par 532 mg de LiAlH₄ à -5°C pendant 1H. Après le traitement habituel on isole 2,60 g de produit sous forme d'huile épaisse.

### Préparation 24.4

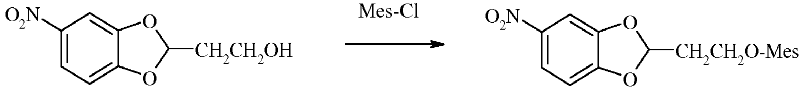

2,59 g d'alcool obtenu à l'étape 24.3 sont traités par le chlorure de mésyle selon le mode opératoire décrit à la préparation 13.3 pour donner 3,52 g de mésylate. Identification par RMN.

### Préparation 24.5

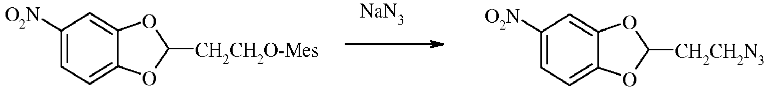

3,51 g du produit obtenu à l'étape 24.4 sont traités par 1,97 g d'azoture de sodium selon le mode opératoire décrit à la préparation 13.6. On obtient 2,60 g de produit attendu.

### Préparation 24.6

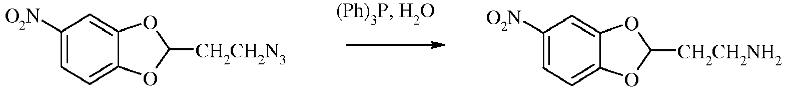

2,59 g du produit obtenu à l'étape 24.5 sont traités par 4,90 g de triphénylphosphine et 2 mL d'eau selon le mode opératoire décrit à la préparation 13.7. On obtient 2 g du produit attendu sous forme d'huile.

### Préparation 24.7

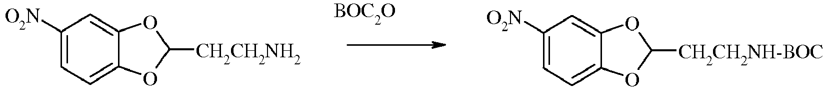

1,99 g de produit obtenu à l'étape 24.6 sont traités par BOC₂O selon le mode opératoire décrit à la préparation 13.8. On obtient 2,41 g de solide.

### Préparation 24.8

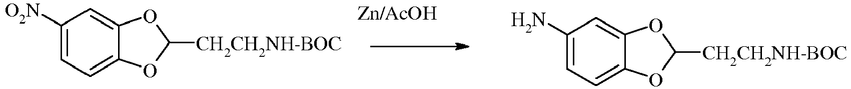

Le produit obtenu à l'étape 24.7 est traité par Zn/AcOH selon le procédé habituel pour réduire le nitro en amino. A partir de 0,93 g de produit de départ, on obtient 0,84 g de produit attendu sous forme de cire.

Les composés de formule (III) et les intermédiaires de formule (IV), dérivés du benzodioxole, sont caractérisés dans le tableau ci-après.

La transformation du composé de formule (IV) en un composé de formule (III) a été effectuée selon la préparation 5.3 pour les composés suivants : 7.2, 9.1, 11.1, 12.1, 13.4 et 14.1.

**TABLEAU 1**

| Préparations des composés de formule (III). | | | |
|---|---|---|---|
| 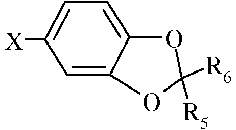 | | | |
| **Préparations** | **CR₅R₆** | **X = NO₂ (IV) RMN** | **X = NH₂ (III) RMN** |
| 2 | CH-CO₂Me | 2.1 | 2.2 |
| | | 3,80 ppm : s : 3H ; 6,95 ppm : s : 1H ; 7,25 ppm : d : 1H 7,85-8,00 ppm : m : 2H | 3,80 ppm : s : 3H ; 4,90 ppm : se : 2H ; 6,10 ppm : dd : 1H ; 6,35 ppm : d : 1H ; 6,55 ppm : s : 1H ; 6,75 ppm : d : 1H |
| 3 | CH-CONH₂ | 3.1 | 3.2 |
| | | 6,60 ppm : s : 1H ; 7,25 ppm : d : 1H ; 7,80-8,30 ppm : m : 4H | 4,80 ppm : se : 2H ; 6,00 ppm : dd : 1H ; 6,10 ppm : s : 1H ; 6,25 ppm : d : 1H ; 6,60 ppm : d : 1H ; 7,65 ppm : se : 1H ; 7,90 ppm : se : 1H |
| 4 | CH-CH₂OH | 4.1 | 4.2 |
| | | 3,75 ppm : m : 2H ; 5,40 ppm : t : 1H ; 6,55 ppm : t : 1H ; 7,10 ppm : d : 1H ; 7,75 ppm : d : 1H ; 7,90 ppm : dd : 1H | 3,65 ppm : m : 2H ; 4,70 ppm : se : 2H ; 5,25 ppm : t : 1H ; 5,90-6,05 ppm : m : 2H; 6,20 ppm : d : 1H ; 6,55 ppm : t : 1H |
| 5 | CH-CH₂NEt₂ | 5.2 | 5.3 |
| | | 0,95 ppm : t : 6H ; 2,55 ppm : qd : 4H ; 2,95 ppm : d : 2H; 6,55 ppm : t : 1H ; 7,10 ppm :d: 1H; 7,70 ppm : d : 1H ; 7,90 ppm : dd : 1H | 0,85 ppm : t : 6H ; 2,45 ppm : qd : 4H ; 2,65 ppm : d : 2H ; 4,55 ppm : se : 2H ; 5,75-5,90 ppm : m : 2H; 6,05 ppm : d : 1H ; 6,40 : d : 1H |
| 6 | 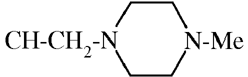 | 6.1 | 6.2 |
| | | 2,60 ppm : s : 3H ; 2,70-3,40 ppm : m : 10H; 6,65 ppm : t : 1H ; 7,05 ppm : d : 1H ; 7,65 ppm : d : 1H ; 7,80 ppm : dd : 1H | 2,30-2,70 ppm : m : 11H 2,80 ppm : d : 2H ; 4,80 ppm : se : 2H ; 6,00 ppm : dd : 1H ; 6,15 ppm : t : 1H ; 6,20 ppm : d : 1H ; 6,60 ppm : d : 1H |
| 7 | CH-CONMe₂ | 7.2 | 7.3 |
| | | 2,85 ppm : s : 3H ; 3,10 ppm : s : 3H ; 7,15 ppm : d : 1H ; 7,35 ppm : s : 1H ; 7,80 ppm : d : 1H ; 7,90 ppm : dd : 1H | 2,85 ppm : s : 3H ; 3,05 ppm : s : 3H ; 4,80 ppm : se : 2H ; 6,00 ppm : dd : 1H ; 6,20 ppm : d : 1H ; 6,60 ppm : d : 1H ; 6,75 ppm : s : 1H |
| 8 | CH-CONHEt | 8.1 | 8.2 |
| | | 1,00 ppm : s : 3H ; 3,10 ppm : qt : 2H ; 6,50 ppm : s : 1H ; 7,10 ppm : d : 1H; 7,70 ppm : d : 1H ; 7,85 ppm : dd : 1H ; 8,70 ppm : te : 1H | 1,00 ppm : t : 3H ; 3,05 ppm : qt : 2H ; 4,70 ppm : se : 2H ; 5,95 ppm : dd : 1H ; 6,05 ppm : s : 1H ; 6,15 ppm : d : 1H ; 6,50 ppm : d : 1H ; 8,40 ppm : te : 1H |
| 9 | CH-CN | 9.1 | 9.2 |
| | | 7,30 ppm : d : 1H ; 7,45 ppm : s : 1H ; 8,05-8,85 ppm : m : 2H | 5,10 ppm : se : 2H ; 6,20 ppm : dd : 1H ; 6,45 ppm : d : 1H ; 6,85 ppm d : 1H ; 7,25 ppm : s : 1H |
| 10 | CH-CONHNMe₂ | 10.1 | 10.2 |
| | | 2,50 ppm : s : 6H ; 6,50 ppm : s : 1H ; 7,10-7,20 ppm : m : 1H ; 7,75-7,80 ppm : m : 1H ; 7,85-7,95 ppm : m : 1H ; 9,80 ppm : se : 1H | 2,50 ppm : s : 6H ; 4,75 ppm : se : 2H ; 5,95-6,50 ppm : m : 4H; 9,50 ppm : se : 1H |
| 11 | 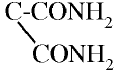 | 11.1 | 11.2 |
| | | 7,05 ppm : d : 1H ; 7,65 ppm : d : 1H ; 7,80 ppm : dd : 1H ; 7,90 ppm : se : 2H ; 8,05 ppm : se : 2H | 4,80 ppm : se : 2H ; 6,00 ppm : dd : 1H ; 6,20 ppm : d : 1H ; 6,55 ppm : d : 1H ; 7,70 ppm : de : 4H |
| 12 | 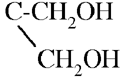 | 12.1 | 12.2 |
| | | 3,80 ppm : d : 4H ; 5,40 ppm : t : 2H ; 7,05 ppm : d : 1H ; 7,65 ppm : d : 1H ; 7,85 ppm : dd : 1H | 3,45 ppm : d : 4H ; 4,50 ppm : se : 2H ; 5,05 ppm : t : 2H ; 5,80 ppm : dd : 1H ; 6,05 ppm : d : 1H ; 6,35 ppm : d : 1H |
| 13 | 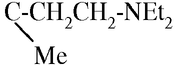 | 13.4 | 13.5 |
| | | 0.90 ppm : t : 6H ; 1,70 ppm : s : 3H ; 2,10-2,60 ppm : m : 8H ; 7,05 ppm : d : 1H ; 7,70 ppm : d : 1H ; 7,85 ppm : dd : 1H. | 0.90 ppm : t : 6H ; 1,50 ppm : s : 3H ; 1,85-2,00 ppm : m: 2H; 2,30-2,60 ppm : m : 6H ; 4,60 ppm : se : 2H ; 5,90 ppm : dd : 1H ; 6,10 ppm : d: 1H ; 6,45 ppm : d : 1H |
| 13a | 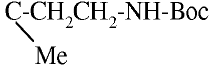 | 13.8 | 13.9 |
| | | 1,35 ppm : s : 9H ; 1,45 ppm: s : 3H; 2,10-2,20 ppm : m : 2H ; 3,0-3,10 ppm : m : 2H ; 6,80-6,90 ppm : m :1H ; 7,10 ppm : d :1H ; 7,65 ppm : d : 1H ; 7,85 ppm : dd : 1H. | 1,35 ppm : s : 9H ; 1,45 ppm : s : 3H ; 1,90-2,05 ppm : m : 2H ; 2,95-3,10 ppm : m : 2H ; 4,60 ppm : se : 2H ; 5,95 ppm : dd : 1H ; 6,15 ppm : d : 1H ; 6,45 ppm : d : 1H ; 6,75 ppm : t:1H. |
| 14 | CH-CH₂OMe | 14.1 | 14.2 |
| | | 3,30 ppm : s : 3H ; 3,75 ppm : d : 2H ; 6,60 ppm : t : 1H ; 7,10 ppm: d: 1H ; 7,70 ppm : d : 1H ; 7,85 ppm : dd : 1H | 3,30 ppm : s : 3H ; 3,60 ppm : d : 2H ; 4,60 ppm : se : 2H ; 5,95 ppm : dd : 1H ; 6,10 ppm: t : 1H ; 6,20 ppm : d : 1H ; 6,50 ppm : d : 1H |
| 15 | 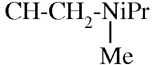 | 15.1 | 15.2 |
| | | 1,30-1,40 ppm : 2d : 6H 2,70 ppm : d : 3H ; 3,50-4,00 ppm : m : 3H ; 7,10 ppm : t : 1H ; 7,20-7,30 ppm : m : 1H ; 7,80-8,00 ppm : m : 2H. | |
| 16 | CH-CO-NH-NHBoc | 16.1 | 16.2 |
| | | 1,40 ppm : s : 9H ; 6,70 ppm : s : 1H ; 7,20 ppm : d : 1H ; 7,80 ppm : d : 1H ; 7,95 ppm : dd : 1H ; 9,00 ppm : s : 1H ; 10,60 ppm : s : 1H. | 1,40 ppm : s : 9H ; 4,70 ppm : se : 2H ; 6,05 ppm : dd : 1H ; 6,30 ppm : d+s : 2H ; 6,65 ppm : d : 1H 8,90 ppm : s : 1H ; 16,20 ppm : s : 1H. |
| 17 | 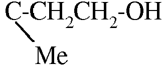 | 17.1 | 17.2 |
| | | 1,60 ppm : s : 3H ; 2,15 ppm : t : 2H ; 3,60 ppm : qd : 2H ; 4,60 ppm : t : 1H ; 7,00 ppm : d : 1H ; 7,70 ppm : d : 1H ; 7,85 ppm : dd : 1H. | 1,50 ppm : s : 3H ; 2,00 ppm : t : 2H ; 3,55 ppm : qd : 2H ; 4,50 ppm : t : 1H ; 4,60 ppm : se : 2H ; 5,90 ppm : dd : 1H ; 6,10 ppm : d : 1H; 6,50 ppm : d : 1H. |
| 21 | 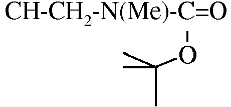 | 21.5 | 21.6 |
| | | 1,35 ppm : s : 9H ; 2,85 ppm : se : 3H ; 3,70 ppm : d : 2H ; 6,60 ppm : te : 1H ; 7,10 ppm : d : 1H ; 7,70 ppm : d : 1H ; 7,85 ppm : dd : 1H. | 1,40 ppm : s : 9H; 2,80 ppm : se : 3H ; 3,50 ppm : d : 2H ; 4,70 ppm : s : 2H ; 5,95 ppm : dd : 1H ; 6,10-6,20 ppm : m : 1H ; 6,20 ppm : d : 1H ; 6,50 ppm : d : 1H. |
| 22 | 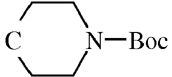 | 22.4 | |
| | | 1,40 ppm : s : 9H ; 2,00 ppm : te : 4H ; 3,55 ppm : te : 4H ; 7,10 ppm : d : 1H ; 7,75 ppm : d : 1H ; 7,90 ppm : dd : 1H. | |
| 23 | CH-CH₂N((CH₂)₂OH)₂ | 23.2 | |
| | | 2,65 ppm : t : 4H ; 3,05 ppm : d : 2H ; 3,40 ppm : t : 4H ; 4,35 ppm : se : 2H ; 6,50 ppm : t : 1H ; 7,05 ppm : d : 1H ; 7,70 ppm : d : 1H ; 7,85 ppm : dd : 1H. | |
| 24 | CH-CH₂CH₂-NH-Boc | 24.7 | 24.8 |
| | | 1,35 ppm : s : 9H ; 2,10 ppm : qd : 2H ; 3,10 ppm : qd : 2H ; 6,50 ppm : t : 1H ; 6,95 ppm : t: 1H ; 7,10 ppm : d : 1H ; 7,70 ppm : d : 1H ; 7,90 ppm : dd : 1H. | 1,35 ppm : s : 9H ; 1,95 ppm : qd : 2H ; 3,10 ppm : qd : 2H ; 4,70 ppm : se : 2H ; 5,90-6,05 ppm : mt : 2H ; 6,15 ppm: d: 1H ; 6,50 ppm : d : 1H ; 6,90 ppm : t :1H. |

### Préparation 18

### 18.1 Chlorhydrate de ((7-nitro-2,3-dihydro-1,4-benzodioxin-2-yl)méthyl) diéthylamine.

A 1,50 g de 7-nitro-2,3-dihydro-1,4-benzodioxin-2-yl méthanesulfonate, décrit dans la demande de brevet WO 01/021 577, dans 30 mL de DMF, on ajoute 800 µl de diéthylamine et chauffe à 80°C, puis on ajoute 3 fois 800 µl de diéthylamine à 12 heures d'intervalle. Après 48 heures, le milieu réactionnel est évaporé à sec ; le résidu est repris dans 100 mL d'AcOEt plus 5 mL de NaOH 4N ; la phase organique est décantée, lavée à l'eau puis par NaCl saturée ; après séchage et évaporation de l'AcOEt, le résidu est repris dans 20 mL d'AcOEt plus 50 mL d'HCl 0,5N et on agite puis décante ; la phase aqueuse est évaporée à sec et le résidu trituré dans l'éther, filtré et séché, m = 0,95 g, F = 192-194°C.

### 18.2 7-Amino-2,3-dihydro-1,4-benzodioxin-2-yl)méthyl)diéthylamine.

A 0,94 g de dérivé nitré obtenu à l'étape précédente en suspension dans 40 mL de THF, on ajoute 3,05 g de zinc en poudre. Après refroidissement à 0°C, on ajoute en 20 minutes 3,1 mL d'acide acétique ; après 10 minutes le bain de glace est enlevé et l'agitation poursuivie 2 heures. Le solide est filtré, lavé au THF puis avec un peu de méthanol. Le filtrat est évaporé à sec, repris dans l'acétate d'éthyle, on ajoute de l'eau et amène à pH = 8 par NaOH 10N ; le précipité formé est éliminé par filtration, lavé par AcOEt ; le filtrat est décanté, lavé par NaCl saturé, séché et évaporé ; on obtient une huile marron, m = 0,51 g.

Les composés de formule (III) et les intermédiaires de formule (IV), dérivés de benzodioxine sont préparés sous forme racémique et sont caractérisés dans le tableau 2 suivant :

**TABLEAU 2**

| Préparation des composés de formule (III) | | | | |
|---|---|---|---|---|
| 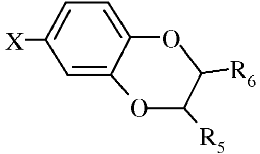 | | | | |
| Préparations | R₅ | R₆ | X = NO₂ (IV), HCl RMN | X = NH₂ (III) RMN |
| 18 | CH₂-NEt₂ | H | 18.1 | 18.2 |
| | | | 1,00-1,10 ppm : m : 6H ; 3,00-3,40 ppm : m : 6H ; 4,00-4,50 ppm : m : 2H ; 4,80-4,95 ppm : m : 1H ; 7,10 ppm : d : 1H ; 7,65-7,80 ppm : m : 2H | 1,00 ppm : t : 6H ; 2,45-2,65 ppm : m : 6H; 3,80-4,20 ppm : m : 3H; 4,70 ppm : se : 2H ; 6,05-6,20 ppm : m : 2H; 6,60 ppm : d: 1H |
| 19 | 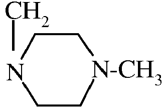 | H | 19.1 | 19.2 |
| | | | 2,85 ppm : s : 3H ; 3,30-3,80 ppm : m : 10H; 4,15-4,50 ppm : m : 2H ; 4,90-5,10 ppm : m : 1H ; 7,15 ppm : d : 1H ; 7,75-7,90 ppm : m : 2H | 2,15 ppm : s : 3H ; 2,30-2,60 ppm : m : 10H ; 3,70-4,30 ppm : m : 3H ; 4,60 ppm : se : 2H ; 6,00-6,10 ppm: m: 2H; 6,50 ppm : d : 1H. |
| 20 | H | CH₂-NEt₂ | 20.1 | 20.2 |
| | | | 1,20-1,30 ppm : m : 6H ; 3,15-3,60 ppm : m : 6H ; 4,10-4,60 ppm : m : 2H ; 5,05 ppm : te : 1H ; 7,15 ppm: d : 1H ; 7,75-7,90 ppm : m : 2H | 0,90 ppm : t : 6H ; 2,40-2,60 ppm : m : 6H ; 3,75-4,25 ppm : m : 3H ; 4,60 ppm : se : 2H ; 5,95-6,10 ppm : m : 2H ; 6,50 ppm : d : 1H. |

Les numéros des composés exemplifiés renvoient à ceux donnés dans le Tableau 3 ci-après qui illustre les structures chimiques et les propriétés physiques de quelques composés selon l'invention. Lorsqu'ils contiennent un carbone asymétrique, ces composés sont obtenus sous forme racémique.

### EXEMPLE 1 : Composé N° 2

### 5-((7-(((tert-Butylamino)carbonyl)amino)-6-(2,6-dichlorophényl)pyrido[2,3-d] pyrimidin-2-yl)amino)-1,3-benzodioxole-2-carboxylate de méthyle.

Un mélange de 0,97 g de composé de la Préparation 2.2 et 1,40 g du composé de la Préparation 1 est porté au reflux dans 15 mL de THF. Après 6 heures, le milieu réactionnel est concentré sous vide. Le produit est purifié par chromatographie sur silice avec AcOEt/toluène (2/3) puis rechromatographié avec CHCl₃/MeOH 98/2, on obtient 0,45 g de solide jaune identifié par spectrométrie de masse, MH⁺ = 583.

### EXEMPLE 2 : Composé N° 4

### Acide de 5-((7-(((tert-butylamino)carbonyl)amino)-6-(2,6-dichlorophényl)pyrido [2,3-d]pyrimidin-2-yl)amino)-1,3-benzodioxole-2-carboxylique.

A 0,25 g de l'ester obtenu à l'Exemple ci-dessus dans 10 mL de méthanol, on ajoute 0,3 mL de soude 2N. Après 1 heure 10 minutes d'agitation à température ambiante, le milieu réactionnel est dilué par AcOEt + H₂O et on amène à pH = 4 par HCl 1N. La phase organique est décantée, lavée par H₂O puis NaCl saturée, séchée et évaporée. Le résidu solide jaune est repris dans de l'éther, trituré, filtré et séché. On obtient 205 mg de produit identifié par spectrométrie de masse, MH⁺ = 569.

### EXEMPLE 3 : Composé N° 3

### 5-((7-(((tert-Butylamino)carbonyl)amino)-6-(2,6-dichlorophényl)pyrido [2,3-d] pyrimidin-2-yl)amino)-1,3-benzodioxole-2-carboxamide.

Un mélange de 0,62 g du composé de la Préparation 3.2 et 1,40 g de dérivé sulfone de la Préparation 1 est porté au reflux dans 20 mL de THF. Après 3 heures, le milieu réactionnel est concentré sous vide puis le résidu chromatographié sur silice avec CHCl₃/MeOH 97/3 v/v. On obtient 315 mg de solide jaune identifié par spectrométrie de masse, MH⁺ = 568.

### EXEMPLE 4 : Composé N° 9

### N-(tert-Butyl)-N'-(6-(2,6-dichlorophényl)-2-((2-hydroxyméthyl)-1,3-benzodioxol-5-yl)amino)pyrido[2,3-d]pyrimidin-7-yl)urée.

A un mélange de 350 mg du composé de la Préparation 4.2 et 937 mg du composé de la Préparation 1 dans 15 mL d'EtOH, on ajoute 43 µl d'HCl concentré et chauffe à 55°C pendant 6 heures. Le milieu réactionnel est concentré sous vide puis le résidu chromatographié sur silice, on obtient 490 mg du solide jaune identifié par spectrométrie de masse, MH⁺ = 555.

### EXEMPLE 5 : Composé N° 6

### 5-((7-(((tert-Butylamino)carbonyl)amino)-6-(2,6-dichlorophényl)pyrido[2,3-d] pyrimidin-2-yl)amino)-1,3-benzodioxole-2-carbonitrile.

A 1,00 g du composé de la Préparation 1 et 450 mg du composé de la Préparation 9.2 dans 15 mL d'EtOH, on ajoute 50 µl d'HCl concentré et porte à léger reflux. Après 1 heure et demie, le milieu réactionnel est concentré sous vide. Le résidu est chromatographié sur silice avec CHCl₃/AcO (90/10 ; v/v). On obtient 465 mg de solide jaune identifié par spectométrie de masse, MH⁺ = 550.

### EXEMPLE 6 : Composé N° 12

### N-(2-((Aminométhyl)-1,3-benzodioxol-5-yl)amino)-6-(2,6-dichlorophényl)pyrido [2,3-d]pyrimidin-7-yl)-N'-(tert-butyl)urée.

A 530 mg de dérivé nitrile de l'Exemple précédent dissous dans 25 mL de THF, on additionne à -10°C en 30 minutes, 1,9 mL de solution de LiAlH₄ 1M dans le THF. 15 minutes après la fin de l'addition, on ajoute 1,5 mL d'AcOEt puis 10 mL d'une solution saturée en NH₄Cl, puis on laisse remonter la température. Après dilution par AcOEt, on lave à l'eau puis par NaCl saturée. Après séchage, la phase organique est concentrée sous vide et le résidu chromatographié sur silice avec CHCl₃/MeOH (95/5 ; v/v). On obtient 165 mg de solide jaune identifié par spectrométrie de masse, MH⁺ = 554.

### EXEMPLE 7 : Composé N° 19

### 2-((5-((7-(((tert-Butylamino)carbonyl)amino)-6-(2,6-dichlorophényl)pyrido [2,3-d]pyrimidin-2-yl)amino)-1,3-benzodioxol-2-yl)carbonyl)hydrazine carboxylate de tert-butyle.

A 0,645 g de produit obtenu à la Préparation 1 et 0,63 g de produit obtenu à la Préparation 16.2 dans 25 mL d'éthanol, on ajoute 0,025 mL d'HCl concentré et agite 5 heures à 70°C. Le milieu réactionnel est évaporé et le résidu est repris dans CHCl₃ que l'on lave à l'eau, avec une solution NaHCO₃ saturée, à l'eau, par NaCl saturée, puis on sèche et évapore sous vide ; le résidu est chromatographié sur silice. On obtient 450 mg de solide jaune identifié par spectrométrie de masse, MH⁺ = 683.

### EXEMPLE 8 : Composé N° 20

### N-(tert-Butyl)-N'-(6-(2,6-dichlorophényl)-2-((2-hydrazinocarbonyl)-1,3-benzodioxol-5-yl)amino)pyrido[2,3-d]pyrimidin-7-yl)urée.

360 mg du composé de l'Exemple précédent sont agités 45 minutes dans un mélange de 4 mL de CH₂Cl₂ et de 14 mL de TFA ; le milieu réactionnel est évaporé sous vide ; le résidu est repris dans CHCl₃ que l'on lave par H₂O, Na₂CO₃ à 15 % dans l'eau, H₂O, une solution saturée de NaCl ; après séchage le chloroforme est évaporé sous vide puis le résidu trituré dans l'éther, filtré, séché. On obtient 225 mg de solide jaune, MH+ = 583.

### EXEMPLE 9 : Composé N° 34

431 mg du composé N° 33, sont agités 30 minutes à température ambiante dans 5mL de DCM et 5mL de TFA. Après évaporation, le résidu est repris dans mélange chloroforme / eau et on amène à pH = 9 par ajout d'une solution aqueuse de Na₂CO₃ 15 % . La phase organique est décantée, lavée à l'eau puis par une solution saturée en NaCl, séchée et concentrée sous pression réduite. On recueille 116 mg d'un solide.
[M + H]⁺ = 568

### EXEMPLE 10 : Composé N°35

### Etape 1 :

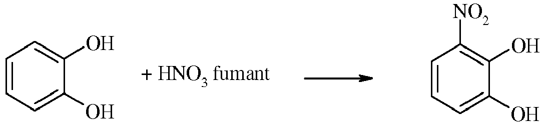

Etape réalisée selon le mode opératoire décrit dans J.Organometall. Chem. 1996, 507, 1-21.

### Etape 2 :

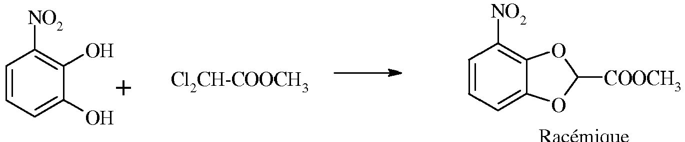

Etape réalisée selon J. Med. Chem. 1988, 31, 84-91.

### Etape 3 :

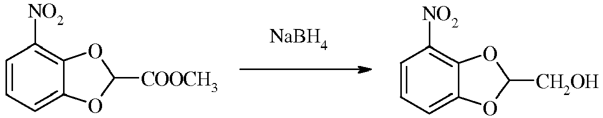

A 2,91 g du produit obtenu à l'étape précédente dans 40 mL de méthanol, on ajoute à 8°C en 35 mn de 800 mg de borohydrure de sodium. Après 50 minutes, le milieu réactionnel est versé sur 150 mL eau/glace plus 400 mL d'acétate d'éthyle ; après 5 minutes d'agitation, décantation, lavages de la phase organique par une solution KHSO₄/K₂SO₄ 5%, eau, solution saturée en NaCl ; après séchage et évaporation de la phase organique un solide brun est isolé : m = 2,25 g.

### Etape 4 :

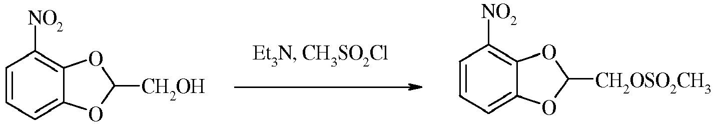

A 2,24 g de l'alcool précédent dans 60 mL de DCM, sont additionnés successivement 1,43 g de triéthylamine, puis en 25 minutes 1,67 g de chlorure de méthane sulfonyle. Après 45 minutes, le milieu réactionnel est dilué par 100 mL de DCM ; lavages 2 fois par de l'eau glacée et 1 fois par une solution saturée par NaCl, séchage et concentration sous vide de la phase organique. On obtient un solide marron m = 3,01 g.

### Etape 5

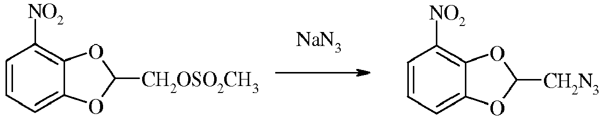

Un mélange de 3,0 g du produit obtenu à l'étape 4 et 1,82 g d'azoture de sodium est chauffé pendant 7h30 dans 20 mL de DMF à 65°C. Le milieu réactionnel est ensuite versé sur 75 mL d'eau glacée et 300 mL d'éther. La phase organique est isolée, lavée plusieurs fois à l'eau puis par solution saturée de NaCl. La phase organique est ensuite séchée et concentrée sous vide pour donner un solide marron : m = 2,20 g .

### Etape 6 :

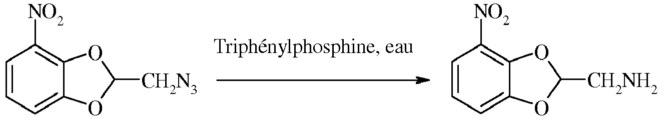

A 2,19 g du produit obtenu à l'étape 5, dissous dans 50 mL d'acétate d'éthyle, sont ajoutés en 15 minutes 4,33 g de triphénylphosphine puis, après 10 minutes, 1,8 mL d'eau en 2 minutes. Le milieu réactionnel est agité pendant 1h40 à 60°C puis est dilué par 150 mL d'acétate d'éthyle. La solution ainsi obtenue est lavée 2 fois à l'eau, 1 fois par une solution saturée en NaCl, séchée, évaporée. Le résidu d'évaporation est dissous dans un mélange de 50 mL d'acétate d'éthyle et 50 mL d'éther éthylique et on extrait par 2 fois 50 mL de HCl 1N. Les phases aqueuses acides sont rassemblées et extraites par un mélange de 25 mL d'acétate d'éthyle et 25 mL d'éther éthylique puis mises en contact de 300 mL d'acétate d'éthyle et le pH est amené à 9 par NaOH 10N. Après décantation, la phase organique est lavée par de l'eau, une solution saturée en NaHCO₃, de l'eau, puis par une solution saturée en NaCl. La phase organique résiduelle est séchée puis concentrée sous vide. On obtient une huile m = 1,40 g.

### Etape 7 :

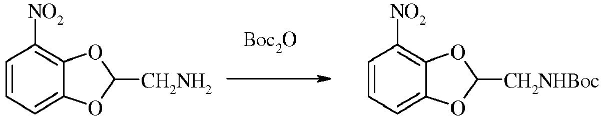

A 1,39 g de l'amine obtenue à l'étape 6 dans 35 mL de DCM, sont additionnés à 5°C 0,35 g de triéthylamine puis 1,75 g de Boc₂O sur une période de 10 minutes. Après 1 nuit d'agitation à température ambiante, le milieu réactionnel est dilué par 150 mL de DCM, lavé par de l'eau, une solution KHSO₄/K2SO₄ 5%, de l'eau, puis par une solution saturée en NaCl. Après séchage et évaporation du DCM, le solide obtenu est dissout dans le minimum d'éther éthylique puis de l'heptane est additionné jusqu'à précipitation totale. On obtient un solide : m = 1,90 g.
RMN : 1,30 ppm :s :9H ; 3,40-3,55 ppm :mt :2H ; 6,55 ppm :t :1H ; 7,00 ppm :t :1H ; 7,15-7,30 ppm :mt :2H ; 7,55 ppm :d :1H.

### Etape 8 :

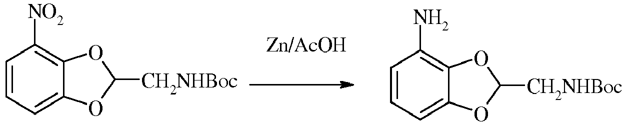

A 0,60 g du produit obtenu à l'étape 7 dans 25 mL de THF est additionné 1,96 g de zinc en poudre puis, à - 3°C 2 mL AcOH sur une durée de 25 minutes. A la fin de l'addition, le milieu réactionnel est laissé revenir à température ambiante. Après 1h15, le milieu réactionnel est filtré, le filtrat est dilué par 150 mL d'acétate d'éthyle et 30 mL d'eau, le pH est amené à 9 par ajout d'une solution de Na₂CO₃ 15 %. Après décantation, la phase organique est isolée, lavée par une solution de NaHCO₃ saturée, de l'eau, puis par une solution de NaCl saturée. La phase organique est isolée, séchée, puis concentrée sous pression réduite. On recueille 0,53 g d'une huile jaune visqueuse. RMN : 1,35 ppm :s :9H ; 3,30 ppm :mt :2H ; 4,80 ppm :s :2H ; 6,05 ppm :t :1H ; 6,10-6,25 ppm :mt :2H ; 6,50 ppm :t :1H ; 7,10 ppm :t :1H..

### Etape 9 :

Couplage pour l'obtention du composé 35, dans les conditions standardisées telles que décrites précédemment.

### EXEMPLE 11 : Composé N° 45

1,035 mL de DIPEA puis 0,675g d'acide formamidinylsulfonique ( H₂N-C(=NH)-SO₃H ) sont ajoutés à 1g du composé 12 dissous dans 15 mL de méthanol et 2 mL de DMF. Après une nuit d'agitation à 25°C, le milieu réactionnel est additionné d'eau, le précipité formé est filtré, lavé à l'eau puis est séché sous pression réduite. Le produit brut est flash-chromatographié sur gel de silice avec un gradient de 10 à 50% de MeOH dans DCM. On obtient 170mg de solide, salifié par une mole/mole de H₂SO₄. MS : MH⁺ = 596

### EXEMPLE 12 : Composé N°39

### Préparation 39.1

A 555 mg de composé 9 (exemple 4) dans 10 mL de DCM, sont ajoutés à 5°C, 0,11 mL de pyridine, puis en 15 minutes 0,20 mL d'anhydride triflique dilué dans 2 mL de DCM. Après 45 minutes, le milieu réactionnel est dilué par 50 mL de DCM, lavé successivement par de l'eau glacée, de l'eau, une solution de KHSO₄/K₂SO₄ 5%, de l'eau, puis par une solution saturée en NaCl. La phase organique est ensuite séchée et concentrée sous pression réduite. On recueille 593 mg de solide.
RMN ¹H : 1,40 ppm : s : 9H ; 4,65 ppm : se : 2H ; 6,50 ppm : t : 1H ; 6,90 ppm : d : 1H ; 7,40 ppm : de : 1H ; 7,50 - 7,70 ppm : m : 3H ; 8,00 ppm : se : 1H ; 8,05 ppm : s : 1H ; 8,20 ppm : se : 1H ; 9,00 ppm : s : 1H ; 10,15 ppm : s : 1H ; 10,70 ppm : s : 1H.

### Préparation 39.2

A 579 mg de composé obtenu à l'étape 39.1 dans 10 mL de DCM est ajouté 0,17 mL de morpholine. Le milieu réactionnel est agité 3h30 à température ambiante, puis l'ensemble est évaporé sous pression réduite. Le résidu est purifié par flash-chromatographie sur gel de silice avec un gradient de 0 à 20 % d'AcOEt dans le DCM. On collecte 300 mg de poudre jaune. MS : MH⁺ = 624.

### EXEMPLE 13 : Composés N°^{s}40-43

Les composés 40 à 43 sont préparés de la même manière que le composé 39 cité à l'exemple 12, en utilisant le composé obtenu à l'étape 39.1 et en remplaçant la morpholine de l'étape 39.2 par, respectivement, de la *tert*-butylamine, de la 1-Boc-pipérazine ; de la cyclopropylamine ; de la cis-2,6-diméthylpipéridine.

### EXEMPLE 14 : Composé N° 44

Le composé N° 44 est obtenu par déprotection du composé 41 obtenu à l'exemple 13 par la méthode décrite précédemment utilisant du TFA.

### EXEMPLE 15 : Composés N°^{s} 46-48

Le composé 12 est mis en réaction avec un acide sous forme activée, par exemple anhydride, chlorure d'acide, acide + agent de couplage, par exemple DCCI, BOP. Ainsi, les composés 46 à 48 sont issus de la réaction entre le composé 12 et, respectivement, de l'anhydride acétique, du chlorure de benzoyle, et du chlorure de cyclopropanecarbonyle.

### EXEMPLE 16 : Composé N° 49

444 mg de composé 12 dans 12 mL d'acétonitrile sont refroidis à 5°C. 0,14 mL de triéthylamine sont ajoutés puis 0,075 mL de chlorure de méthanesulfonyle. Après 40 minutes d'agitation à 25°C, le milieu réactionnel est repris dans AcOEt. La phase organique est lavée par de l'eau, lavée par une solution saturée en NaCl, séchée et évaporée sous pression réduite. Le résidu est purifié par flash-chromatographie sur gel de silice avec un gradient de 0 à 5 % de méthanol dans le chloroforme. On obtient 220 mg de solide. MS : MH⁺ = 632.

### EXEMPLE 17 : Composé N° 50

A 284 mg du composé 4 (exemple 2) dans 5 mL de DMF sont ajoutés 40 mg de tertiobutylamine, 71 mg de diisopropylamine et 176 mg de tétrafluoroborate de O-(1H-benzotriazol-1-yl)-N,N,N',N'-tétraméthyluronium (TBTU). Le milieu réactionnel est agité une heure à 25°C puis est dilué par AcOEt. La phase organique est lavée successivement par de l'eau, une solution saturée de NaHCO₃, de l'eau, puis par une solution saturée de NaCl. La phase organique est séchée, évaporée sous pression réduite, et le résidu est purifié par flash-chromatographie sur gel de silice avec un gradient de 0 à 5% de méthanol dans DCM. On obtient 196 mg d'un solide jaune. MS : MH⁺ = 624.

### EXEMPLE 18 : Composés N°^{s} 51-54

Les composés 51 à 54 sont préparés de la même manière que le composé 50 au départ du composé 4 en remplaçant la tertiobutylamine par, respectivement, de la cyclopropylamine, de la pyrrolidine, de la N-isopropyl-méthylamine, et de la méthylamine.

### EXEMPLE 19 : Composé N° 62

819 mg du composé décrit à la préparation 1 et 0,314 mg de 6-amino-2,3-dihydrobenzo[b]furane, qui peut être préparé selon Eur. J. Med. Chem. Chimica Therapeutica, 1977, Vol. 12, 231-235, sont agités 3 heures à 70°C dans 25 mL d'alcool absolu contenant 0,02mL d'HCl concentré. Après refroidissement, le précipité est filtré, lavé par MeOH tiède puis par Et₂O. On recueille 637 mg d'un solide jaune fondant à 197°C. MS : MH⁺ = 523

### EXEMPLE 20 : Composé N° 69

### Etape 1

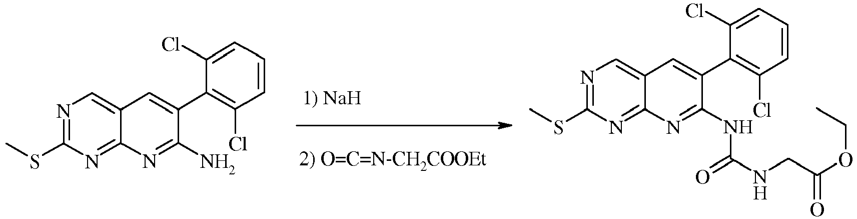

A 3,0 g du produit obtenu à l'étape 1.4 dans 25 mL de DMF sont ajoutés en 10 minutes 432 mg de NaH à 60 %. Après 30 minutes sous agitation, 1,40 g d'isocyanatoacétate d'éthyle sont ajoutés en 10 minutes, puis le milieu réactionnel est laissé 3h30 sous agitation à température ambiante. Le milieu réactionnel est extrait par AcOEt, lavé successivement par de l'eau, une solution de KHSO₄/K₂SO₄ 5%, de l'eau, et par une solution saturée en NaCl. La phase organique est séchée, concentrée sous vide et le produit brut obtenu est purifié par chromatographie sur gel de silice, éluant : CHCl₃/AcOEt 85/15 v/v pour obtenir 1,52 g du produit attendu.

### Etape 2

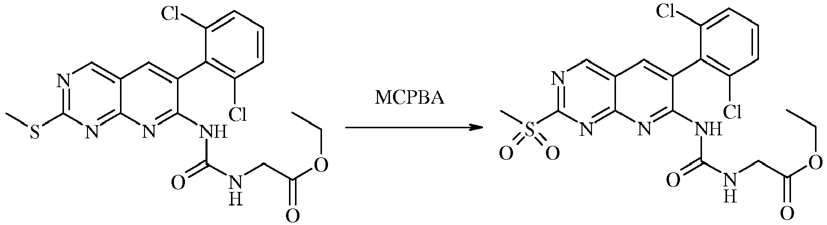

Le produit obtenu à l'étape 1 est oxydé par l'acide métachloroperbenzoïque (MCPBA) selon le procédé décrit à la préparation 1.6. On obtient 900 mg du produit attendu sous la forme d'un solide beige.

### Etape 3

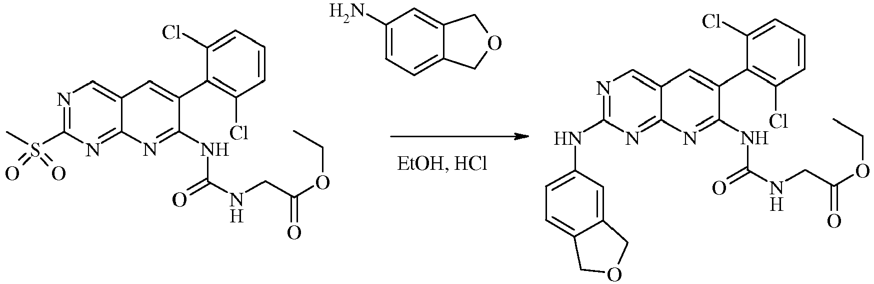

798 mg du produit de l'étape 2 et 295 mg de 1,3-dihydro-2-benzofuran-5-amine sont chauffés à 65°C pendant 6 heures en présence de 15 mL de EtOH et de 0,06 mL de HCl concentré. Le milieu réactionnel est dilué par CHCl₃ et de l'eau, puis on amène la phase aqueuse à pH = 9 par addition d'une solution saturée en NaHCO₃. Après décantation, la phase organique est isolée, lavée par de l'eau puis par une solution saturée en NaCl, séchée et concentrée sous pression réduite. Le produit est cristallisé dans AcOEt. On collecte 0,67 g d'un solide jaune.

### Etape 4

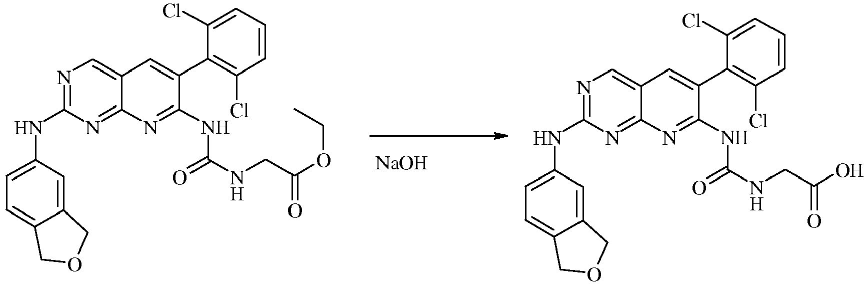

0,66 g de l'ester obtenu à l'étape 3 dans 25 mL d'éthanol et 2 mL de DMF sont traités par 1,5 mL de NaOH 2N pendant 5 heures. Le milieu réactionnel est dilué par CHCl₃ puis est amené à pH = 4 par HCl 1N et décanté. La phase organique est isolée, lavée à l'eau puis par une solution saturée en NaCl, séchée et concentrée sous pression réduite pour obtenir 0,61 g d'une poudre jaune.

### Etape 5

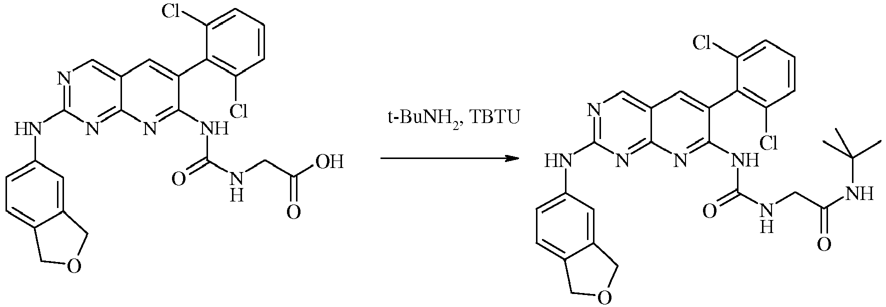

105 mg du produit obtenu à l'étape 4, 16 mg de *tert*-butylamine, 28 mg de DIPEA (diisopropyléthylamine) et 70 mg de TBTU sont agités pendant 1h15 dans 2,5 mL de DMF. Le milieu réactionnel est ensuite extrait par CHCl₃, la phase organique est lavée successivement par de l'eau, une solution de KHSO₄/K₂SO₄ 5%, une solution saturée de NaCl, puis est séchée et évaporée sous pression réduite. Le brut est purifié par chromatographie sur silice, éluant CHCl₃/MeOH, 94/6 v/v. On obtient 90 mg du produit attendu sous forme d'un solide jaune. MS : MH⁺ = 580.

### EXEMPLE 21 : Composés 31 et 32

Le composé 12, préparé à l'exemple 6, peut faire l'objet d'une séparation de ses énantiomères par chromatographie sur phase stationaire chirale comme suit :

| Technique | Berger Prep SFC |
|---|---|
| Détection | UV 230 nm |
| Phase stationnaire | Chiralpack AD-H 250 x 21 mm (5µm) |
| Phase mobile | 60% / 40% CO₂ / (Ethanol + 0,5% isopropylamine) |
| Débit | 50 mL/minute |
| Pression | 100 bar |
| Nombre d'injections | Stacking 350 injections de 28 mg |

A partir de 10 g de mélange racémique, après séparation, on obtient 4,147 g de l'énantiomère dextrogyre (composé 31) et 4,077 g de l'énantiomère lévogyre (composé 32).

Les tableaux 3 et 4 qui suivent, illustrent les structures chimiques et les propriétés physiques de quelques exemples selon l'invention. Dans ces tableaux, Me, Et, iPr et tBu représentent respectivement les groupes méthyle, éthyle, isopropyle et *tert*-butyle et Boc représente le groupe *tert*-butoxycarbonyle. Sauf indication contraire, les produits comprenant un carbone assymétrique sont obtenus sous forme racémique.

**TABLEAU 3**

| 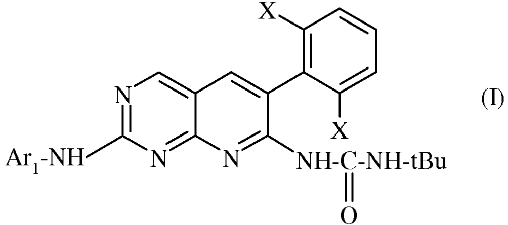 | | | |
|---|---|---|---|
| Composés | Ar₁ | X | Caractérisation RMN |
| 1 | 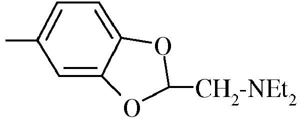 | Cl | 0,90 ppm : t : 6H ; 1,40 ppm : s : 9H ; 2,55 ppm : qd : 4H ; 2,80 ppm : de : 2H ; 6,15 ppm : t : 1H ; 6,70 ppm : d : 1H ; 7,20 ppm : de : 1H ; 7,40-7,65 ppm : m : 3H ; 7,95 ppm : se : 1H ; 8,00 ppm : s : 1H ; 8,10 ppm : s : 1H ; 9,00 ppm : s : 1H ; 10,05 ppm : s : 1H ; 10,60 ppm : s : 1H. |
| 2 | 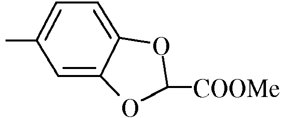 | Cl | 1,40 ppm : s : 9H ; 3,75 ppm : s : 3H ; 6,65 ppm : s : 1H ; 6,90 ppm : d : 1H ; 7,25-7,65 ppm : m : 4H ; 8,00 ppm : se : 2H ; 8,20 ppm : s : 1H ; 9,00 ppm : s : 1H ; 10,15 ppm : s : 1H ; 10,65 : s : 1H. |
| 3 | 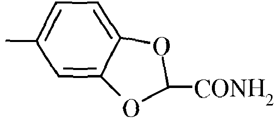 | Cl | 1,35 ppm : s : 9H ; 6,20 ppm : s : 1H ; 6,75 ppm : d : 1H ; 7,25-8,05 ppm : m : 9H ;9,00 ppm : s : 1H ; 10,00 ppm : s : 1H ; 10,55 ppm : s : 1H. |
| 4 | 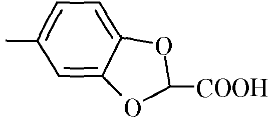 | Cl | 1,45 ppm : s : 9H ; 6,60 ppm : s : 1H ; 7,00 ppm : d : 1H ; 7,40-7,75 ppm : m : 4H; 8,10 ppm: s : 1H ; 8,15 ppm: s : 1H ; 8,25 ppm : s : 1H ; 9,15 ppm : s : 1H ; 10,20 ppm : s : 1H ; 10,75 ppm : s : 1H ; 14 ppm : se : 1H |
| 5 | 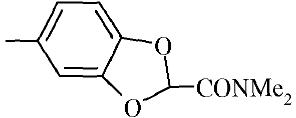 | Cl | 1,45 ppm : s : 9H ; 2,95 ppm : s : 3H ; 3,15 ppm : s : 3H ; 6,90 ppm : d : 1H ; 7,05 ppm : s : 1H ; 7,35 ppm : de : 1H ; 7,50-7,75 ppm : m : 3H ; 8,05 ppm : se : 1H ; 8,10 ppm : s : 1H ; 8,20 ppm : s : 1H ; 9,15 ppm : s : 1H ; 10,20 ppm : s : 1H ; 10,70 ppm : s : 1H. |
| 6 | 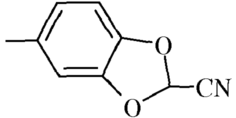 | Cl | 1,40 ppm : s : 9H ; 7,10 ppm : d : 1H ; 7,35-7,65 ppm : m : 5H ; 8,05 ppm : s : 1H ; 8,20 ppm : s : 1H ; 8,30 ppm : s : 1H ; 9,05 ppm: s : 1H ; 10,25 ppm: s : 1H ; 10,75 ppm : s : 1H. |
| 7 | 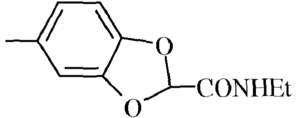 | Cl | 1,05 ppm : t : 3H ; 1,40 ppm : s : 9H ; 3,10 ppm : qt : 2H ; 6,30 ppm : s : 1H ; 6,90 ppm : d : 1H ; 7,30-7,65 ppm : m : 4H ; 7,85 ppm : s : 1H ; 8,05 ppm : s : 1H ; 8,15 ppm : s : 1H ; 8,55 ppm : t : 1H ; 9,05 ppm : s : 1H ; 10,10 ppm : s : 1H ; 10,65 ppm : s : 1H. |
| 8 | 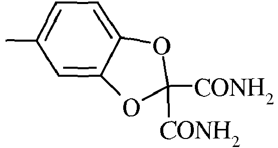 | Cl | 1,45 ppm : s : 9H ; 6,85 ppm : d : 1H ; 7,50-7,90 ppm : m : 9H ; 8,05 ppm : s : 1H ; 8,10 ppm: s : 1H ; 9,10 ppm: s : 1H ; 10,10 ppm : s : 1H ; 10,60 ppm : s : 1H. |
| 9 | 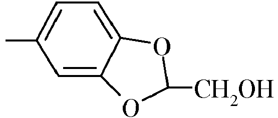 | Cl | 1,50 ppm : s : 9H ; 3,80 ppm : m : 2H ; 5,35 ppm : t : 1H ; 6,25 ppm : t : 1H ; 6,85 ppm : d : 1H ; 7,40-8,25 ppm : m : 7H ; 9,10 ppm : s : 1H ; 10,15 ppm : s : 1H ; 10,75 ppm : s : 1H. |
| 10 | 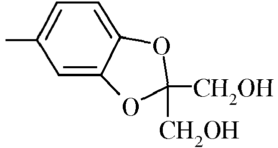 | Cl | 1,40 ppm : s : 9H ; 3,60 ppm : d : 4H ; 5,15 ppm : t : 2H ; 6,60 ppm : d : 1H ; 7,30-7,60 ppm : m : 5H ; 7,95 ppm : s : 1H ; 8,05 ppm : s : 1H ; 9,00 ppm : s : 1H ; 9,95 ppm : s : 1H ; 10,55 ppm : s : 1H. |
| 11 | 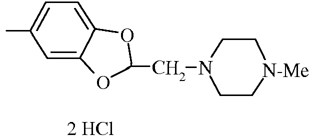 | Cl | 1,40 ppm : s : 9H ; 2,80 ppm : s : 3H ; 3,20-3,70 ppm : m : 10H ; 6,65 ppm : te : 1H ; 6,95 ppm : d : 1H ; 7,35 ppm : de : 1H ; 7,50-7,75 ppm : m : 3H ; 7,95 ppm : se : 1H ; 8,25 ppm : s : 1H ; 9,15 ppm : s : 1H ; 10,40 ppm : se : 2H ; 11,50 ppm: se: 1H. |
| 12 | 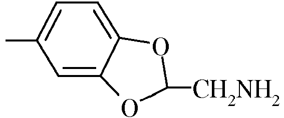 | Cl | 1,40 ppm : s : 9H ; 1,55 ppm : se : 2H ; 2,90 ppm : d : 2H ; 6,10 ppm : t : 1H ; 6,75 ppm : d : 1H ; 7,30 ppm : de : : 1H; 7,45-7,70 ppm : m : 3H ; 7,85 ppm : se : 1H ; 8,05 ppm : s : 1H ; 8,10 ppm : se : 1H ; 9,00 ppm : s : 1H ; 10,00 ppm : s : 1H ; 10,70 ppm : s : 1H. |
| 13 | 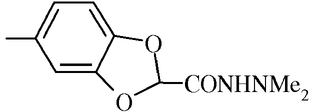 | Cl | 1,40 ppm : s : 9H ; 2,45 ppm : s : 6H ; 6,20 ppm : s : 1H ; 6,80-6,95 ppm : m : 2H ; 7,30-7,70 ppm : m : 4H ; 7,85-8,15 ppm : m : 3H ; 9,00 ppm : s : 1H ; 10,10 ppm : s : 1H ; 10, 70 ppm : s : 1H. |
| 14 | 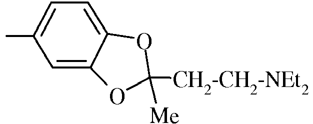 | Cl | 0,95 ppm : t : 6H ; 1,40 ppm : s : 9H ; 1,60 ppm : s : 3H ; 1,95-2,05 ppm : m : 2H ; 2,35-2,70 ppm : m : 6H ; 6,70 ppm : d : 1H ; 7,20 ppm : de : 1H ; 7,40-7,70 ppm : m : 3H ; 7,90 ppm : se : 1H ; 8,00 ppm : s : 1H ; 8,10 ppm : s : 1H ; 9,00 ppm : s : 1H ; 10,00 ppm : s : 1H ; 10,60 ppm : s : 1H. |
| 15 | 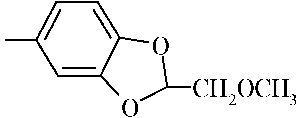 | Cl | 1,40 ppm : s : 9H ; 3,40 ppm : s : 3H ; 3,65 ppm : d : 2H ; 6,30 ppm : t : 1H ; 6,75 ppm : d : 1H ; 7,30 ppm : dd : 1H ; 7,45-7,60 ppm : m : 3H ; 7,85 ppm : s : 1H ; 8,05 ppm : s : 1H ; 8,10 ppm : s : 1H ; 9,00 ppm : s : 1H ; 10,00 ppm : s : 1H ; 10,70 ppm : s : 1H. |
| 16 | 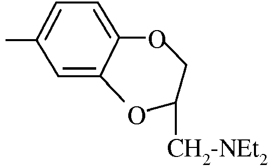 | Cl | 0,85 ppm : t : 6H ; 1,35 ppm : s : 9H ; 2,40-2,60 ppm : m : 6H ; 3,80-4,20 ppm : m : 3H ; 6,65 ppm : d : 1H ; 7,15 ppm : de : 1H ; 7,40-7,60 ppm : m : 3H ; 7,80 ppm : se : 1H ; 7,95 ppm : s : 1H ; 8,00 ppm : se : 1H ; 8,95 ppm : s : 1H ; 9,95 ppm : s : 1H ; 10,50 ppm : s : 1H. |
| 17 | 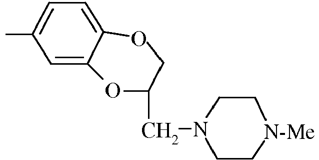 | Cl | 1,50 ppm : s : 9H ; 2,20 ppm : s : 3H ; 2,40-2,60 ppm : m : 10H ; 3,90-4,40 ppm : m : 3H ; 6,80 ppm : d : 1H ; 7,35 ppm : de : 1H ; 7,55-7,75 ppm : m : 3H ; 8,00 ppm : se : 1H ; 8,10 ppm : s : 1H ; 8,20 ppm : s : 1H ; 9,10 ppm : s : 1H ; 10,10 ppm : s : 1H ; 10,65 ppm : s : 1H. |
| 18 | 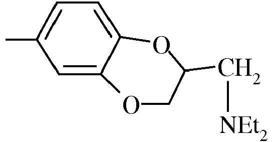 | Cl | 0,95 ppm : t : 6H ; 1,40 ppm : s : 9H ; 2,40-2,70 ppm : m : 6H ; 3,90-4,35 ppm : m : 3H ; 6,75 ppm : d : 1H ; 7,35-7,65 ppm : m : 4H ; 7,90 ppm : se : 1H ; 8,05 ppm : s : 1H ; 8,10 ppm : s : 1H ; 9,05 ppm : s : 1H ; 10 ppm : s : 1H ; 10,60 ppm : s : 1H. |
| 19 | 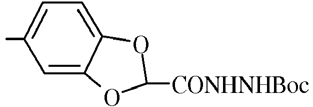 | Cl | 1,40 ppm : s : 9H ; 1,45 ppm : s : 9H ; 6,45 ppm : s : 1H ; 6,85 ppm : s : 1H ; 7,40-7,70 ppm : m : 4H ; 7,90 ppm : se : 1H ; 8,05 ppm: : s: 1H ; 8,15 ppm: : s : 1H ; 8,90 ppm : se : 1H ; 9,05 ppm : s : 1H ; 10,10 ppm : s : 1H ; 10,40 ppm : s : 1H ; 10,70 ppm : s : 1H. |
| 20 | 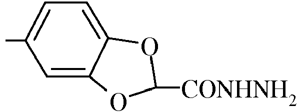 | Cl | 1,40 ppm : s : 9H ; 4,50 ppm : se : 2H ; 6,45 ppm : s : 1H ; 6,60 ppm : d : 1H ; 7,30-7,70 ppm : m : 4H ; 7,85 ppm : se : 1H ; 8,00 ppm : s : 1H ; 8,10 ppm : s : 1H ; 9,00 ppm : s : 1H ; 9,80 ppm : s : 1H ; 10,10 ppm : s : 1H ; 10,70 ppm : s : 1H. |
| 21 | 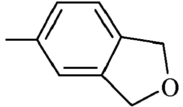 | Cl | 1,45 ppm : s : 9H ; 5,00 ppm : s : 4H ; 7,25 ppm : d : 1H ; 7,45-7,75 ppm : m : 4H ; 8,05 ppm : s : 1H ; 8,10 ppm : se : 1H ; 8,35 ppm : se : 1H ; 9,10 ppm : s : 1H ; 10,10 ppm : se : 1H ; 10,70 ppm : se : 1H. |
| 22 | 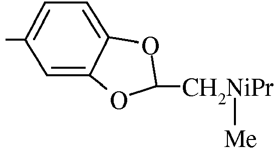 | Cl | 0,95 ppm : d : 6H ; 1,40 ppm : s : 9H ; 2,25 ppm : s : 3H ; 2,70-2,85 ppm : m : 3H ; 6,20 ppm : t : 1H ; 6,75 ppm : d : 1H ;7,25 ppm : de : 1H ; 7,45-7,65 ppm : m : 3H ;7,95 ppm : se : 1H ; 8,05 ppm : s : 1H ;8,10 ppm : se : 1H ; 9,00 ppm : s : 1H ; 10,10 ppm : se : 1H ; 10,70 ppm : se : 1H. |
| 23 | 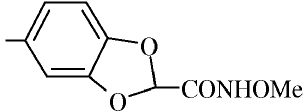 | Cl | 1,45 ppm : s : 9H ; 3,60 ppm : s : 3H ; 6,35 ppm : s : 1H ; 6,75 ppm : d : 1H ; 7,35-7,65 ppm : m : 4H ; 7,90 ppm : se : 1H ; 8,05 ppm : s : 1H ;8,15 ppm : se: 1H ; 9,00 ppm : s : 1H ;10,10 ppm : se : 1H ; 10,60 ppm : se : 1H; 11,90 ppm : se : 1H. |
| 24 | 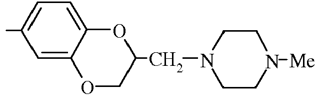 | Cl | 1,40 ppm : s : 9H ; 2,20-2,60 ppm : m : 13H ;3,80-4,30 ppm : m : 3H ; 6,75 ppm : d : 1H ; 7,30 ppm : de : 1H ;7,45-7,70 ppm : m : 3H ; 7,85 ppm : se : 1H ; 8,00 ppm : s : 1H ;8,10 ppm : se: 1H ; 9,00 ppm : s : 1H ; 10,05 ppm : se : 1H ; 10,70 ppm : se : 1H. |
| 25 | 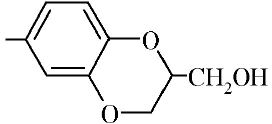 | Cl | 1,45 ppm : s : 9H ; 3,55-3,65 ppm : m : 2H ;3,90-4,30 ppm : m : 3H ; 5,05 ppm : t : 1H ; 6,75 ppm : d : 1H ;7,35 ppm : dd : 1H ;7,45-7,70 ppm : m : 3H ; 7,85 ppm : se : 1H ; 8,05 ppm : s : 1H ;8,10 ppm : se : 1H ; 9,00 ppm : s : 1H ; 10,00 ppm : se : 1H ; 10,70 ppm : se : 1H. |
| 26 | 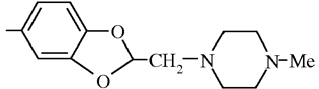 | Me | 1,40 ppm : s : 9H ; 2,00 ppm : s : 6H ; 2,20 ppm : s : 3H ; 2,40-2,75 ppm : m : 10H ; 6,25 ppm : t : 1H ; 6,45 ppm : s : 1H ; 6,75 ppm : d : 1H ;7,15-7,40 ppm : m : 4H ; 7,95 ppm : s : 1H ; 8,05 ppm : se : 1H ; 9,00 ppm : s : 1H ; 10,00 ppm : se : 1H ; 10,50 ppm : se : 1H. |
| 27 | 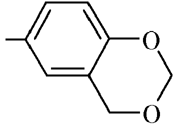 | Cl | 1,40 ppm : s : 9H ; 4,80 ppm : s : 2H ; 5,25 ppm : s : 2H ; 6,70 ppm : d : 1H ; 7,45-7,70 ppm : m : 4H ;7,90-8,10 ppm : m : 3H ; 9,00 ppm : s : 1H ; 10,00 ppm : se : 1H ; 10,50 ppm : se : 1H. |
| 28 | 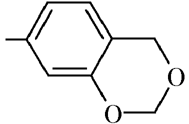 | Cl | 1,40 ppm : s : 9H ; 4,80 ppm : s : 2H ; 5,25 ppm : s : 2H ; 6,90 ppm : d : 1H ; 7,40-7,70 ppm : m : 4H ; 7,90 ppm : se : 1H ; 8,05 ppm : s : 1H ; 8,15 ppm : se : 1H ; 9,10 ppm : s : 1H ; 10,20 ppm : se : 1H ; 10,70 ppm : se : 1H. |
| 29 | 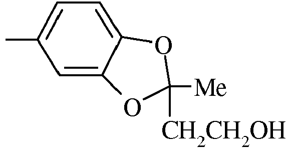 | Cl | 1,40 ppm : s : 9H ; 1,60 ppm : s : 3H ; 2,05 ppm : t : 2H ; 3,60 ppm : t : 2H ; 3,60 ppm : se : 1H ; 6,70 ppm : d : 1H ; 7,20 ppm : de : 1H ; 7,40-7,70 ppm : m : 3H ; 7,80 ppm : se : 1H ; 8,00 ppm : s : 1H ; 8,20 ppm : se : 1H ; 9,00 ppm : s : 1H ; 10,00 ppm : se : 1H ; 10,70 ppm : se : 1H. |
| 30 | 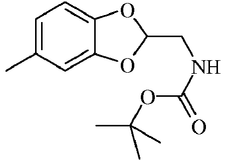 | Cl | 1,30 ppm : s : 9H ; 1,40 ppm : s : 9H ; 3,30 ppm - 3,40 ppm : mt : 2H ; 6,15 ppm : t: 1H; 6,75 ppm: d: 1H; 7,15 ppm: t: 1H ; 7,35 ppm : de : 1H ; 7,45-7,65 ppm : m : 3H ; 7,85 ppm : se : 1H ; 8,05 ppm : s : 1H ; 8,15 ppm : se : 1H ; 9,00 ppm : s : 1H ; 10,05 ppm : s : 1H ; 10,65 ppm : s : 1H. |
| 31 | 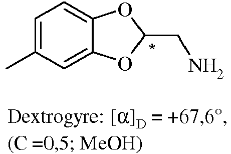 | Cl | 1,40 ppm : s : 9H ; 1,55 ppm : se : 2H ; 2,90 ppm : d : 2H ; 6,10 ppm : t : 1H ; 6,75 ppm : d : 1H ; 7,30 ppm : de : 1H ; 7,45-7,70 ppm : m : 3H ; 7,85 ppm : se : 1H ; 8,05 ppm : s : 1H ; 8,10 ppm : se : 1H ; 9,00 ppm : s : 1H ; 10,00 ppm : s : 1H ; 10,70 ppm : s : 1H. |
| 32 | 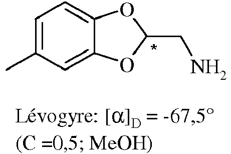 | Cl | 1,40 ppm : s : 9H ; 1,55 ppm : se : 2H ; 2,90 ppm : d : 2H ; 6,10 ppm : t : 1H ; 6,75 ppm : d : 1H ; 7,30 ppm : de : 1H ; 7,45-7,70 ppm : m : 3H ; 7,85 ppm : se : 1H ; 8,05 ppm : s : 1H ; 8,10 ppm : se : 1H ; 9,00 ppm : s : 1H ; 10,00 ppm : s : 1H ; 10,70 ppm : s : 1H. |
| 33 | 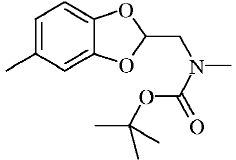 | Cl | 1,35 ppm : s : 9H ; 1,40 ppm : s : 9H ; 2,85 ppm : se : 3H ; 3,60 ppm : d : 2H ; 6,30 ppm : t : 1H ; 6,80 ppm : d : 1H ; 7,30 ppm : de : 1H ; 7,45-7,70 ppm : m : 3H ; 8,00 ppm : se : 1H ; 8,05 ppm : s : 1H ; 8,15 ppm : se : 1H ; 9,00 ppm : s : 1H ; 10,10 ppm : s : 1H ; 10,70 ppm : s : 1H. |
| 34 | 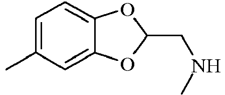 | Cl | 1,40 ppm : s : 9H ; 1,80 ppm : se : 1H ; 2,30 ppm : s : 3H ; 2,85 ppm : d : 2H ; 6,20 ppm : t : 1H ; 6,75 ppm : d : 1H ; 7,30 ppm : dd : 1H ; 7,45-7,65 ppm : m : 3H ; 7,85 ppm : se : 1H ; 8,00 ppm : s : 1H ; 8,10 ppm : se : 1H ; 9,00 ppm : s : 1H ; 10,00 ppm : s : 1H ; 10,70 ppm : s : 1H. |
| 35 | 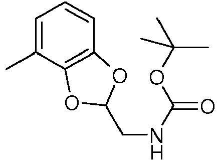 | Cl | 1,35 ppm : s : 9H ; 1,40 ppm : s : 9H ; 3,25 ppm - 3,40 ppm : m : 2H ; 6,25 ppm : t : 1H ; 6,65 ppm - 6,80 ppm : mt : 2H ; 7,10 ppm : t : 1H ; 7,50 ppm - 7,65 ppm : m : 4H ; 8,05 ppm : s : 1H ; 8,10 ppm : s : 1H ; 9,05 ppm : s : 1H ; 9,20 ppm : s : 1H ; 10,40 ppm : s : 1H. |
| 36 | 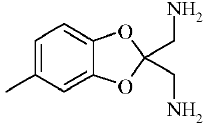 | Cl | 1,40 - 1,60 ppm : m + s : 13H ; 2,90 ppm : s : 4H ; 6,70 ppm : d : 1H ; 7,30 ppm : de : 1H ; 7,45 ppm - 7,60 ppm : se : 4H ; 8,05 ppm : s : 1H ; 8,20 ppm : se : 1H ; 9,00 ppm : s : 1H ; 10,00 ppm : s : 1H ; 10,65 ppm : s : 1H. |
| 37 | 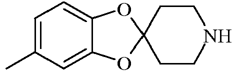 | Cl | LC/MS : MH⁺ = 594 |
| 38 | 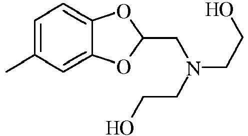 | Cl | 1,45 ppm : s : 9H ; 2,70 ppm : t : 4H ; 3,00 ppm : d : 2H ; 3,45 ppm : qd : 4H ; 4,35 ppm : t : 2H ; 6,20 ppm : t : 1H ; 6,75 ppm : d : 1H ; 7,25 ppm : d : 1H ; 7,50 ppm - 7,70 ppm : m : 3H ; 7,90 ppm : se : 1H ; 8,05 ppm : s : 1H ; 8,10 ppm : se : 1H ; 9,05 ppm: : s : 1H ; 10,10 ppm : s : 1H ; 10,70 ppm : s : 1H. |
| 39 | 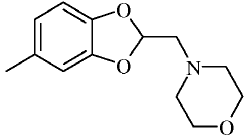 | Cl | 1,40 ppm : s : 9H ; 2,60 ppm : te : 4H ; 2,80 ppm : te : 2H ; 3,55 ppm : t : 4H ; 6,30 ppm : t : 1H ; 6,80 ppm : d : 1H ; 7,25 ppm : de : 1H ; 7,45 ppm - 7,70 ppm : m : 3H ; 8,00 ppm : se : 1H ; 8,05 ppm : s : 1H ; 8,15 ppm : se : 1H ; 9,00 ppm : s : 1H ; 10,10 ppm : s : 1H ; 10,75 ppm : s : 1H. |
| 40 | 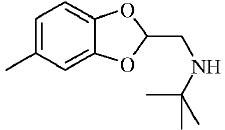 | Cl | 1,00 ppm : s : 9H ; 1,45 ppm : s : 9H ; 2,90 ppm : d : 2H ; 6,10 ppm : t : 1H ; 6,75 ppm : d : 1H ; 7,30 ppm : de : 1H ; 7,40 ppm - 7,65 ppm : m : 3H ; 7,85 ppm : se : 1H ; 8,05 ppm : s : 1H ; 8,15 ppm : se : 1H ; 9,00 ppm : s : 1H ; 10,00 ppm : s : 1H ; 10,65 ppm : s : 1H. |
| 41 | 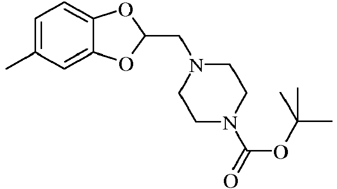 | Cl | 1,35 ppm : s : 9H ; 1,45 ppm : s : 9H ; 2,45 ppm - 2,60 ppm : mt : 4H ; 2,85 : te : 2H ; 3,25 - 3,35 ppm : mt : 4H ; 6,30 ppm : t : 1H ; 6,80 ppm : d : 1H ; 7,25 ppm : de : 1H ; 7,45 ppm - 7,70 ppm : m : 3H ; 8,05 ppm : s : 2H ; 8,15 ppm : se : 1H ; 9,05 ppm : s : 1H ; 10,10 ppm : s : 1H ; 10,70 ppm : s : 1H. |
| 42 | 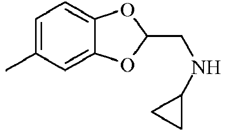 | Cl | 0,20 ppm : mt : 2H ; 0,35 ppm : mt : 2H ; 1,40 ppm : s : 9H ; 2,20 ppm : mt : 1H ; 2,35 ppm : se : 1H ; 2,95 ppm : d : 2H ; 6,20 ppm : t : 1H ; 6,75 ppm : d : 1H ; 7,25 ppm : de : 1H ; 7,45 ppm - 7,60 ppm : m : 3H ; 7,85 ppm : se : 1H ; 8,05 ppm : s : 1H ; 8,10 ppm : se : 1H ; 9,00 ppm : s : 1H ; 10,00 ppm : s : 1H ; 10,65 ppm : s : 1H. |
| 43 | 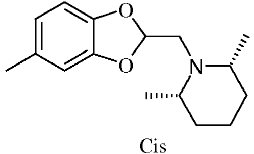 | Cl | 1,05 ppm : s : 3H ; 1,10 ppm : s : 3H ; 1,40 ppm : s : 9H ; 1,10 - 1,60 ppm : m : 6H ; 2,45 - 2,65 ppm : m : 2H ; 3,00 ppm : d : 2H ; 6,15 ppm: t: 1H; 6,75 ppm: d: 1H ; 7,25 ppm : de : 1H ; 7,45 ppm - 7,65 ppm : m : 3H ; 7,95 ppm : se : 1H ; 8,05 ppm : s : 1H ; 8,10 ppm : se : 1H ; 9,00 ppm : s : 1H ; 10,05 ppm : s : 1H ; 10,70 ppm : s : 1H. |
| 44 | 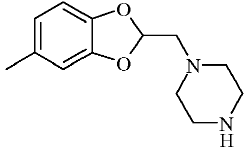 | Cl | (DMSO + TFA) 1,30 ppm : s : 9H ; 3,40 - 3,90 ppm : m : 10H ; 6,75 ppm : t : 1H ; 6,95 ppm : d: 1H ; 7,25 ppm : de : 1H ; 7,55 ppm - 7,75 ppm : m : 4H ; 8,55 ppm : se : 1H ; 9,20 ppm : s : 1H. |
| 45 | 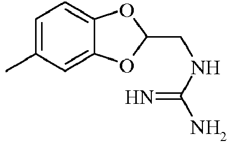 | Cl | (DMSO + TFA) 1,35 ppm : s : 9H ; 3,75 ppm : d : 2H ; 6,35 ppm : t : 1H ; 6,90 ppm : d : 1H ; 7,35 ppm : de : 1H ; 7,45 ppm - 7,75 ppm : m : 4H ; 8,40 ppm : s : 1H ; 9,25 ppm : s : 1H. |
| 46 | 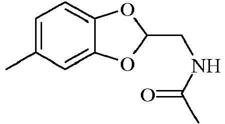 | Cl | 1,40 ppm : s : 9H ; 1,80 ppm : s : 3H ; 3,50 ppm : de : 2H ; 6,15 ppm : t : 1H ; 6,80 ppm : d : 1H ; 7,35 ppm : de : 1H ; 7,45 ppm - 7,65 ppm : m : 3H ; 7,85 ppm : se : 1H ; 8,00 ppm : s : 1H ; 8,10 ppm : se : 1H ; 8,20 ppm : t : 1H ; 9,00 ppm : s : 1H ; 10,00 ppm : s : 1H ; 10,70 ppm : s : 1 H. |
| 47 | 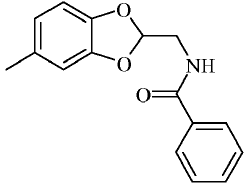 | Cl | 1,40 ppm : s : 9H ; 3,70 ppm : te : 2H ; 6,35 ppm : t : 1H ; 6,80 ppm : d : 1H ; 7,30 ppm : de : 1H ; 7,35 ppm - 7,70 ppm : m : 6H ; 7,85 ppm : d : 2H ; 8,00 ppm : se : 1H ; 8,05 ppm : s : 1H ; 8,10 ppm : se : 1H ; 8,80 ppm : t : 1H ; 9,05 ppm : s : 1H ; 10,10 ppm : s : 1H ; 10,65 ppm : s : 1H. |
| 48 | 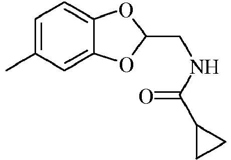 | Cl | 0,60 - 0,75 ppm: m: 4H; 1,40 ppm : s : 9H ; 1,60 ppm : mt : 1H ; 3,55 ppm : mt : 2H ; 6,20 ppm : t : 1H ; 6,70 ppm : d : 1H ; 7,35 ppm : de : 1H ; 7,45 ppm - 7,70 ppm : m : 3H ; 7,85 ppm : se : 1H ; 8,00 ppm : se : 1H ; 8,10 ppm : se : 1H ; 8,40 ppm : t : 1H ; 9,05 ppm : s : 1H ; 10,05 ppm : s : 1H ; 10,70 ppm : s : 1H. |
| 49 | 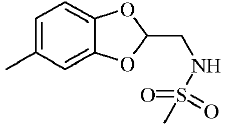 | Cl | 1,45 ppm : s : 9H ; 2,95 ppm : s : 3H ; 3,40 ppm : mt : 2H ; 6,25 ppm : t : 1H ; 6,80 ppm : d: 1H ; 7,40 ppm : de : 1H ; 7,45 ppm - 7,65 ppm : m : 4H ; 7,85 ppm : se : 1H ; 8,05 ppm : s : 1H ; 8,15 ppm : se : 1H ; 9,00 ppm : s : 1H ; 10,10 ppm : s : 1H ; 10,60 ppm : s : 1H. |
| 50 | 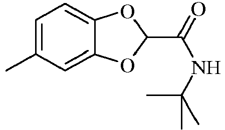 | Cl | 1,25 ppm : s : 9H ; 1,40 ppm : s : 9H ; 6,25 ppm : s : 1H ; 6,80 ppm : d : 1H ; 7,30 ppm : de : 1H ; 7,45 ppm - 7,65 ppm : m : 3H ; 7,85 ppm : se : 1H ; 8,05 ppm : s : 1H ; 8,10 ppm : se : 2H ; 9,00 ppm : s : 1H ; 10,10 ppm : s : 1H ; 10,75 ppm : s : 1H. |
| 51 | 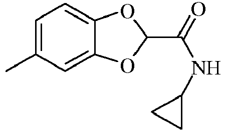 | Cl | 0,40 - 0,70 ppm : mt : 4H ; 1,40 ppm : s : 9H ; 2,70 ppm : mt : 1H ; 6,30 ppm : s : 1H ; 6,80 ppm : d : 1H ; 7,40 ppm : de : 1H ; 7,45 ppm - 7,65 ppm : m : 3H ; 7,85 ppm : se : 1H ; 8,05 ppm : s : 1H ; 8,15 ppm : se : 1H ; 8,60 ppm : d : 1H ; 9,05 ppm : s : 1H ; 10,10 ppm : s : 1H ; 10,65 ppm : s : 1H. |
| 52 | 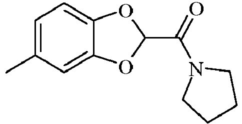 | Cl | 1,40 ppm : s : 9H ; 1,70 - 1,95 ppm : m : 4H ; 3,35 ppm : t : 2H; 3,60 ppm : t : 2H ; 6,75 ppm : s : 1H ; 6,85 ppm : d : 1H ; 7,30 ppm : de : 1H ; 7,45 ppm - 7,65 ppm : m : 3H ; 8,00 ppm : se : 1H ; 8,05 ppm : s : 1H ; 8,15 ppm : se : 1H ; 9,05 ppm : s : 1H ; 10,10 ppm : s : 1H ; 10,65 ppm : s : 1H. |
| 53 | 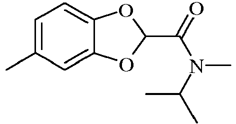 | Cl | 1,05 ppm : d : 3H ; 1,20 ppm : d : 3H ; 1,40 ppm : s : 9H ; 2,70 ppm et 2,90 ppm : 2s : 3H ; 4,20 - 4,60 ppm : m : 1H ; 6,80 - 7,05 ppm : mt : 2H ; 7,30 ppm : de : 1H ; 7,45 ppm - 7,65 ppm : m : 3H ; 8,00 ppm : se : 1H ; 8,05 ppm : s : 1H ; 8,15 ppm : se: 1H ; 9,05 ppm : s : 1H ; 10,15 ppm : s : 1H ; 10,60 ppm : s : 1H. |
| 54 | 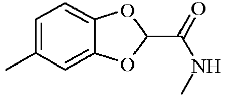 | Cl | 1,40 ppm : s : 9H ; 2,65 ppm : d : 3H ; 6,35 ppm : s : 1H ; 6,85 ppm : d : 1H ; 7,40 ppm : de : 1H ; 7,45 ppm - 7,65 ppm : m : 3H ; 7,85 ppm : se : 1H ; 8,00 ppm : s : 1H ; 8,15 ppm : se : 1H ; 8,50 ppm : qd : 1H ; 9,05 ppm : s : 1H ; 10,10 ppm : s : 1H ; 10,60 ppm : s : 1H. |
| 55 | 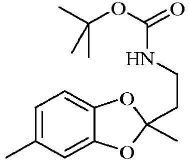 | Cl | 1,35 ppm : s : 9H ; 1,40 ppm : s : 9H ; 1,60 ppm : s : 3H ; 2,65 ppm : t : 2H ; 3,00 - 3,20 ppm : mt : 2H ; 6,70 ppm : d : 1H ; 6,80 ppm : t : 1H ; 7,20 ppm : de : 1H ; 7,45 ppm - 7,70 ppm : m : 3H ; 7,80 ppm : se : 1H ; 8,05 ppm : s : 2H ; 9,00 ppm : s : 1H ; 10,00 ppm : s : 1H ; 10,60 ppm : s : 1H. |
| 56 | 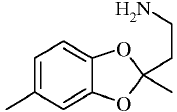 | Cl | 1,40 ppm : s : 9H ; 1,60 ppm : s : 3H ; 2,00 ppm : t : 2H ; 2,70 ppm : t : 2H ; 6,70 ppm : d : 1H ; 7,20 ppm : de : 1H ; 7,50 ppm - 7,70 ppm : m : 4H ; 7,80 ppm : se : 1H ; 8,05 ppm : s : 1H ; 9,00 ppm : s : 1H ; 10,00 ppm : s : 1H ; 10,70 ppm : s : 1H. |
| 57 | 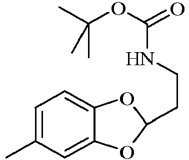 | Cl | 1,35 ppm : s : 9H ; 1,45 ppm : s : 9H ; 2,00 ppm : qd : 2H ; 3,10 : qd : 2H ; 6,20 ppm : t : 1H ; 6,75 ppm : d : 1H ; 6,95 ppm : t : 1H ; 7,30 ppm : de : 1H ; 7,45 ppm - 7,65 ppm : m : 3H ; 7,90 ppm : se : 1H ; 8,05 ppm : s : 1H ; 8,10 ppm : se : 1H ; 9,05 ppm : s : 1H ; 10,10 ppm : s : 1H ; 10,65 ppm : s : 1H. |
| 58 | 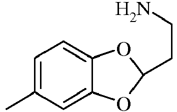 | Cl | (DMSO + TFA) 1,25 ppm : s : 9H ; 2,20 ppm : qd : 2H ; 3,00 ppm : t : 2H ; 6,30 ppm : t : 1H ; 6,75 ppm : d : 1H ; 7,25 ppm : de : 1H ; 7,50 - 7,70 ppm : m : 4H ; 8,50 ppm : s : 1H ; 9,15 ppm : s : 1H. |
| 59 | 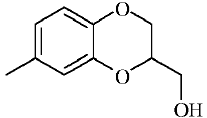 | Cl | 1,45 ppm : s : 9H ; 3,50 - 4,35 ppm : m : 5H ; 5,05 ppm : t : 1H ; 6,75 ppm : d : 1H ; 7,30 ppm : de : 1H ; 7,45 ppm - 7,70 ppm : m : 3H ; 7,90 ppm : se : 1H ; 8,05 ppm : s : 1H ; 8,10 ppm : se : 1H ; 9,00 ppm : s : 1H ; 10,00 ppm : s : 1H ; 10,60 ppm : s : 1H. |
| 60 | 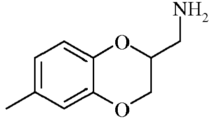 | Cl | 1,40 ppm : s : 9H ; 2,70 -2,85 ppm : m : 2H ; 3,90 - 4,05 ppm : m : 2H ; 4,35 ppm : d : 1H ; 6,75 ppm : d : 1H ; 7,35 ppm : de : 1H ; 7,45 ppm - 7,70 ppm : m : 4H ; 7,85 ppm : se : 1H ; 8,05 ppm : s : 1H ; 9,00 ppm : s : 1H ; 10,00 ppm : s : 1H ; 10,60 ppm : s : 1H. |
| 61 | 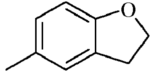 | Cl | 1,40 ppm : s : 9H ; 3,15 ppm : t : 2H ; 4,50 ppm : t : 2H ; 6,65 ppm : d : 1H ; 7,45 ppm - 7,65 ppm : m : 4H ; 8,00 ppm : s : 1H ; 8,05 ppm : se : 1H ; 8,30 ppm : se : 1H ; 9,00 ppm : s : 1H ; 10,00 ppm : s : 1H ; 10,70 ppm : s : 1H. |
| 62 | 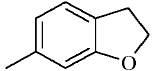 | Cl | 1,40 ppm : s : 9H ; 3,10 ppm : t : 2H ; 4,50 ppm : t : 2H ; 7,10 ppm : d : 1H ; 7,35 ppm : dd : 1H ; 7,45 ppm - 7,70 ppm : m : 3H ; 7,75 ppm : s : 1H ; 8,05 ppm : s : 1H ; 8,15 ppm: s : 1H ; 9,05 ppm : s : 1H ; 10,10 ppm : s : 1H ; 10,65 ppm : s : 1H. |
| 63 | 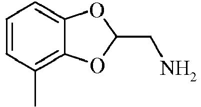 | Cl | 1,35 ppm : s : 9H ; 1,40 - 1,75 ppm : m : 2H ; 2,90 ppm : d : 2H ; 6,15 ppm : t : 1H ; 6,60 ppm - 6,80 ppm : mt : 2H ; 7,40 ppm - 7,65 ppm : mt : 4H ; 8,05 ppm : s : 2H ; 9,05 ppm : s : 1H ; 9,35 ppm : s : 1H ; 10,45 ppm : s : 1H. |
| 64 | 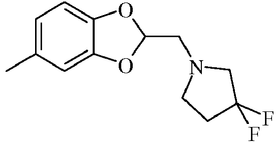 | Cl | 1,40 ppm : s : 9H ; 2,10 - 2,35 ppm : mt : 2H ; 2,80 ppm - 3,15 ppm : m : 6H ; 6,30 ppm : t : 1H ; 6,80 ppm : d : 1H ; 7,25 ppm : de : 1H; 7,45 ppm - 7,70 ppm : m : 3H ; 8,05 ppm : s : 2H ; 8,15 ppm : s: 1H ; 9,05 ppm : s : 1H ; 10,10 ppm : s : 1H ; 10,60 ppm : s : 1H. |
| 65 | 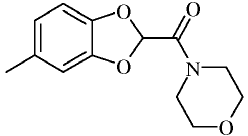 | Cl | 1,40 ppm : s : 9H ; 3,40 - 3,70 ppm : m : 8H ; 6,85 ppm : d : 1H ; 7,00 ppm : s: 1H; 7,30 ppm : dd : 1H ; 7,45 ppm - 7,70 ppm : m : 3H ; 8,05 ppm : s : 2H ; 8,15 ppm : s: 1H ; 9,05 ppm : s : 1H ; 10,10 ppm : s : 1H ; 10,65 ppm : s : 1H. |
| 66 | 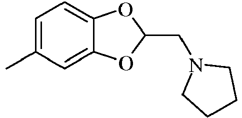 | Cl | 1,40 ppm : s : 9H ; 1,60 - 1,75 ppm : m : 4H ; 2,50 ppm - 2,65 ppm : m : 4H ; 2,75 ppm - 3,00 ppm : mt : 2H ; 6,25 ppm : t : 1H ; 6,75 ppm : d : 1H ; 7,25 ppm : de : 1H; 7,45 ppm - 7,70 ppm : m : 3H ; 7,95 ppm : s : 1H ; 8,05 ppm : s : 1H ; 8,15 ppm : s: 1H ; 9,05 ppm : s : 1H ; 10,10 ppm : s : 1H ; 10,70 ppm : s : 1H. |
| 67 | 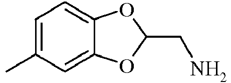 | Br | 1,40 ppm : s : 9H ; 1,60 ppm : se : 2H ; 2,90 ppm : d : 2H ; 6,10 ppm : t : 1H ; 6,75 ppm : d : 1H ; 7,20 ppm - 7,40 ppm : m : 2H ; 7,70 ppm - 7,85 ppm : d : 4H ; 8,00 ppm : s : 1H ; 9,05 ppm : s : 1H ; 10,05 ppm : s : 1H ; 10,70 ppm : s : 1H. |
| 68 | 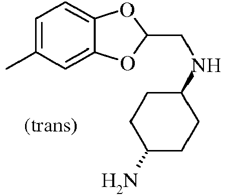 | Cl | 0,95 ppm : t : 4H ; 1,35 ppm : s : 9H ; 1,60 - 1,80 ppm : m : 4H ; 2,25 ppm - 2,45 ppm : m : 2H ; 2,85 ppm: d : 2H ; 6,10 ppm : t : 1H ; 6,70 ppm : d : 1H ; 7,25 ppm : d : 1H; 7,40 ppm - 7,60 ppm : m : 3H ; 7,85 ppm : se : 1H ; 8,00 ppm : s: 1H ; 9,00 ppm : s : 1H ; 10,00 ppm : s : 1H ; 10,65 ppm : s : 1H. |
| 80 | 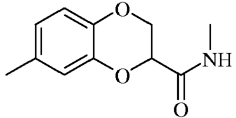 | Cl | 1,40 ppm : s : 9H ; 2,65 ppm : d : 3H ; 4,10 - 4,35 ppm : mt: 2H ; 4,65 - 4,75 ppm : mt : 1H; 6,80 ppm : d : 1H ; 7,35 ppm: dd : 1H ; 7,45 - 7,70 ppm : mt : 3H ; 8,00 - 8,15 ppm : m : 4H ; 9,05 ppm : s : 1H ; 10,10 ppm : s : 1H ; 10,60 ppm : s : 1H. |
| 81 | 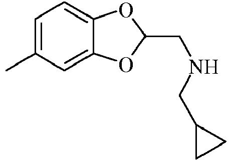 | Cl | 0,00 - 0,10 ppm : m : 2H ; 0,30 - 0,40 ppm : m : 2H ; 0,80 - 0,90 ppm : m : 1H ; 1,40 ppm : s : 9H ; 1,85 ppm : se : 1H ; 2,40 - 2,50 ppm : m : 2H ; 2,95 ppm : d : 2H ; 6,20 ppm : t : 1H; 6,75 ppm : d : 1H ; 7,25 ppm: de : 1H ; 7,45 - 7,70 ppm : m : 3H ; 7,90 ppm : s : 1H ; 8,05 ppm : s : 1H ; 8,20 ppm : s : 1H ; 9,00 ppm : s : 1H ; 10,05 ppm : s : 1H ; 10,70 ppm : s : 1H. |

**TABLEAU 4**

| Composés | Structure | Caractérisation RMN |
|---|---|---|
| 69 | 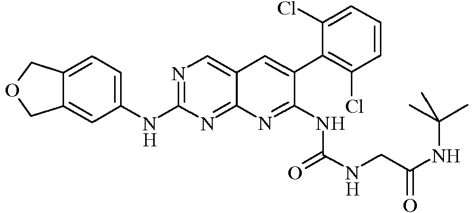 | 1,30 ppm : s : 9H ; 3,90 d : 2H ; 5,00 ppm : d : 4H ; 7,25 ppm : d : 1H; 7,40 ppm - 7,65 ppm : m : 4H ; 7,90 ppm : d : 1H ; 8,00 ppm : s : 1H ; 8,10 ppm : s : 1H ; 8,50 ppm : s : 1H ; 9,10 ppm : s : 1H ; 10,10 ppm : s : 1H ; 10,25 ppm : s : 1H. |
| 70 | 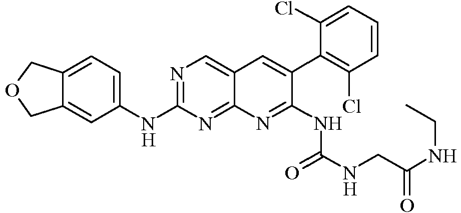 | 1,00 ppm : mt : 3H ; 3,10 ppm : qt : 2H ; 3,90 d : 2H ; 5,00 ppm : d : 4H ; 7,25 ppm : d : 1H; 7,40 ppm - 7,65 ppm : m : 3H ; 7,75 ppm : d : 1H ; 8,05 ppm : s : 1H ; 8,10 ppm : s : 1H ; 8,25 ppm : s : 1H ; 8,60 ppm : s : 1H ; 9,10 ppm : s : 1H ; 10,20 ppm : s : 1H ; 10,30 ppm : s : 1H. |
| 71 | 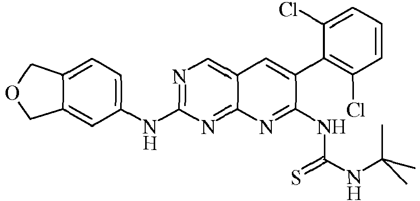 | 1,65 ppm : s : 9H ; 5,00 ppm : s : 4H ; 7,20 ppm : d : 1H; 7,50 ppm - 7,70 ppm : m : 5H ; 8,25 ppm : s: 1H ; 8,30 ppm : s : 1H ; 9,20 ppm : s : 1H ; 10,40 ppm : s : 1H ; 12,80 ppm : s : 1H. |
| 72 | 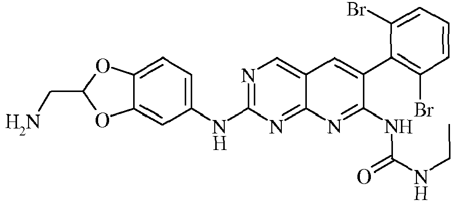 | (DMSO + TFA) 1,10 ppm : t : 3H ; 3,20 ppm : qd : 2H ; 3,45 ppm : d : 2H ; 6,45 ppm : t : 1H ; 7,00 ppm : d : 1H ; 7,25 ppm : de : 1H; 7,45 ppm : t : 1H ; 7,70 ppm : se : 1H ; 7,85 ppm : s : 1H ; 7,90 ppm : s : 1H ; 8,50 ppm : s: 1H ; 9,20 ppm : s : 1H. |
| 73 | 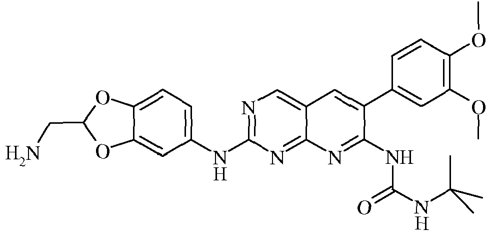 | 1,40 ppm : s : 9H ; 1,60 - 1,80 ppm : se : 2H ; 2,95 ppm: d : 2H ; 3,80 ppm : s : 3H ; 3,85 ppm : s : 3H ; 6, 10 ppm : t : 1H ; 6,75 ppm : d : 1H ; 6,95 ppm - 7,35 ppm : m : 5H; 7,80 ppm: s : 1H ; 8,05 ppm : s : 1H ; 9,05 ppm : s : 1H ; 9,95 ppm : s : 1H ; 10,55 ppm : s : 1H. |
| 74 | 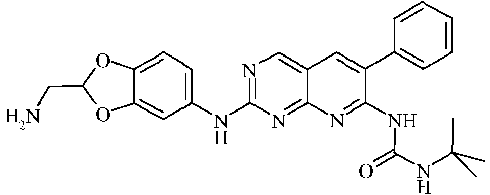 | 1,40 ppm : s : 9H ; 1,50 - 1,70 ppm : se : 2H ; 2,90 ppm: d : 2H ; 6,10 ppm : t: 1H; 6,75 ppm : d : 1H; 7,10 ppm : se : 1H; 7,30 ppm: d : 1H ; 7,50 ppm : se : 5H ; 7,85 ppm : s : 1H ; 8,10 ppm : s: 1H ; 9,05 ppm : s : 1H ; 9,95 ppm : s : 1H ; 10,50 ppm : s : 1H. |
| 75 | 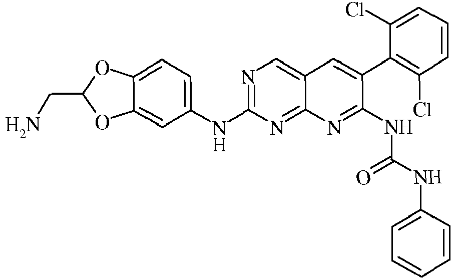 | 1,30 ppm - 1,80 ppm : se : 2H ; 3,00 ppm: d : 2H ; 6,20 ppm : t : 1H ; 6,85 ppm : d : 1H; 7,10 ppm : t : 1H; 7,20 ppm: d : 1H ; 7,40 ppm : t : 2H ; 7,45 ppm - 7,70 ppm : m : 6H ; 8,10 ppm : s: 2H ; 9,10 ppm : s : 1H ; 10,15 ppm : s : 1H ; 13,20 ppm : s : 1H. |
| 76 | 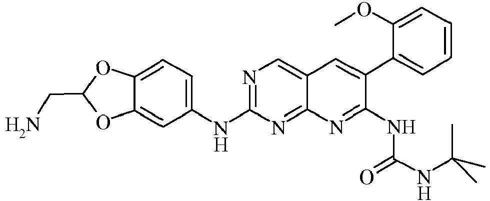 | 1,40 ppm : s : 9H ; 1,50 - 1,65 ppm : se : 2H ; 2,90 ppm: d : 2H ; 3,80 ppm : s : 3H ; 6,10 ppm : t : 1H ; 6,75 ppm : d : 2H ; 7,10 ppm - 7,40 ppm : m : 4H; 7,50 ppm: t : 1H ; 7,85 ppm : s : 1H ; 8,00 ppm : s: 1H ; 9,05 ppm : s : 1H ; 10,00 ppm : s : 1H ; 10,50 ppm : s : 1H. |
| 77 | 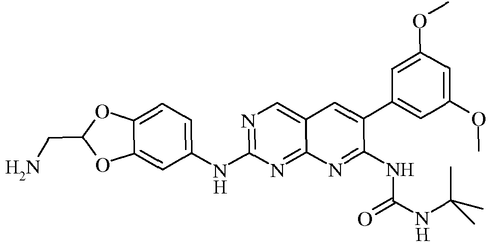 | 1,40 ppm : s : 9H ; 1,50 - 1,65 ppm : s : 2H ; 2,90 ppm: d : 2H ; 3,80 ppm : s : 6H ; 6,10 ppm : t : 1H ; 6,60 ppm : s : 3H ; 6,75 ppm : d : 1H ; 7,20 ppm -7,35 ppm: mt : 2H ; 7,80 ppm : s : 1H ; 8,10 ppm : s: 1H ; 9,05 ppm : s : 1H ; 10,00 ppm : s : 1H ; 10,50 ppm : s : 1H. |
| 78 | 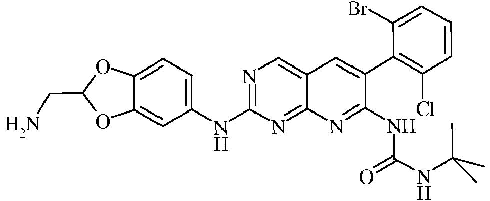 | 1,35 ppm : s : 9H ; 1,50 - 1,70 ppm : se : 2H ; 2,85 ppm: d : 2H ; 6,05 ppm : t : 1H ; 6,70 ppm : d : 1H ; 7,20 ppm - 7,80 ppm: mt : 5H ; 7,95 ppm : se : 1H ; 8,00 ppm : s: 1H ; 9,00 ppm : s : 1H ; 10,00 ppm : s : 1H ; 10,60 ppm : s : 1H. |
| 79 | 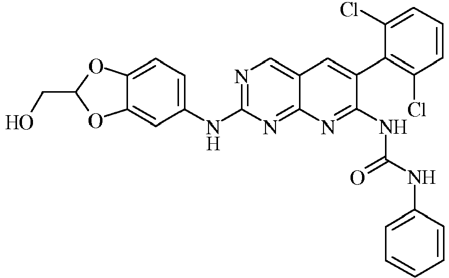 | LC/MS : MH⁺ = 575 |
| 82 | 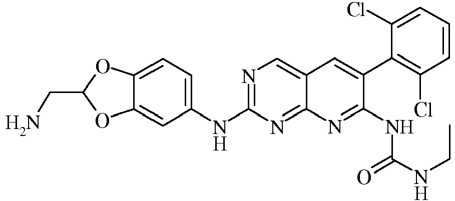 | (DMSO + TFA) 1,05 ppm : t : 3H ; 3,20 ppm : qd : 2H ; 3,40 ppm: d : 2H ; 6,45 ppm : t : 1H ; 6,95 ppm : d : 1H ; 7,20 ppm: de : 1H ; 7,50 - 7,75 ppm : m : 4H ; 8,55 ppm : s: 1H ; 9,20 ppm : s : 1H. |
| 83 | 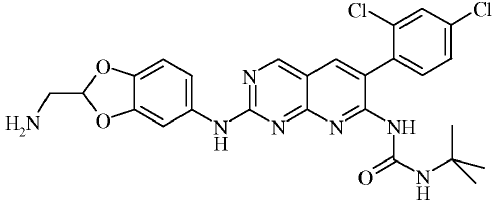 | 1,40 ppm : s : 9H ; 1,60 ppm : se : 2H ; 2,90 ppm: d : 2H ; 6,05 ppm : t : 1H ; 6,75 ppm : d : 1H ; 7,30 ppm : d : 1H ; 7,45 ppm - 7,60 ppm: mt : 2H ; 7,75 - 7,90 ppm : mt : 3H ; 8,00 ppm : s: 1H ; 9,00 ppm : s : 1H ; 10,05 ppm : s : 1H; 10,60 ppm : s : 1H. |

Le Composé 34 a fait l'objet d'une séparation en ses deux énantiomères par chromatographie chirale. Enantiomère dextrogyre : [α]_{D} = +72,1° ; t = 25°C ; C = 0,5 (MeOH). Enantiomère lévogyre : [α]_{D} = -73,0° ; t = 25°C ; C = 0,5 (MeOH).

Des librairies de produits selon l'invention ont également été préparées en mettant en oeuvre les techniques classiques de la chimie combinatoire et en application des procédures de préparation décrites dans la présente invention. La structure des produits préparés ainsi que leur caractérisation sont présentées ici.

Les analyses LC/MS ont été réalisées sur un ; appareil Micromass, modèle LCT, couplé à un appareil de modèle HP1100. L'abondance des composés a été mesurée avec un détecteur à barrette de diodes G1315A dans le domaine de longueur d'onde de 200-600 nm et à l'aide d'un détecteur évaporatif à diffusion de lumière (DEDL) Sedex, modèle 65. Les spectres de masse ont été enregistrés dans un domaine de 180 à 800 (M/z). Les données ont été analysées via un logiciel Micromass MassLynx. Les séparations ont été conduites sur une colonne Hypersil BDS C18 (50 x 4,6 mm), taille des particules : 3µm. Elution par un gradient de 5 à 90 % d'acétonitrile contenant 0,05 % (v/v) d'acide trifluoroacétique (TFA), dans de l'eau contenant 0,05 % (v/v) de TFA en 3,5 minutes à un débit de 1 mL/minute. La durée totale de la procédure, incluant le rééquilibrage de la colonne, est de 7 minutes.

**TABLEAU 5**

| 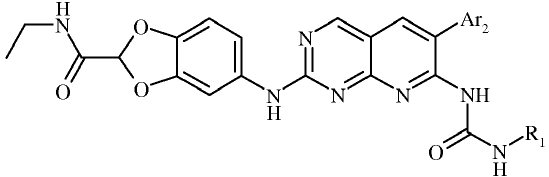 | | | |
|---|---|---|---|
| **Composés** | **R₁** | **Ar₂** | **LC/MS (temps de rétention (minutes); MH⁺ ; MH⁻)** |
| 1 | Phényl | 2,6-Dichlorophényl | 4,42 ; 616 ; 614 |
| 2 | *t-*Butyl | 3,5-Diméthoxyphényl | 4,24 ; 588; 586 |
| 3 | Ethyl | 2,6-Dichlorophényl | 3,77 ; 568 ; 566 |
| 4 | *t-*Butyl | 3,4-Diméthoxyphényl | 3,93 ; 588 ; 586 |
| 5 | *t-*Butyl | Phényl | 3,80 ; 528 ; 526 |
| 6 | *t-*Butyl | 2-Méthoxyphényl | 4,06 ; 558; 556 |
| 7 | *t-*Butyl | 2,6-Dibromophényl | 4,32 ; 685 ; 684 |
| 8 | *t-*Butyl | 2-Bromo-6-chlorophényl | 4 29 ; 640 ; 638 |
| 9 | Ethyl | 2,6-Dibromophényl | 3,83 ; 658 ; 656 |
| 10 | Phényl | 2-Bromo-6-chlorophényl | 4,46 ; 660 ; 658 |
| 11 | Phényl | 2,6-Dibromophényl | 4,49 ; -- ; 704 |

**TABLEAU 6**

| 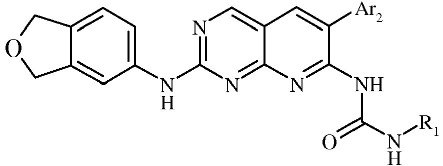 | | | |
|---|---|---|---|
| **Composés** | **R₁** | **Ar₂** | **LC/MS (temps de rétention (minutes); MH⁺)** |
| 1 | Phényl | 2,6-Dichlorophényl | 4,75 ; 543,28 |
| 2 | *t*-Butyl | 3,5-Diméthoxyphényl | 4,52 ; 515,42 |
| 3 | Ethyl | 2,6-Dichlorophényl | 4,95 ; 495,30 |
| 4 | *t*-Butyl | 3,4-Diméthoxyphényl | 4,21 ; 515,43 |
| 5 | *t*-Butyl | Phényl | 4,42 ; 455,41 |
| 6 | *t*-Butyl | 2-Méthoxyphényl | 4,34 ; 485,41 |
| 7 | *t*-Butyl | 2,6-Dibromophényl | 4,63 ; 611,25 |
| 8 | *t*-Butyl | 2-Bromo-6-chlorophényl | 4,72 ; 567,09 |
| 9 | Ethyl | 2,6-Dibromophényl | 4,14 ; 582,96 |
| 10 | Phényl | 2-Bromo-6-chlorophényl | 4,88 ; 587,06 |
| 11 | Phényl | 2,6-Dibromophényl | 4,91 ; 630,97 |

Les composés selon l'invention ont fait l'objet d'essais pharmacologiques permettant de déterminer leur activité anticancéreuse.

Les composés de formule (I) selon la présente invention ont été testés in vitro sur un panel de lignées tumorales d'origine humaine provenant :
- de cancer du sein: MDA-MB231 (American Type culture collection, Rockville, Maryland, USA, ATCC-HTB26), MDA-A1 ou MDA-ADR (dite lignée multi-drug resistant MDR, et décrite par E.Collomb et al., dans Cytometry, 12(1):15-25, 1991), et MCF7 (ATCC-HTB22),
- de cancer de la prostate: DU145 (ATCC-HTB81) et PC3 (ATCC-CRL1435),
- de cancer du colon: HCT116 (ATCC-CCL247) et HCT15 (ATCC-CCL225),
- de cancer du poumon: H460 (décrite par Carmichael dans Cancer Research 47 (4):936-942, 1987 et délivré par le National Cancer Institute, Frederick Cancer Research and Development Center, Frederick, Maryland, USA),
- de glioblastome (SF268 décrite par Westphal dans Biochemical & Biophysical Research Communications 132 (1): 284-289, 1985 et délivré par le National Cancer institute, Frederick Cancer Research and Development Center, Frederick, Maryland, USA),
- de leucémie (CMLT1 décrite par Kuriyama et al. dans Blood, 74: 1989, 1381-1387, par Soda et al. dans British Journal of Haematology, 59: 1985, 671-679 et par Drexler, dans Leukemia Research, 18: 1994, 919-927 et délivré par la société DSMZ, Mascheroder Weg 1b, 38124 Braunschweig, Germany).

La prolifération et la viabilité cellulaire ont été déterminée dans un test utilisant le 3-(4,5-diméthylthiazol-2-yl)-5-(3-carboxyméthoxyphényl)-2-(4-sulfophényl)-2*H-*tétrazolium (MTS) selon Fujishita T. et al., Oncology, 2003, 64 (4), 399-406. Dans ce test, on mesure la capacité mitochondriale des cellules vivantes à transformer le MTS en un composé coloré après 72 heures d'incubation d'un composé de formule (I) selon l'invention. Les concentrations en composé selon l'invention, qui conduisent à 50 % de perte de prolifération et de viabilité cellulaire (CI₅₀) sont comprises entre 1 nM et 10 µM, selon la lignée tumorale et le composé testé.

Ainsi, selon la présente invention, il apparaît que les composés de formule (I) entrainent une perte de prolifération et de viabilité des cellules tumorales. Il apparaît donc que les composés selon l'invention ont une activité anticancéreuse et une activité dans le traitement des autres maladies prolifératives telles que le psoriasis, la resténose, l'arthérosclérose, le SIDA par exemple, ainsi que dans les maladies provoquées par la prolifération des cellules du muscle lisse vasculaire et dans la polyarthrite rhumatoïde.

Ainsi selon un autre de ses aspects, l'invention a pour objet des médicaments qui comprennent un composé de formule (I), ou un sel d'addition de ce dernier à un acide pharmaceutiquement acceptable ou encore un hydrate ou un solvate du composé de formule (I).

Ces médicaments trouvent leur emploi en thérapeutique, notamment dans le traitement ou la prévention des maladies causées ou exacerbées par la prolifération des cellules et en particulier des cellules tumorales.

Comme inhibiteur de la prolifération des cellules tumorales, ces composés sont utiles dans la prévention et le traitement des leucémies, des tumeurs solides à la fois primaires et métastatiques, des carcinomes et cancers, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage ; cancer du foie, cancer de l'estomac, cancer des canaux biliaires, cancer de la vésicule biliaire, cancer du pancréas ; cancers des voies urinaires y compris rein, urothelium et vessie; cancers du tractus génital féminin y compris cancer de l'utérus, du col de l'utérus, des ovaires, chloriocarcinome et trophoblastome; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs malignes hématopoïétiques ; leucémies, (Acute Lymphocytic Leukemia (ALL), Acute Myeloid Leukemia (AML), Chronic Myeloid Leukemia (CML), Chronic lymphocytic leukemia (CLL)) chloromes, plasmocytomes, leucémies des cellules T ou B, lymphomes non hodgkiniens ou hodgkiniens, myélomes, hémopathies malignes diverses.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Les composés de formule (I) ci-dessus peuvent être utilisés à des doses journalières de 0,002 à 2000 mg par kilogramme de poids corporel du mammifère à traiter, de préférence à des doses journalières de 0,1 à 300 mg/kg. Chez l'être humain, la dose peut varier de préférence de 0,02 à 10000 mg par jour, plus particulièrement de 1 à 3000 mg selon l'âge du sujet à traiter ou le type de traitement : prophylactique ou curatif.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvates.

Selon la présente invention, le ou les composés de formule (I) peuvent être administrés en association avec un (ou plusieurs) principe(s) actif(s) anticancéreux, en particulier des composés antitumoraux tels que les agents alkylants tels que les alkylsulfonates (busulfan), la dacarbazine, la procarbazine, les moutardes azotées (chlorméthine, melphalan, chlorambucil), cyclophosphamide, ifosfamide; les nitrosourées tels que la carmustine, la lomustine, la sémustine, la streptozocine; les alcaloïdes antinéoplasiques tels que la vincristine, la vinblastine ; les taxanes tel que le paclitaxel ou le taxotère ; les antibiotiques antinéoplasiques tels que l'actinomycine; les agents intercalants, les antimétabolites antinéoplasiques, les antagonistes des folates, le méthotrexate; les inhibiteurs de la synthèse des purines; les analogues de la purine tels que mercaptopurine, 6-thioguanine; les inhibiteurs de la synthèse des pyrimidines, les inhibiteurs d'aromatase, la capécitabine, les analogues de la pyrimidine tels que fluorouracil, gemcitabine, cytarabine et cytosine arabinoside; le bréquinar ; les inhibiteurs de topoisomérases tels que la camptothécine ou l'étoposide ; les agonistes et antagonistes hormonaux anticancéreux incluant le tamoxifène; les inhibiteurs de kinase, l'imatinib; les inhibiteurs de facteurs de croissance; les antiinflammatoires tels que le pentosane polysulfate, les corticostéroïdes, la prednisone, la dexamethasone; les antitopoisomérases tels que l'étoposide, les antracyclines incluant la doxorubicine, la bléomycine, la mitomycine et la méthramycine; les complexes métalliques anticancéreux, les complexes du platine, le cisplatine, le carboplatine, l'oxaliplatine ; l'interféron alpha, le triphénylthiophosphoramide, l'altrétamine ; les agents antiangiogéniques ; la thalidomide ; les adjuvants d'immunothérapie ; les vaccins.

Selon la présente invention les composés de formule (I) peuvent également être administrés en association avec un ou plusieurs autres principes actifs utiles dans une des pathologies indiquées ci-dessus, par exemple un agent anti-émétiques, anti-douleurs, anti-inflammatoires, anti-cachexie.

Un produit conforme à l'invention pourra être utilisé pour la fabrication d'un médicament utile pour traiter un état pathologique, en particulier un cancer.

La présente invention concerne aussi les compositions thérapeutiques contenant un composé selon l'invention, en association avec un excipient pharmaceutiquement acceptable selon le mode d'administration choisi. La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

Les formes liquides seront de préférence injectables et, de ce fait, auront une formulation acceptable pour une telle utilisation.

Des voies d'administration par injection acceptables incluent les voies intraveineuse, intra-péritonéale, intramusculaire, et sous cutanée, la voie intraveineuse étant préférée.

La dose administrée des composés de l'invention sera adaptée par le praticien en fonction de la voie d'administration du patient et de l'état de ce dernier.

Les composés de la présente invention peuvent être administrés seuls ou en mélange avec d'autres anticancéreux. Parmi les associations possibles on peut citer :
- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine
- les dérivés du platine comme notamment le cisplatine, le carboplatine ou l'oxaliplatine
- les agents antibiotiques comme notamment la bléomycine, la mitomycine, la dactinomycine
- les agents antimicrotubules comme notamment la vinblastine, la vincristine, la vindésine, la vinorelbine, les taxoides (paclitaxel et docétaxel)
- les anthracyclines comme notamment la doxorubicine, la daunorubicine, l'idarubicine, l'épirubicine, la mitoxantrone, la losoxantrone
- les topoisomérases des groupes I et II telles que l'étoposide, le teniposide, l'amsacrine, l'irinotecan, le topotecan et le tomudex
- les fluoropyrimidines telles que le 5-fluorouracile, l'UFT, la floxuridine
- les analogues de cytidine telles que la 5-azacytidine, la cytarabine, la gemcitabine, la 6-mercaptomurine, la 6-thioguanine
- les analogues d'adénosine telles que la pentostatine, la cytarabine ou le phosphate de fludarabine
- le méthotrexate et l'acide folinique
- les enzymes et composés divers tels que la L-asparaginase, l'hydroxyurée, l'acide trans-rétinoique, la suramine, la dexrazoxane, l'amifostine, l'herceptin ainsi que les hormones oestrogéniques, androgéniques
- les agents antivasculaires tels que les dérivés de la combretastatine ou de la colchicine et leurs prodrogues.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

## Revendications

1. Composé de formule (I) : 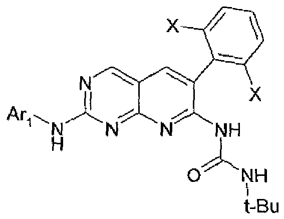 dans laquelle :
- soit Ar₁ représente 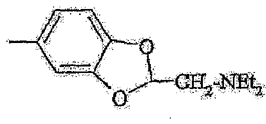 et X représente Cl ;
- soit Ar₁ représente 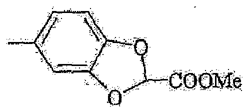 et X représente Cl ;
- soit Ar₁ représente 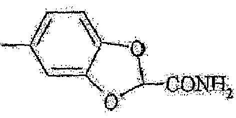 et X représente Cl ;
- soit Ar₁ représente 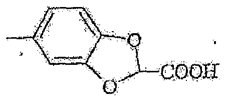 et X représente Cl ;
- soit Ar₁ représente 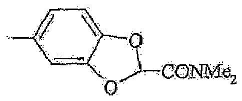 et X représente Cl ;
- soit Ar₁ représente 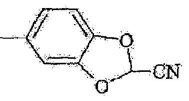 et X représente Cl ;
- soit Ar₁ représente 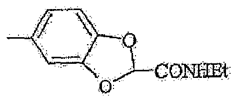 et X représente Cl ;
- soit Ar₁ représente 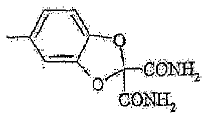 et X représente Cl ;
- soit Ar₁ représente 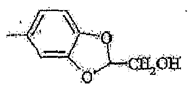 et X représente Cl ;
- soit Ar₁ représente 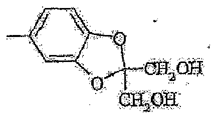 et X représente Cl ;
- soit Ar₁ représente 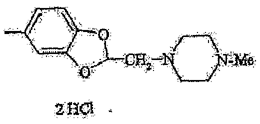 et X représente Cl ;
- soit Ar₁ représente 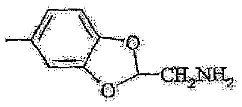 et X représente Cl ;
- soit Ar₁ représente 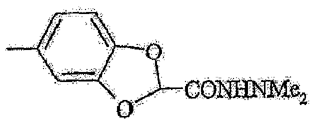 et X représente Cl ;
- soit Ar₁ représente 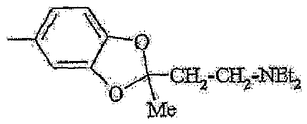 et X représente Cl ;
- soit Ar₁ représente 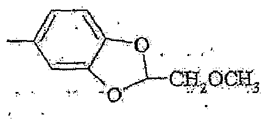 et X représente Cl ;
- soit Ar₁ représente 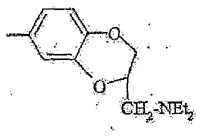 et X représente Cl ;
- soit Ar₁ représente 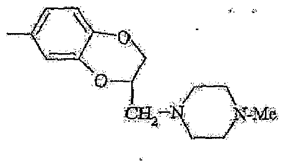 et X représente Cl ;
- soit Ar₁ représente 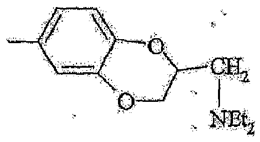 et X représente Cl ;
- soit Ar₁ représente 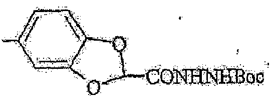 et X représente Cl ;
- soit Ar₁ représente 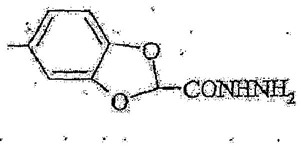 et X représente Cl ;
- soit Ar₁ représente 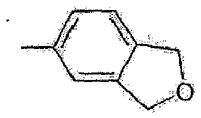 et X représente Cl ;
- soit Ar₁ représente 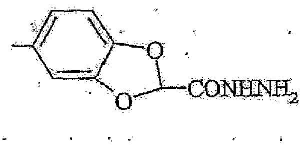 et X représente Cl ;
- soit Ar₁ représente 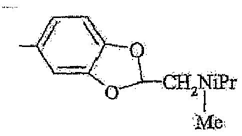 et X représente Cl ;
- soit Ar₁ représente 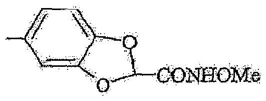 et X représente Cl ;
- soit Ar₁ représente 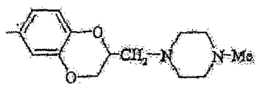 et X représente Cl ;
- soit Ar₁ représente 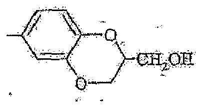 et X représente Cl ;
- soit Ar₁ représente 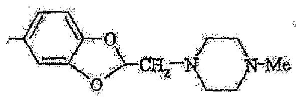 et X représente Me;
- soit Ar₁ représente 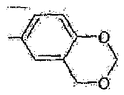 et X représente Cl ;
- soit Ar₁ représente 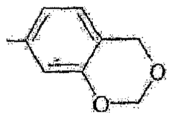 et X représente Cl ;
- soit Ar₁ représente 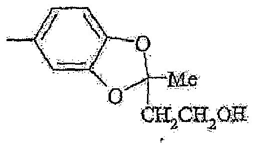 et X représente Cl ;
- soit Ar₁ représente 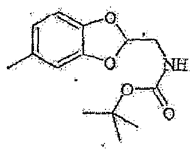 et X représente Cl ;
- soit Ar₁ représente 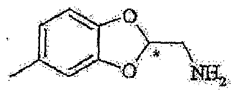 (dextrogyre) et X représente Cl ;
- soit Ar₁ représente 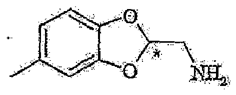 (levogyre) et X représente Cl ;
- soit Ar₁ représente 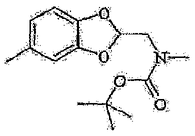 et X représente Cl ;
- soit Ar₁ représente 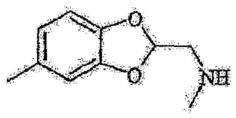 et X représente Cl ;
- soit Ar₁ représente 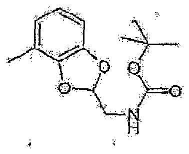 et X représente Cl ;
- soit Ar₁ représente 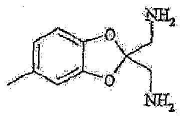 et X représente Cl ;
- soit Ar₁ représente 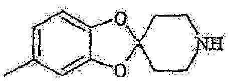 et X représente Cl ;
- soit Ar₁ représente 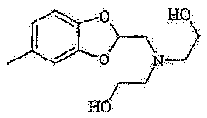 et X représente Cl ;
- soit Ar₁ représente 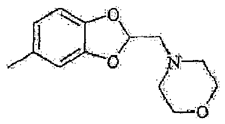 et X représente Cl ;
- soit Ar₁ représente 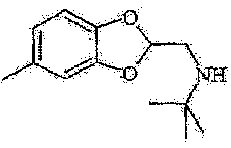 et X représente Cl ;
- soit Ar₁ représente 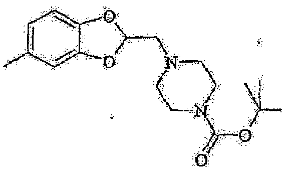 et X représente Cl ;
- soit Ar₁ représente 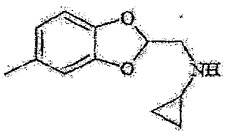 et X représente CI ;
- soit Ar₁ représente 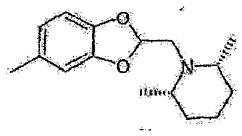 et X représente Cl ;
- soit Ar₁ représente 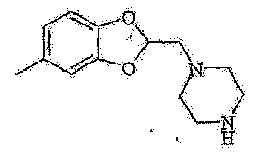 et X représente Cl ;
- soit Ar₁ représente 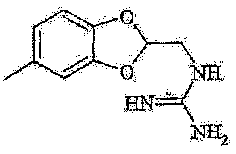 et X représente Cl ;
- soit Ar₁ représente 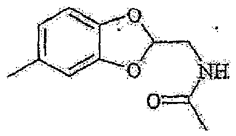 et X représente Cl ;
- soit Ar₁ représente 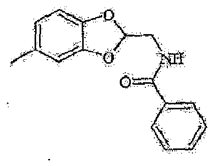 et X représente Cl ;
- soit Ar₁ représente 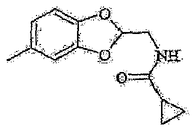 et X représente Cl ;
- soit Ar₁ représente 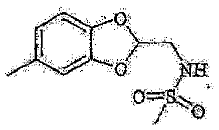 et X représente Cl ;
- soit Ar₁ représente 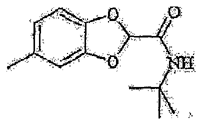 et X représente Cl ;
- soit Ar₁ représente 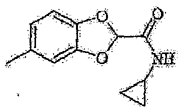 et X représente Cl ;
- soit Ar₁ représente 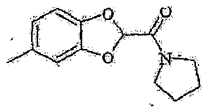 et X représente Cl ;
- soit Ar₁ représente 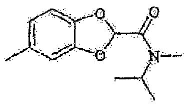 et X représente Cl ;
- soit Ar₁ représente 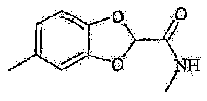 et X représente Cl ;
- soit Ar₁ représente 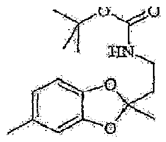 et X représente Cl ;
- soit Ar₁ représente 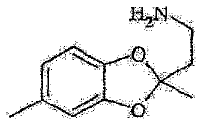 et X représente Cl ;
- soit Ar₁ représente 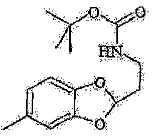 et X représente Cl ;
- soit Ar₁ représente 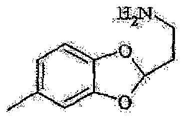 et X représente Cl ;
- soit Ar₁ représente 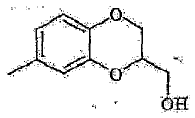 et X représente Cl ;
- soit Ar₁ représente 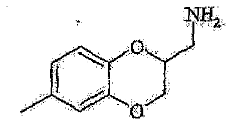 et X représente Cl ;
- soit Ar₁ représente 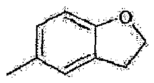 et X représente Cl ;
- soit Ar₁ représente 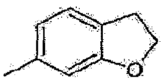 et X représente Cl ;
- soit Ar₁ représente 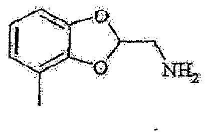 et X représente Cl ;
- soit Ar₁ représente 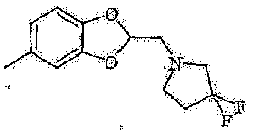 et X représente Cl ;
- soit Ar₁ représente 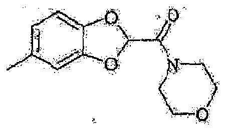 et X représente Cl ;
- soit Ar₁ représente 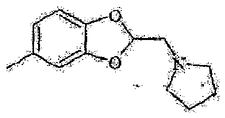 et X représente Cl ;
- soit Ar₁ représente 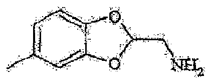 et X représente Br ;
- soit Ar₁ représente 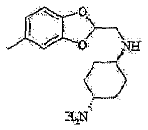 et X représente Cl ;
- soit Ar₁ représente 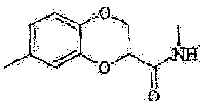 et X représente Cl ;
- soit Ar₁ représente 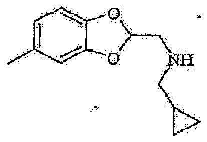 et X représente Cl ;

2. Composé choisi parmi l'un des composés suivants :

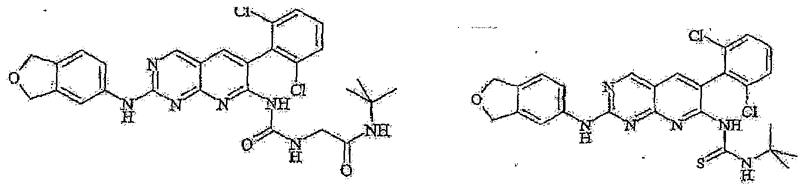

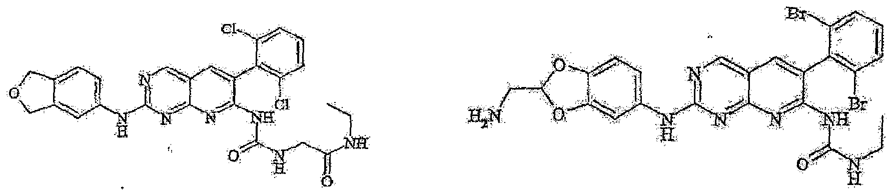

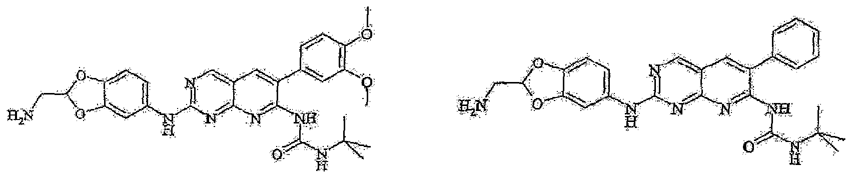

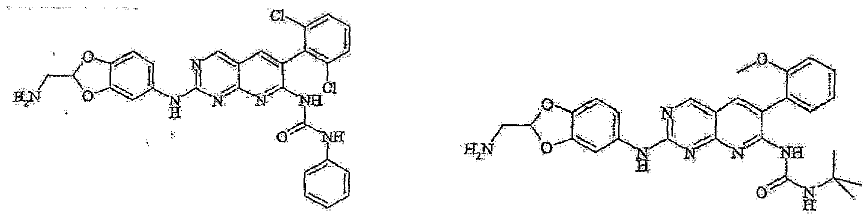

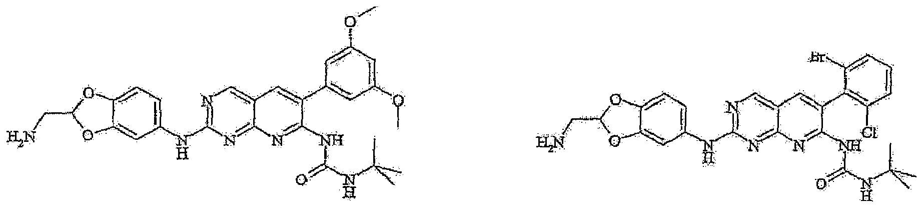

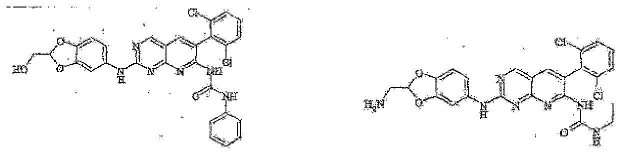

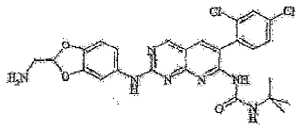

3. Composé répondant à la formule suivante : 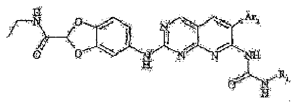 dans laquelle :
- soit R1 représente Phényle et Ar2 représente 2,6-dichlorophényle ;
- soit R1 représente t-Butyle et Ar2 représente 3,5-diméthoxyphényle ;
- soit R, représente éthyle et Ar2 représente 2,6-dichlorophényle ;
- soit R1 représente t-Butyle et Ar2 représente 3,4-diméthoxyphényle ;
- soit R1 représente t-Butyle et Ar2 représente phényle ;
- soit R1 représente t-Butyle et Ar2 représente 2-méthoxyhényle ;
- soit R1 représente t-Butyle et Ar2 représente 2,6-dibromophényle ;
- soit R1 représente t-Butyle et Ar2 représente 2-bromo-6-chlorophényle ;
- soit R1 représente éthyle et Ar2 représente 2,6-dibromophényle ;
- soit R, représente Phényle et Ar2 représente 2-bromo-6-chlorophényle ;
- soit R1 représente Phényle et Ar2 représente 2,6-dibromophényle ;

4. Composé répondant à la formule suivante : 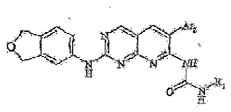 dans laquelle :
- soit R1 représente Phényle et Ar2 représente 2,6-dichlorophényle ;
- soit R1 représente t-Butyle et Ar2 représente 3,5-diméthoxyphényle ;
- soit R1 représente éthyle et Arz représente 2,6-dichlorophényle ;
- soit R1 représente t-Butyle et Ar2 représente 3,4-diméthoxyphényle ;
- soit R1 représente t-Butyle et Ar2 représente phényle ;
- soit R1 représente t-Butyle et Ar2 représente 2-méthoxyhényle ;
- soit R1 représente t-Butyle et Arz représente 2,6-dibromophényle ;
- soit R1 représente t-Butyle et Ar2 représente 2-bromo-6-chlorophényle ;
- soit R1 représente éthyle et Ar2 représente 2,6-dibromophényle ;
- soit R1 représente Phényle et Arz représente 2-bromo-6-chlorophényle ;
- soit R, représente Phényle et Ar2 représente 2,6-dibromophényle.

## Patentansprüche

1. Verbindung der Formel (I):
worin: 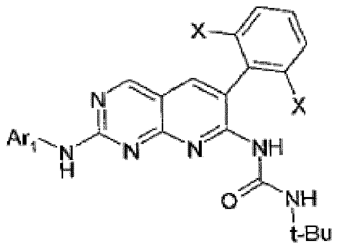
- entweder Ar₁ für 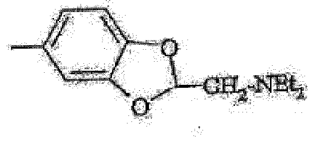 steht und X für Cl steht;
- oder Ar₁ für 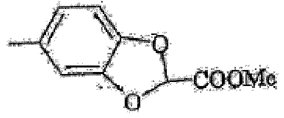 steht und X für Cl steht;
- oder Ar₁ für 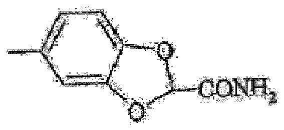 steht und X für Cl steht;
- oder Ar₁ für 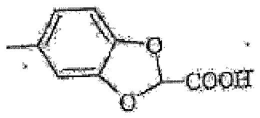 steht und X für Cl steht;
- oder Ar₁ für 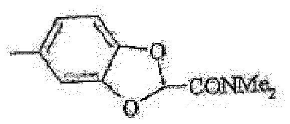 steht und X für Cl steht;
- oder Ar₁ für steht; 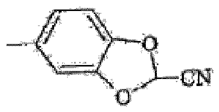 steht und X für Cl
- oder Ar₁ für Cl steht; 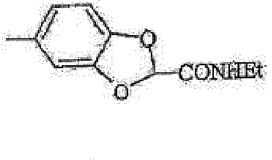 steht und X für
- oder Ar₁ für 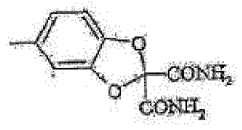 steht und X für Cl steht;
- oder Ar₁ für 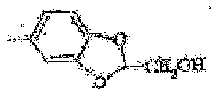 steht und X für Cl steht;
- oder Ar₁ für 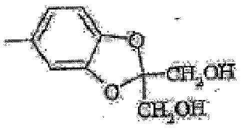 steht und X für Cl steht;
- oder Ar₁ für 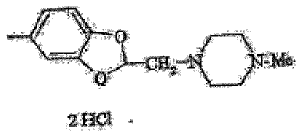 steht und X für Cl steht;
- oder Ar₁ für 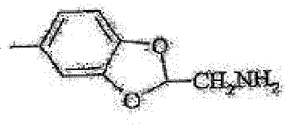 steht und X für Cl steht;
- oder Ar₁ für 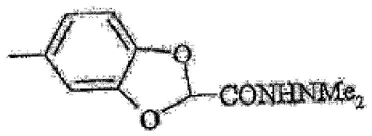 steht und X für Cl steht;
- oder Ar₁ für 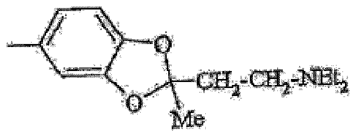 steht und X für Cl steht;
- oder Ar₁ für 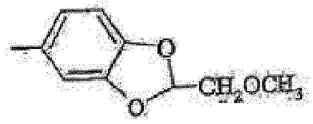 steht und X für Cl steht;
- oder Ar₁ für 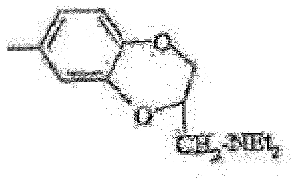 steht und X für Cl steht;
- oder Ar₁ für 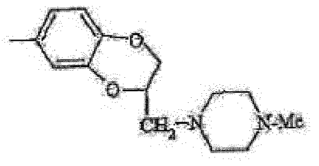 steht und X für Cl steht;
- oder Ar₁ für 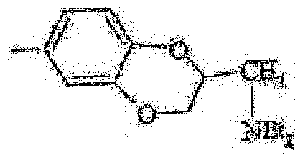 steht und X für Cl steht;
- oder Ar₁ für 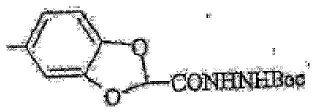 steht und X für Cl steht;
- oder Ar₁ für 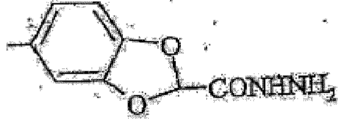 steht und X für Cl steht;
- oder Ar₁ für 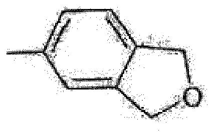 steht und X für Cl steht;
- oder Ar₁ für 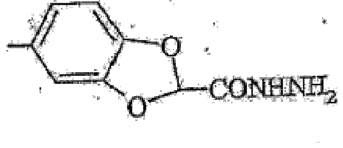 steht und X für Cl steht;
- oder Ar₁ für 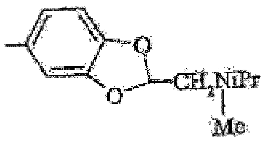 steht und X für Cl steht;
- oder Ar₁ für 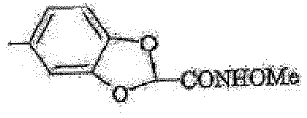 steht und X für Cl steht;
- oder Ar₁ für 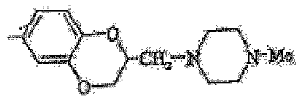 steht und X für Cl steht;
- oder Ar₁ für 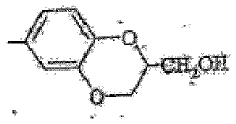 steht und X für Cl steht;
- oder Ar₁ für 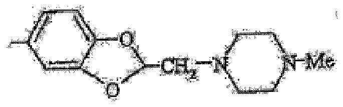 steht und X für Me steht;
- oder Ar₁ für 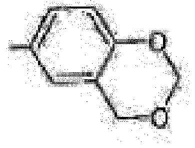 steht und X für Cl steht;
- oder Ar₁ für 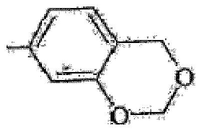 steht und X für Cl steht;
- oder Ar₁ für 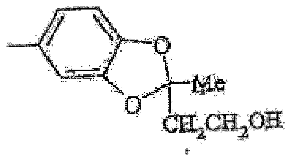 steht und X für Cl steht;
- oder Ar₁ für 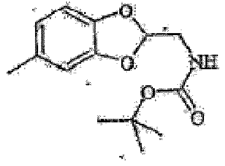 steht und X für Cl steht;
- oder Ar₁ für 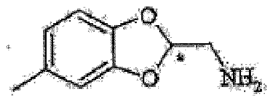 (rechtsdrehend) steht und X für Cl steht;
- oder Ar₁ für 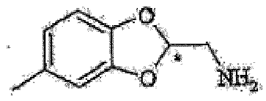 (linksdrehend) steht und X für Cl steht;
- oder Ar₁ für 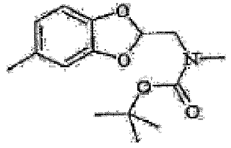 steht und X für Cl steht;
- oder Ar₁ für 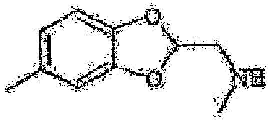 steht und X für Cl steht;
- oder Ar₁ für 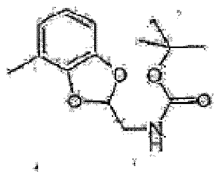 steht und X für Cl steht;
- oder Ar₁ für 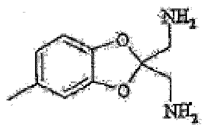 steht und X für Cl steht;
- oder Ar₁ für 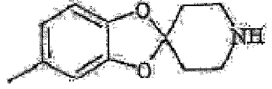 steht und X für Cl steht;
- oder Ar₁ für 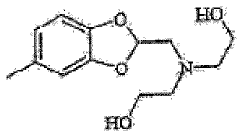 steht und X für Cl steht;
- oder Ar₁ für 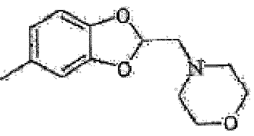 steht und X für Cl steht;
- oder Ar₁ für 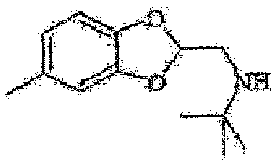 steht und X für Cl steht;
- oder Ar₁ für 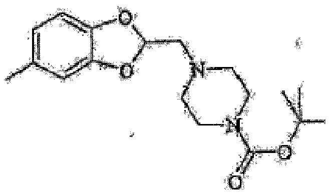 steht und X für Cl steht;
- oder Ar₁ für 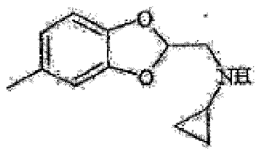 steht und X für Cl steht;
- oder Ar₁ für 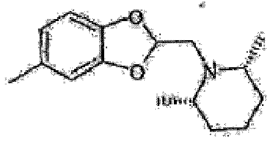 steht und X für Cl steht;
- oder Ar₁ für 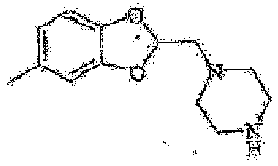 steht und X für Cl steht;
- oder Ar₁ für 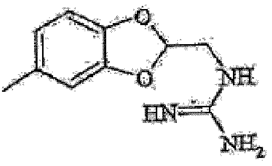 steht und X für Cl steht;
- oder Ar₁ für 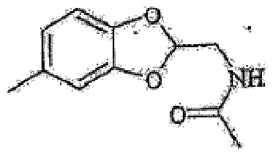 steht und X für Cl steht;
- oder Ar₁ für 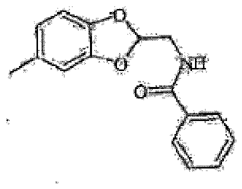 steht und X für Cl steht;
- oder Ar₁ für 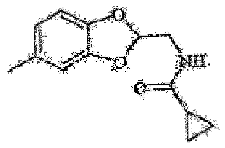 steht und X für Cl steht;
- oder Ar₁ für 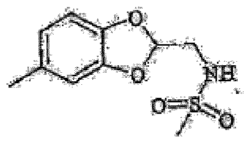 steht und X für Cl steht;
- oder Ar₁ für 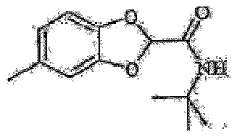 steht und X für Cl steht;
- oder Ar₁ für 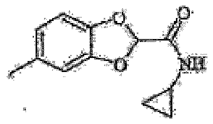 steht und X für Cl steht;
- oder Ar₁ für 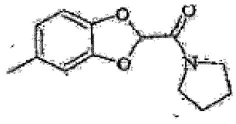 steht und X für Cl steht;
- oder Ar₁ für 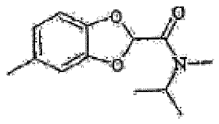 steht und X für Cl steht;
- oder Ar₁ für 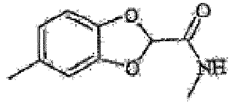 steht und X für Cl steht;
- oder Ar₁ für 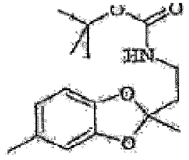 steht und X für Cl steht;
- oder Ar₁ für 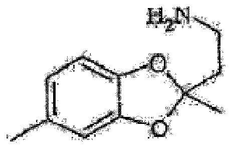 steht und X für Cl steht;
- oder Ar₁ für 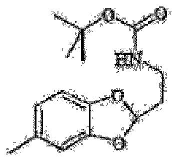 steht und X für Cl steht;
- oder Ar₁ für 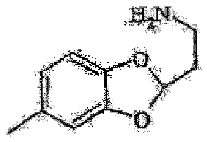 steht und X für Cl steht;
- oder Ar₁ für 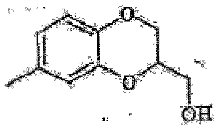 steht und X für Cl steht;
- oder Ar₁ für 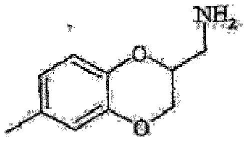 steht und X für Cl steht;
- oder Ar₁ für 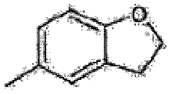 steht und X für Cl steht;
- oder Ar₁ für 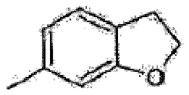 steht und X für Cl steht;
- oder Ar₁ für 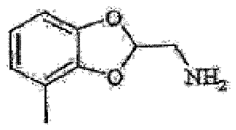 steht und X für Cl steht;
- oder Ar₁ für 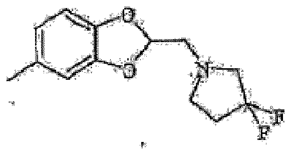 steht und X für Cl steht;
- oder Ar₁ für 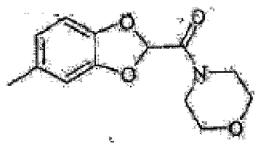 steht und X für Cl steht;
- oder Ar₁ für 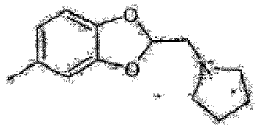 steht und X für Cl steht;
- oder Ar₁ für 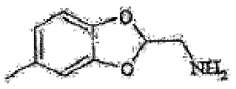 steht und X für Br steht;
- oder Ar₁ für 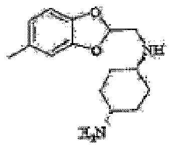 steht und X für Cl steht;
- oder Ar₁ für 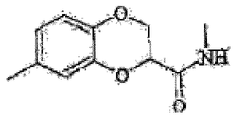 steht und X für Cl steht;
- oder Ar₁ für 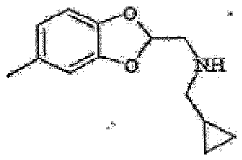 steht und X für Cl steht.

2. Verbindung, ausgewählt aus den folgenden Verbindungen:

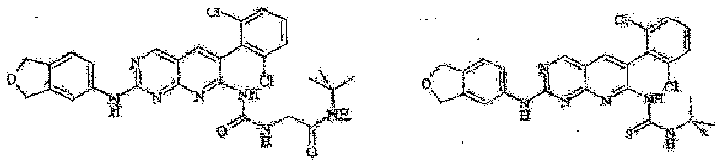

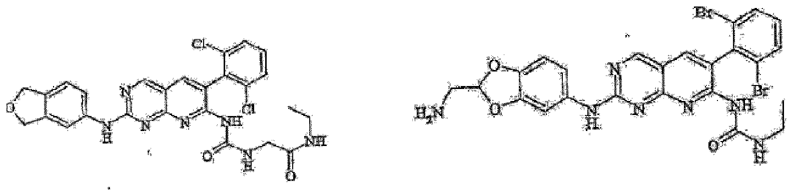

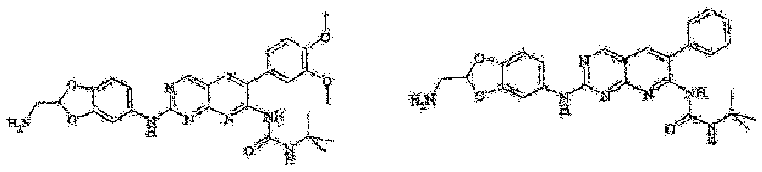

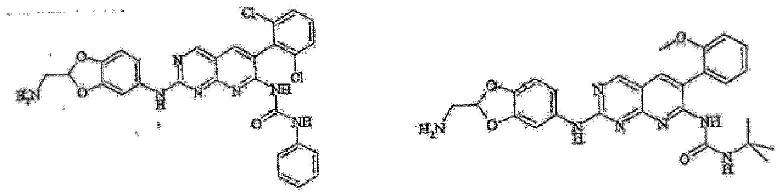

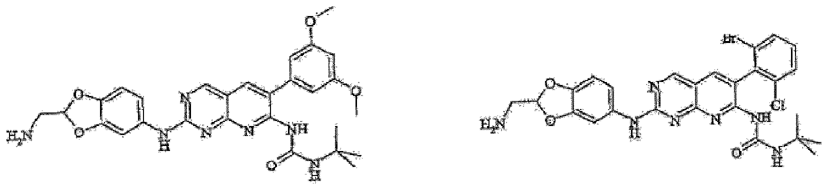

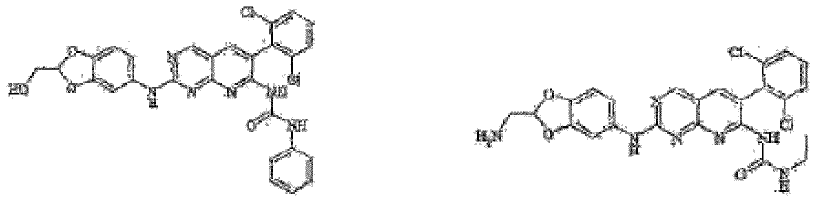

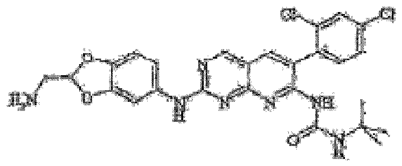

3. Verbindung der folgenden Formel: 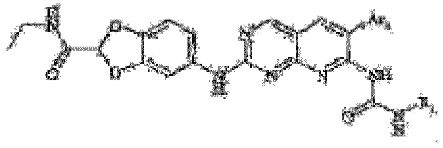 worin:
- entweder R1 für Phenyl steht und Ar2 für 2,6-Dichlorphenyl steht;
- oder R1 für t-Butyl steht und Ar2 für 3,5-Dimethoxyphenyl steht;
- oder R1 für Ethyl steht und Ar2 für 2,6-Dichlorphenyl steht;
- oder R1 für t-Butyl steht und Ar2 für 3,4-Dimethoxyphenyl steht;
- oder R1 für t-Butyl steht und Ar2 für Phenyl steht;
- oder R1 für t-Butyl steht und Ar2 für 2-Methoxyphenyl steht;
- oder R1 für t-Butyl steht und Ar2 für 2,6-Dibromphenyl steht;
- oder R1 für t-Butyl steht und Ar2 für 2-Brom-6-chlorphenyl steht;
- oder R1 für Ethyl steht und Ar2 für 2, 6-Dibromphenyl steht;
- oder R1 für Phenyl steht und Ar2 für 2-Brom-6chlorphenyl steht;
- oder R1 für Phenyl steht und Ar2 für 2,6-Dibromphenyl steht.

4. Verbindung der folgenden Formel: 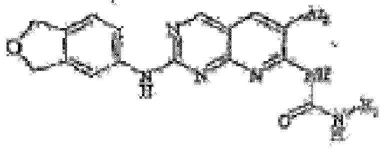 worin:
- entweder R1 für Phenyl steht und Ar2 für 2,6-Dichlorphenyl steht;
- oder R1 für t-Butyl steht und Ar2 für 3,5-Dimethoxyphenyl steht;
- oder R1 für Ethyl steht und Ar2 für 2,6-Dichlorphenyl steht;
- oder R1 für t-Butyl steht und Ar2 für 3,4-Dimethoxyphenyl steht;
- oder R1 für t-Butyl steht und Ar2 für Phenyl steht;
- oder R1 für t-Butyl steht und Ar2 für 2-Methoxyphenyl steht;
- oder R1 für t-Butyl steht und Ar2 für 2,6-Dibromphenyl steht;
- oder R1 für t-Butyl steht und Ar2 für 2-Brom-6-chlorphenyl steht;
- oder R1 für Ethyl steht und Ar2 für 2, 6-Dibromphenyl steht;
- oder R1 für Phenyl steht und Ar2 für 2-Brom-6-chlorphenyl steht;
- oder R1 für Phenyl steht und Ar2 für 2,6-Dibromphenyl steht.

## Claims

1. Compound of formula (I): 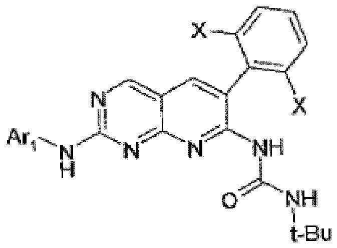 in which:
- either Ar₁ represents 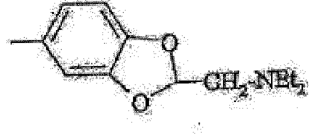 and X represents Cl;
- or Ar₁ represents 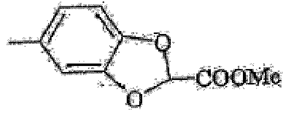 and X represents Cl;
- or Ar₁ represents 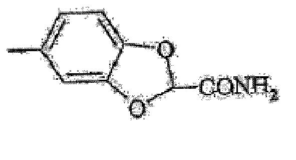 and X represents Cl;
- or Ar₁ represents 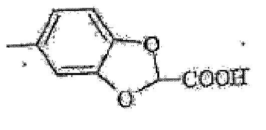 and X represents Cl;
- or Ar₁ represents 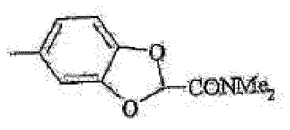 and X represents Cl;
- or Ar₁ represents 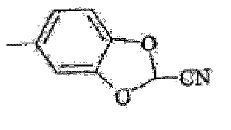 and X represents Cl;
- or Ar₁ represents 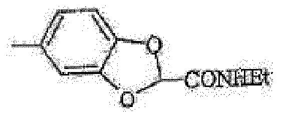 and X represents Cl;
- or Ar₁ represents 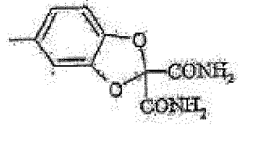 and X represents Cl;
- or Ar₁ represents 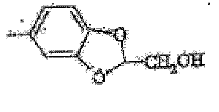 and X represents Cl;
- or Ar₁ represents 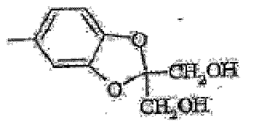 and X represents Cl;
- or Ar₁ represents 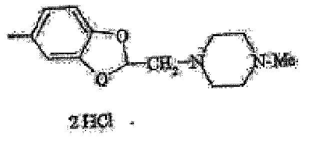 and X represents Cl;
- or Ar₁ represents 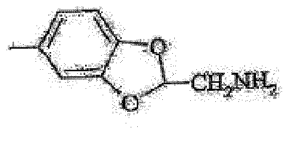 and X represents Cl;
- or Ar₁ represents 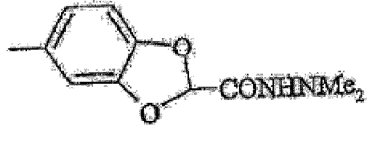 and X represents Cl;
- or Ar₁ represents 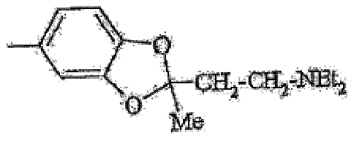 and X represents Cl;
- or Ar₁ represents 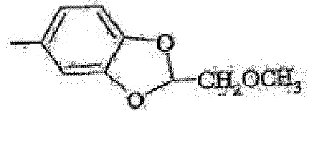 and X represents Cl;
- or Ar₁ represents 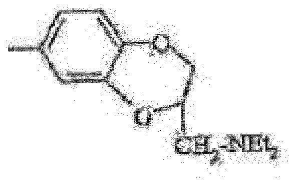 and X represents Cl;
- or Ar₁ represents 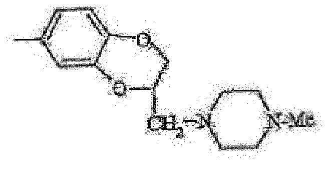 and X represents Cl;
- or Ar₁ represents 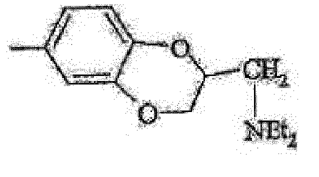 and X represents Cl;
- or Ar₁ represents 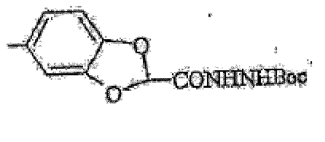 and X represents Cl;
- or Ar₁ represents 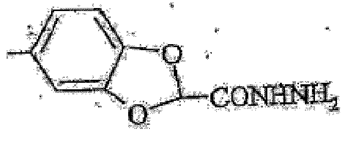 and X represents Cl;
- or Ar₁ represents 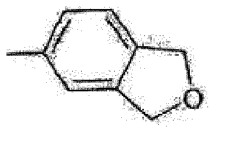 and X represents Cl;
- or Ar₁ represents 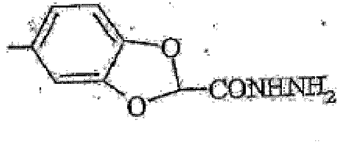 and X represents Cl;
- or Ar₁ represents 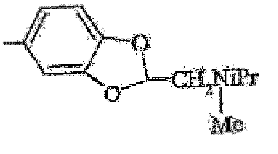 and X represents Cl;
- or Ar₁ represents 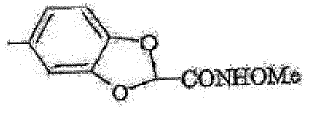 and X represents Cl;
- or Ar₁ represents 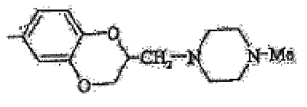 and X represents Cl;
- or Ar₁ represents 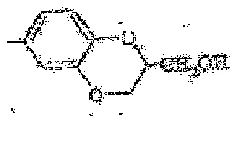 and X represents Cl;
- or Ar₁ represents 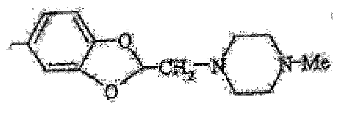 and X represents Me;
- or Ar₁ represents 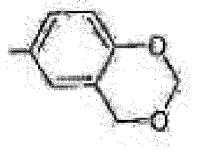 and X represents Cl;
- or Ar₁ represents 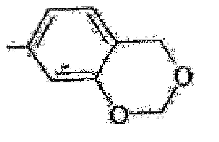 and X represents Cl;
- or Ar₁ represents 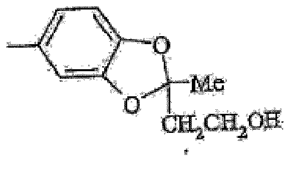 and X represents Cl;
- or Ar₁ represents 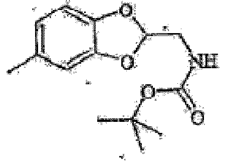 and X and X represents Cl;
- or Ar₁ represents 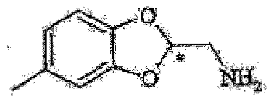 (dextrogyre) and X represents Cl;
- or Ar₁ represents 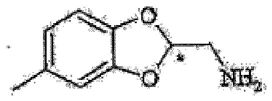 (levogyre) and X represents Cl;
- or Ar₁ represents 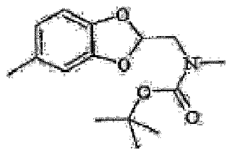 and X represents Cl;
- or Ar₁ represents 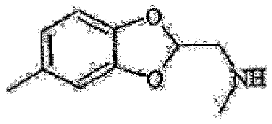 and X represents Cl;
- or Ar₁ represents 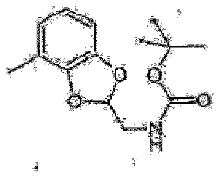 and X represents Cl;
- or Ar₁ represents 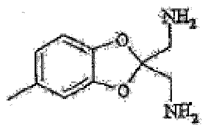 and X represents Cl;
- or Ar₁ represents 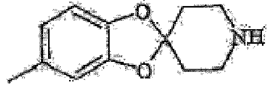 and X represents Cl;
- or Ar₁ represents 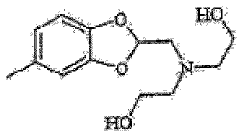 and X represents Cl;
- or Ar₁ represents 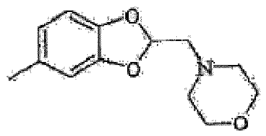 and X represents Cl;
- or Ar₁ represents 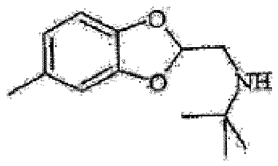 and X represents Cl;
- or Ar₁ represents 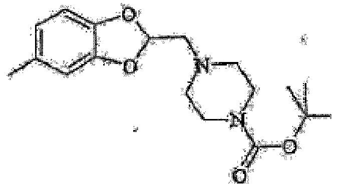 and X represents Cl;
- or Ar₁ represents 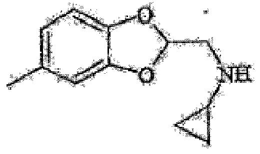 and X represents Cl;
- or Ar₁ represents 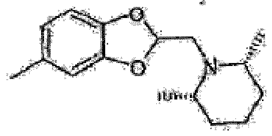 and X represents Cl;
- or Ar₁ represents 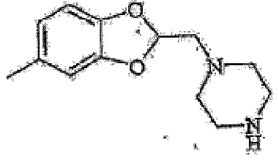 and X represents Cl;
- or Ar₁ represents 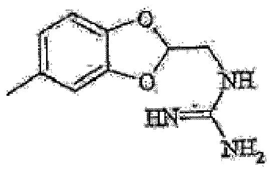 and X represents Cl;
- or Ar₁ represents 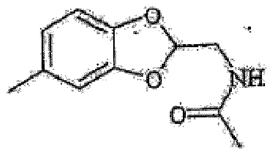 and X represents Cl;
- or Ar₁ represents 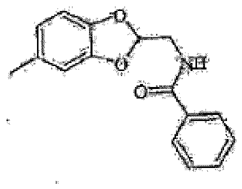 and X represents Cl;
- or Ar₁ represents 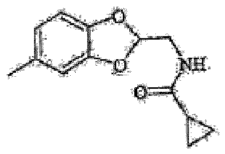 and X represents Cl;
- or Ar₁ represents 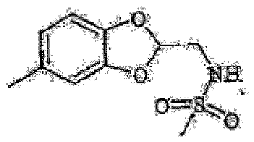 and X represents Cl;
- or Ar₁ represents 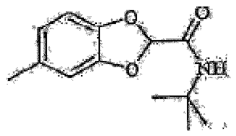 and X represents Cl;
- or Ar₁ represents 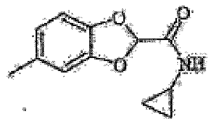 and X represents Cl;
- or Ar₁ represents 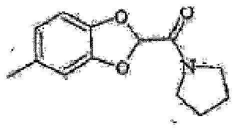 and X represents Cl;
- or Ar₁ represents 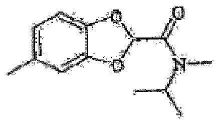 and X represents Cl;
- or Ar₁ represents 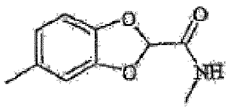 and X represents Cl;
- or Ar₁ represents 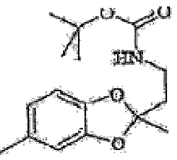 and X represents Cl;
- or Ar₁ represents 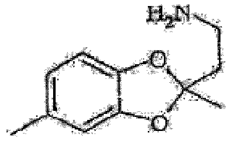 and X represents Cl;
- or Ar₁ represents 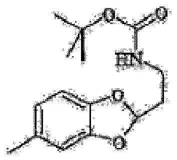 and X represents Cl;
- or Ar₁ represents 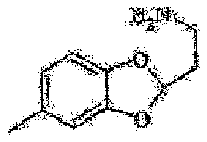 and X represents Cl;
- or Ar₁ represents 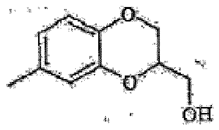 and X represents Cl;
- or Ar₁ represents 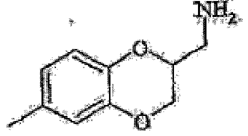 and X represents Cl;
- or Ar₁ represents 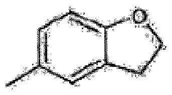 and X represents Cl;
- or Ar₁ represents 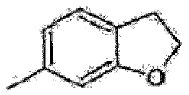 and X represents Cl;
- or Ar₁ represents 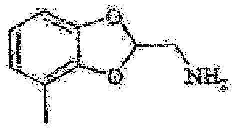 and X represents Cl;
- or Ar₁ represents 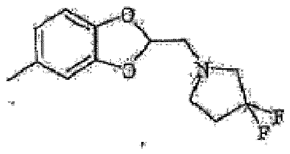 and X represents Cl;
- or Ar₁ represents 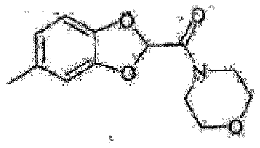 and X represents Cl;
- or Ar₁ represents 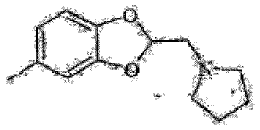 and X represents Cl;
- or Ar₁ represents 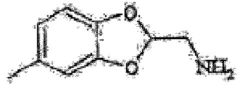 and X represents Br;
- or Ar₁ represents 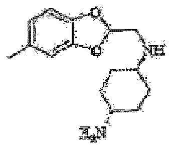 and X represents Cl;
- or Ar₁ represents 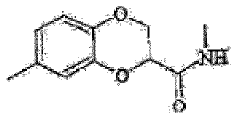 and X represents Cl;
- or Ar₁ represents 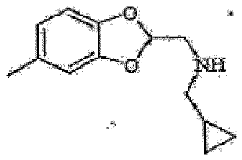 and X represents Cl.

2. Compound chosen from one of the following compounds:

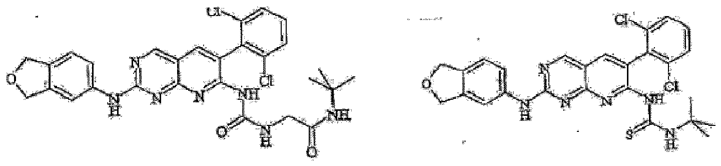

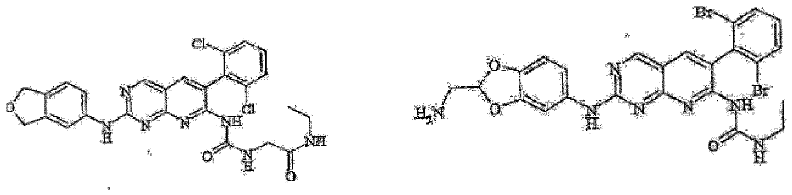

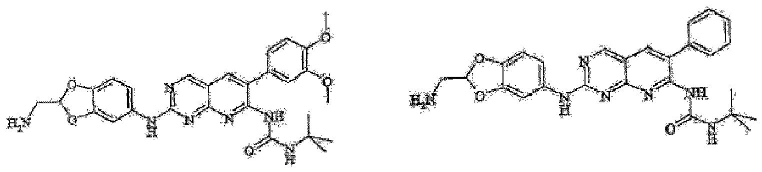

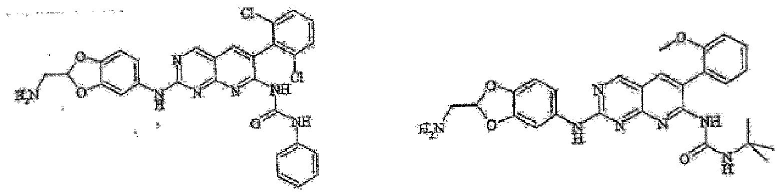

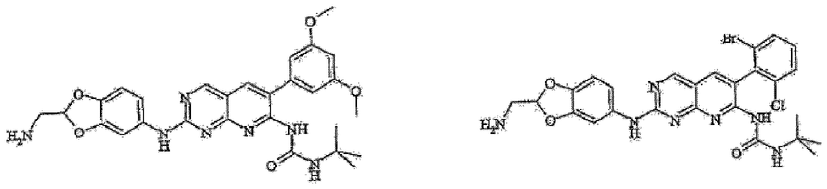

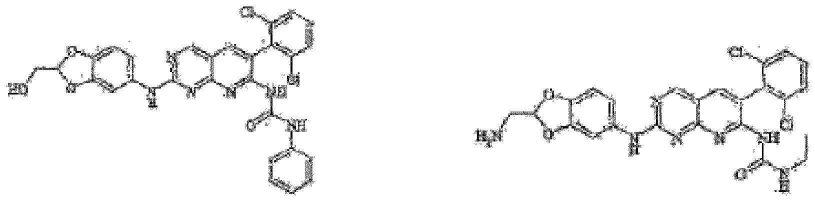

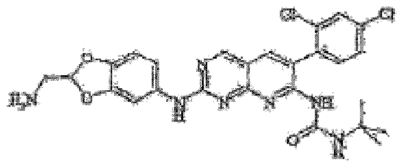

3. Compound corresponding to the formula below: 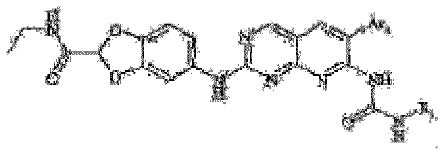 in which
- either R1 represents phenyl and Ar2 represents 2,6-dichlorophenyl;
- or R1 represents t-butyl and Ar2 represents 3,5-dimethoxyphenyl;
- or R1 represents ethyl and Ar2 represents 2,6-dichlorophenyl;
- or R1 represents t-butyl and Ar2 represents 3,4-dimethoxyphenyl;
- or R1 represents t-butyl and Ar2 represents phenyl;
- or R1 represents t-butyl and Ar2 represents 2-methoxyphenyl;
- or R1 represents t-butyl and Ar2 represents 2,6-dibromophenyl;
- or R1 represents t-butyl and Ar2 represents 2-bromo-6-chlorophenyl;
- or R1 represents ethyl and Ar2 represents 2,6-dibromophenyl;
- or R1 represents phenyl and Ar2 represents 2-bromo-6-chlorophenyl;
- or R1 represents phenyl and Ar2 represents 2,6-dibromophenyl.

4. Compound corresponding to the formula below: 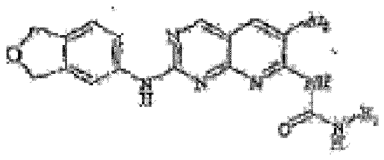 in which
- either R1 represents phenyl and Ar2 represents 2,6-dichlorophenyl;
- or R1 represents t-butyl and Ar2 represents 3,5-dimethoxyphenyl;
- or R1 represent ethyl and Ar2 represents 2,6-dichlorophenyl;
- or R1 represents t-butyl and Ar2 represents 3,4-dimethoxyphenyl;
- or R1 represents t-butyl and Ar2 represents phenyl;
- or R1 represents t-butyl and Ar2 represents 2-methoxyphenyl;
- or R1 represents t-butyl and Ar2 represents 2,6-dibromophenyl;
- or R1 represents t-butyl and Ar2 represents 2-bromo-6-chlorophenyl;
- or R1 represents ethyl and Ar2 represents 2,6-dibromophenyl;
- or R1 represents phenyl and Ar2 represents 2-bromo-6-chlorophenyl;
- or R1 represents phenyl and Ar2 represents 2,6-dibromophenyl.
